# EUROPEAN PATENT APPLICATION

(11) **EP 2 292 642 A1**
(43) Date of publication of application: **09.03.2011**
(21) Application number: 10184894.3
(22) Date of filing: 10.06.2004
(51) Int. Cl.: C07K 14/02, C07K 7/00, C12N 5/00, A61K 38/00, A61P 37/00, A61P 31/12

(54) **Immunomodulating compositions, uses therefor and processes for their production**

(30) Priority: 10.06.2003 AU 2003902875; 25.03.2004 AU 2004901589
(62) Divisional of application: 04737428.5
(71) Applicant: Opal Therapeutics Pty Ltd, Melbourne, Victoria 3000 (AU)
(72) Inventor: Kent, Stephen John, Camberwell Victoria 3124 (AU)
(74) Representative: Jones, Elizabeth Louise

(57) **Abstract**

The present invention relates to the use of at least one set of peptides in composition and methods for modulating an immune response to one or more polypeptide antigens. In certain embodiments, the sequences of a respective set of peptides are derived in whole, or in part, from a single polypeptide antigen. Individual peptides of a respective peptide set comprise different portions of an amino acid sequence corresponding to a single polypeptide antigen and display partial sequence identity or similarity to at least one other peptide of the same set of peptides. The invention also extends to methods of using such peptides in a range of preventive, diagnostic and therapeutic applications. Additionally, the inventions relates to the use of uncultured antigen-presenting cells or their precursors, which have not been subjected to activating conditions, and which have been contacted with an antigen, in methods and compositions for modulating an immune response in a recipient of those cells.

## Description

### FIELD OF THE INVENTION

THIS INVENTION relates generally to modulation of immune responses More particularly, the present invention relates to the use of at least one set of peptides in compositions and methods for modulating an immune response to one or more polypeptide antigens. In certain embodiments, the sequences of a respective set of peptides are derived in whole, or in part, from a single polypeptide antigen. Individual peptides of a respective peptide set comprise different portions of an amino acid sequence corresponding to a single polypeptide antigen and display partial sequence identity or similarity to at least one other peptide of the same set of peptides. The invention also extends to methods of using such peptides in a range of preventive, diagnostic and therapeutic applications. Additionally, the invention relates to the use of uncultured antigen-presenting cells or their precursors, which have not been subjected to activating conditions, and which have been contacted with an antigen, in methods and compositions for modulating an immune response in a recipient of those cells,

Bibliographic details of various publications numerically referred to in this specification are collected at the end of the description.

### BACKGROUND OF THE INVENTION

Since its discovery almost 20 years ago, the human immunodeficiency virus type-1 (HIV-1) has claimed more than 22 million lives and is continuing to devastate communities worldwide (1). Forty-two million people are currently living with HIV-1 and, despite efforts to modify high-risk behaviour, an estimated 5 million new infections occur yearly (2). Similarly, Hepatitis C virus (HCV) and Hepatitis B virus infections result in chronic liver damage and hepatocellular damage in millions of people worldwide. Safe and effective preventative or therapeutic vaccines for these viruses are desperately needed. Additionally, it is now believed that immune protection from, or clearance of, many cancers requires specific T cell responses.

The elimination of persistent intracellular pathogens such as replicating viruses generally requires the mobilisation of cell-mediated immunity (CMI). CD8+ cytotoxic T lymphocytes (CTL) are the primary effector cells of CMI; they kill viral-infected cells by recognising viral peptides presented on the cell surface in the context of MHC class I molecules. Prior to the appearance of virus-specific antibodies, a robust HIV-1-specific CTL response temporally correlates with reduced viremia during the acute stage of HIV-1 infection (3, 4). Furthermore, strong CTL responses are associated with reduced HIV-1 viremia during chronic infection (5, 6), whereas a decline in HIV-1-specific CTL is linked to rapid progression to AIDS (4, 7-9). Similarly, clearance of HCV infections is generally thought to be assisted by virus-specific T cell responses.

There are no effective vaccines against HIV-1, HCV or cancers. Early HIV-1 vaccine strategies were based on whole-inactivated virus and recombinant structural proteins such as the envelope (env) glycoprotein. Non-human primate models revealed only limited strain-specific protection by these vaccines against pathogenic simian immunodeficiency virus (SIV) and highly pathogenic SHIV (SIV-HIV-1 chimeric) challenges (10-13). The first human phase III trials also failed to show efficacy (14).

Particle- and recombinant whole protein-based vaccines, although safe, favour the generation of antibodies that are insufficient for protection against many chronic viral pathogens. Alternatively, intracellularly expressed antigens are subsequently more likely to induce CTL responses. Live-attenuated viruses generate potent cell-mediated immunity (CMI) responses, however their clinical safety is of concern (15). Consequently, much focus has shifted toward genetically engineered vectors (such as DNA plasmids and poxviruses) expressing HIV-I/SIV genes (such as *env, gag* and *pol*) or HCV genes (16).

It is not known which immune-target antigens are protective, but a large breadth of T cell responses has been shown to reduce the opportunity for viral escape mutations to arise (17). It is this large breadth of potential epitopes, however, which renders the construct of large vectors frequently difficult and as well as being complicated by potential safety issues. Concerns have been raised about the potential ability of DNA vaccines to integrate with host DNA, as well as the safety of viral vector vaccines in immunocompromised hosts. These represent the significant regulatory hurdles for these recombinant vaccines.

Also, despite significant advances towards understanding how T and linear B cell epitopes are processed and presented to the immune system, the full potential of epitope-based vaccines has not been fully exploited. The main reason for this is the large number of different T cell epitopes, which must be identified for inclusion into such vaccines to cover the extreme human leucocyte antigen (HLA) polymorphism in the human population.

Infusion of whole antigen-pulsed or single epitope-pulsed cultured antigen presenting cells (APC) has previously been reported to be immunogenic in mouse models (22-27). However, other reports in inbred mouse models suggest the infusion of cells pulsed with single peptides may even be tolerogenic (induces a state of tolerance to the antigen which would be counterproductive for a vaccine) (28-31).

### SUMMARY OF THE INVENTION

The present invention discloses the discovery that autologous cells, which have been contacted with overlapping peptides of a viral polypeptide antigen of interest produce a strong immunogenic response in an outbred population that protects against subsequent viral challenge. The present inventors propose that similar protective responses would be achieved using systemic administration of the overlapping peptides per se. The use of multiple overlapping peptides provides several advantages, including reducing the emergence of escape mutants and the facile production of peptide-based immunogenic compositions without prior knowledge of any epitopes. In this regard, the sequence overlap between peptides reduces or prevents loss of potential epitopes, which broadens the immunological coverage of the composition to cover potentially the diversity in the major histocompatability complex (MHC) across an outbred population.

Accordingly, in one aspect of the present invention, there is provided at least one set of peptides for modulating an immune response to one or more polypeptides of interest.Individual peptides of a respective set comprise different portions of an amino acid sequence corresponding to a single polypeptide of interest (e.g., particular pathogenic regions of a polypeptide), and display partial sequence identity or similarity to at least one other peptide of the same set of peptides. In certain embodiments, at least 2, 3, 4, 5, 6 or 7 sets of peptides are employed, wherein peptide sequences in each set are derived from a distinct polypeptide of interest.

The partial sequence identity or similarity is typically contained at one or both ends of an individual peptide. Suitably, at one or both of these ends there are at least 4, 5, 6, 7, 8, 9, 10,11,12, 13, 14 contiguous amino acid residues whose sequence is identical or similar to an amino acid sequence contained within at least one other of the peptides.

In certain embodiments, the peptide is at least 6, 7, 8, 9, 10,11,12,13,14,15, 20,25, 30 amino acid residues in length and suitably no more than about 500, 200, 100, 80, 60, 50, 40 amino acid residues in length. Suitably, the length of the peptides is selected to enhance the production of a cytolytic T lymphocyte response (e.g., peptides of about 8 to about 10 amino acids in length), or a T helper lymphocyte response (e.g., peptides of about 12 to about 20 amino acids in length).

In certain embodiments, the peptide sequences are derived from at least about 30, 40, 50, 60, 70, 80, 90, 91, 92,93,94. 95, 96, 97, 98, 99% of the sequence corresponding to the polypeptide of interest.

The polypeptide of interest is suitably an antigen selected from a protein antigen, an antigen expressed by cancer cells, a particulate antigen, an alloantigen, an autoantigen or an allergen, or an immune complex. In certain embodiments, the polypeptide of interest is a disease- or condition-associated polypeptide such as but not limited to a polypeptide produced by a pathogenic organism or a cancer. Examples of pathogenic organisms include, but are not restricted to, yeast, viruses, bacteria, helminths, protozoans and mycoplasmas. Examples of cancers include, but are not restricted to, melanoma, lung cancer, breast cancer, cervical cancer, prostate cancer, colon cancer, pancreatic cancer, stomach cancer, bladder cancer, kidney cancer, post transplant lymphoproliferative disease (PTLD), Hodgkin's Lymphoma and the like.

In another aspect, the invention provides antigen-presenting cells or their precursors which have been contacted with a set of peptides as broadly described above for a time and under conditions sufficient for the peptides or processed forms thereof to be presented by the antigen-presenting cells or by their precursors,

In a related aspect, the invention provides a process for producing antigen-presenting cells for modulating an immune response to a polypeptide of interest. The process generally comprises contacting antigen-presenting cells or their precursors with at least one set of peptides as broadly described above for a time and under conditions sufficient for the peptides or processed form thereof to be presented by the antigen-presenting cells or by their precursors. Suitably, when precursors are used, the precursors are cultured for a time and under conditions sufficient to differentiate antigen-presenting cells from the precursors.

In some embodiments, the or each set of peptides is contacted with substantially purified antigen-presenting cells or their precursors. In other embodiments, the or each set of peptides is contacted with a heterogeneous population of antigen-presenting cells or their precursors. In these embodiments, the heterogenous pool of cells can be blood or peripheral blood mononuclear cells. Typically, the antigen-presenting cells or their precursors are selected from monocytes, macrophages, cells of myeloid lineage, B cells, dendritic cells or Langerhans cells. In still other embodiments, the or each set of peptides is contacted with an uncultured population of antigen-presenting cells or their precursors. The population can be homogenous or heterogeneous, illustrative examples of which include whole blood, fresh blood, or fractions thereof such as, but not limited to, peripheral blood mononuclear cells, buffy coat fractions of whole blood, packed red cells, irradiated blood, dendritic cells, monocytes, macrophages, neutrophils, lymphocytes, natural killer cells and natural killer T cells.

The antigen-presenting cells broadly described above are also useful for producing lymphocytes, including T lymphocytes and B lymphocytes, for modulating an immune response to a specified antigen or group of antigens. Accordingly, in yet another aspect, the invention provides a method for producing antigen-specific lymphocytes. The method comprises contacting a population of lymphocytes, or their precursors, with an antigen-presenting cell as broadly described above for a time and under conditions sufficient to produce the antigen-specific lymphocytes that modulate an immune response to at least one polypeptide from which the overlapping peptides were derived.

In yet another aspect, the invention contemplates a composition comprising at least one set of peptides, or the antigen-presenting cells, or the lymphocytes, as broadly described above, and a pharmaceutically acceptable carrier and/or diluent. In certain embodiments, the composition may further comprise an adjuvant or compounds that stabilise the peptides or antigens against degradation by host enzymes.

In yet another aspect, the invention embraces a method for modulating an immune response to a polypeptide of interest, comprising administering to a patient in need of such treatment at least one set of peptides, or the antigen-presenting cells, or the lymphocytes, or the composition as broadly described above for a time and under conditions sufficient to modulate the immune response.

In a related aspect, the invention encompasses a method for treatment and/or prophylaxis of a disease or condition associated with the presence of a polypeptide of interest, comprising administering to a patient in need of such treatment or prophylaxis an effective amount of at least one set of peptides, or the antigen-presenting cells, or the lymphocytes, or the composition as broadly described above. In some embodiments, peptides or antigen-presenting cells or the lymphocytes are administered systemically, typically by injection.

In still yet another aspect, the invention contemplates the use of at least one set of peptides, or of the antigen-presenting cells, or of the lymphocytes, as broadly described above, in the preparation of a medicament for modulating an immune response to a polypeptide of interest or for treating or preventing a disease or condition associated with the presence of a polypeptide of interest,

The present invention also discloses the discovery that it is not necessary to culture a population of antigen-presenting cells or their precursors to expand that population prior to contacting it with a target antigen so that the contacted population is useful for modulating an immune response to the target antigen in a suitable recipient. Instead, the present inventors have unexpectedly discovered that uncultured antigen-presenting cells or their precursors, when contacted with an antigen that corresponds to a target antigen, are sufficient to modulate an immune response to the target antigen. The use of uncultured antigen-presenting cells or their precursors circumvents the need for expensive culturing and cell processing facilities and, in certain desirable embodiments, provides much faster vaccination regimens, as compared to current protocols. Additionally, the present inventors have discovered that it is not necessary to incubate the uncultured antigen-presenting cells under conditions that lead to their activation, in order to effectively modulate the immune response to the target antigen, which further reduces the number of process steps and manipulations.

Accordingly, in another aspect, the present invention features a composition of matter for modulating an immune response in a subject to a target antigen, the composition comprising uncultured antigen-presenting cells or their precursors, which have not been subjected to activating conditions, and which have been contacted with an antigen corresponding to the target antigen for a time (e.g., from about 1 minute to about 5 days) and under conditions sufficient to express a processed or modified form of the antigen for presentation to the subject's immune system (e.g., T lymphocytes). Illustrative examples of uncultured cells include whole blood, fresh blood, or fractions thereof such as but not limited to peripheral blood mononuclear cells, buffy coat fractions of whole blood, packed red cells, irradiated blood, dendritic cells, monocytes, macrophages, neutrophils, lymphocytes, natural killer cells and natural killer T cells.

The antigen corresponding to the target antigen can be of any type including, for example, nucleic acids, peptides, hormones, whole protein antigens, cellular material (e.g., live or inactivated cancer cells), particulate matter such as, but not limited to, cell debris, apoptotic cells, lipid aggregates such as liposomes, membranous vehicles, microspheres, heat aggregated proteins, virosomes, virus-like particles and whole organisms including, for example, bacteria, mycobacteria, viruses, fungi, protozoa or parts thereof. In some embodiments, the antigen is selected from a proteinaceous molecule or a nucleic acid molecule. In some embodiments, the uncultured cells are contacted with at two or more antigens. In illustrative examples of this type, the antigens are in the form of overlapping or non-overlapping peptides or one or more polynucleotides from which the peptides are expressible.

In a related aspect, the invention extends to the use of uncultured antigen-presenting cells or their precursors in the preparation of a medicament for the treatment of a disease or condition in a subject, which disease or condition is associated with the presence or aberrant expression of a target antigen, wherein the antigen-presenting cells or their precursors have not been subjected to activating conditions but have been contacted with an antigen that corresponds to the target antigen for a time and under conditions sufficient to express a processed or modified form of the antigen for presentation to the subject's immune system.

### BRIEF DESCRIPTION OF THE DRAWINGS

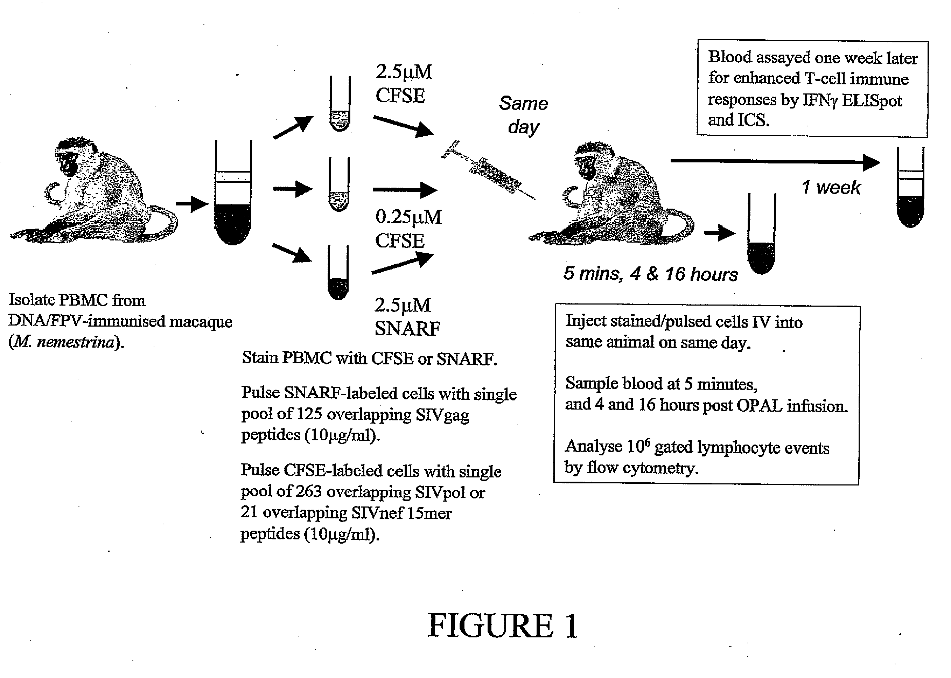

Figure 1 is a schematic representation of an *in vivo* CTL killing assay performed at weeks 10, 15 and 20.

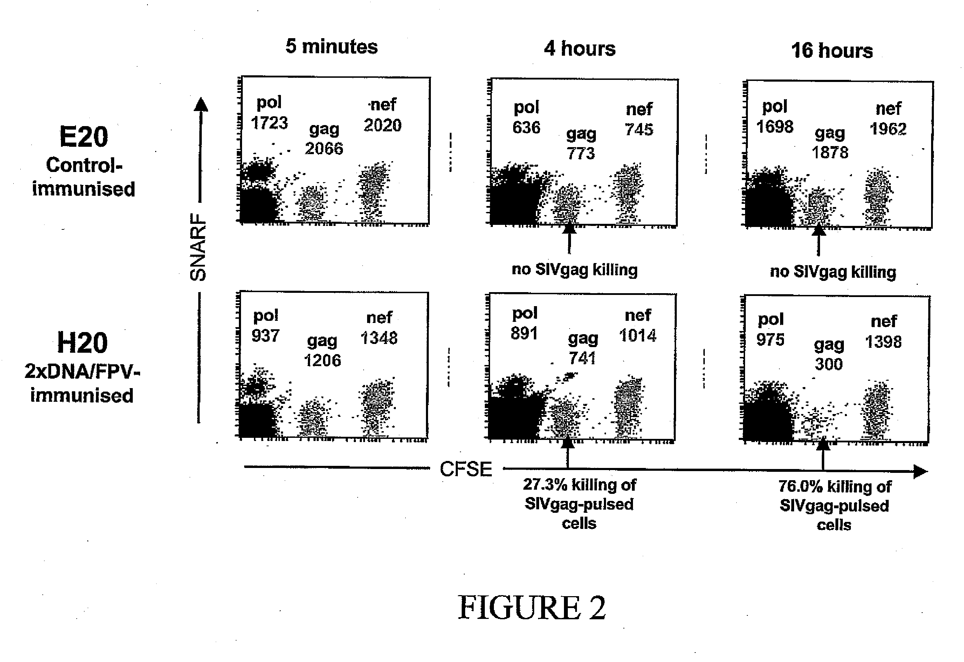

Figure 2 is a graphical representation showing *in vivo* CTL killing of SIVgag overlapping peptide-pulsed cells. Two weeks after the FPV-boost (week 10), 3 equal PBMC populations were labelled with SNARF (2.5µM) or CFSE (2.5 µM or 0.25 µM) and were pulsed with SIVpol, nef or gag overlapping peptide pools (OPAL), respectively. Blood sampled at 5 min, and at 4 and 16 h post-OPAL infusion was RBC-lysed and 10⁶ lymphocyte events were acquired by flow cytometry. At 5 min, all 3 populations of labelled PBMC are of relatively equal numbers. By 4 and 16 hours, 2xDNA/FPV-immunised monkey H20 displayed 27.3% and 76.0% clearance of SIVgag-pulsed PBMC with respect to SIVnef-pulsed PBMC, respectively, whereas no SIVgag-specific killing was observed in control-immunised monkey E20. Note that less events were collected at 4 h than 16 h.

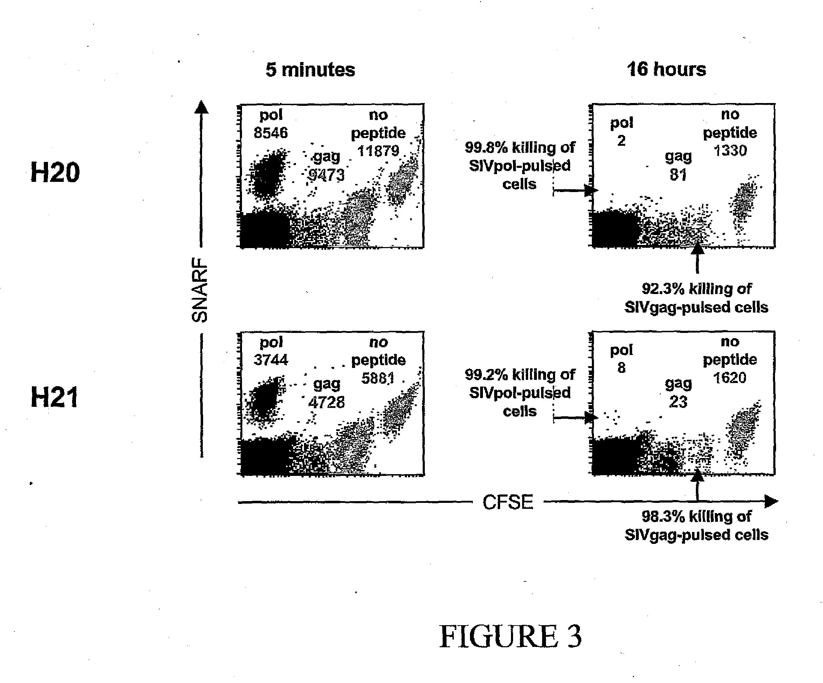

Figure 3 is a graphical representation showing vigorous killing of SIVgag- and SIVpol-pulsed PBMC following SHIV challenge. Two weeks after SHIV challenge (week 20), equal PBMC populations were labelled with SNARF (5µM) or CFSE (6µM or 2.5 µ4 and were pulsed with SIVpol, no peptide, or SIVgag overlapping peptide pools (OPAL), respectively. 10⁶ RBC-lysed lymphocyte events were acquired by flow cytometry. 2xDNA/FPV-immunised monkeys H20 and H21, Displayed 92.3% and 98.3% killing of SIVgag-pulsed PBMC. These animals received 2 separate infusions of SIVpol- pulsed PBMC, furthermore displaying >99% SIVpol-specific killing. Previously CFSE-labelled PBMC were accounted for by flow cytometric analysis of 10⁶ lymphocytes immediately prior to OPAL-infusion (not shown).

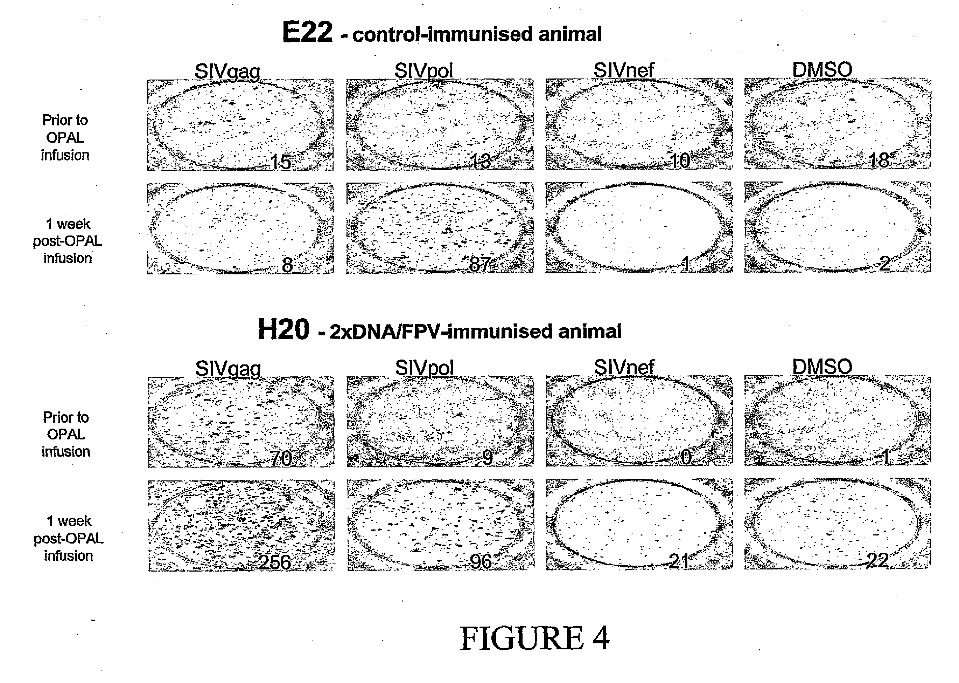

Figure 4 is a photographic representation showing a boost in T-cell immunogenicity week following OPAL-infusion analysed by IFNγ ELISpot. A boost in SIVgag and pol peptide pool responses is evident in 2xDNA/FPV-immunised monkey H21, where as a primed response to SIVpol peptide pool is detected in control-immunised monkey E20 (week 10 shown above).

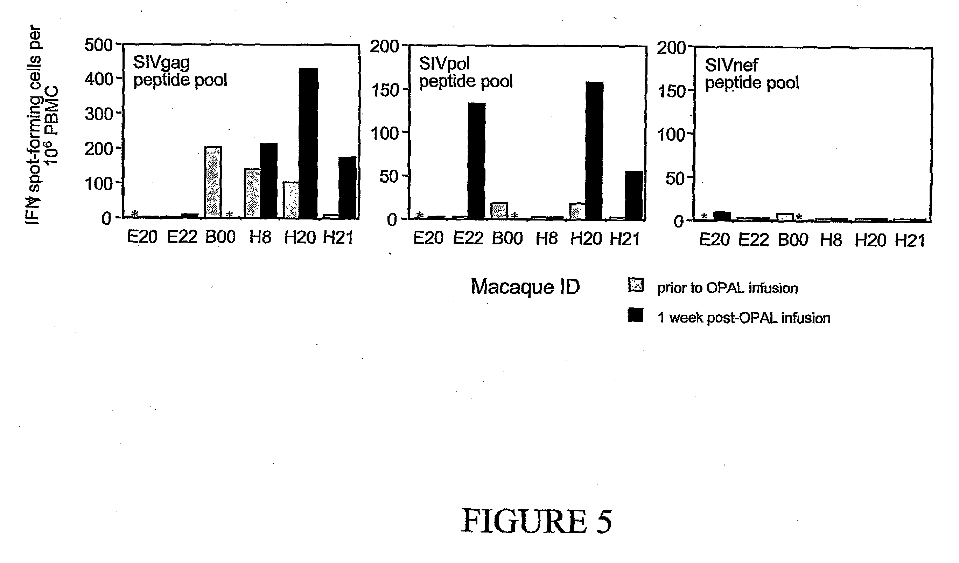

Figure 5 is a graphical representation depicting INFγ ELISpot analysis 1 week following OPAL infusion at week 10. A boost in T-cell immunogenicity to SIVgag, pol and nef overlapping peptide pools by OPAL infusion at week 10 was analysed 1 week later by ELISpot. Increased responses to SIVgag were detected in all four 2xDNA/FPV-immunised animals. Increased SIVpol responses were present in the 2xDNA/PPV-immunised monkeys, H20 and H21 (monkeys B00 and H8 did not receive any pol-pulsed PBMC), and in one control-immunised monkey, E20, No responses to' SIVnef were primed in any animals. *IFNγ spots in monkeys E20 (prior to OPAL infusion) and B00 (post-OPAL infusion) were excluded due to ELISpot developmental problems.

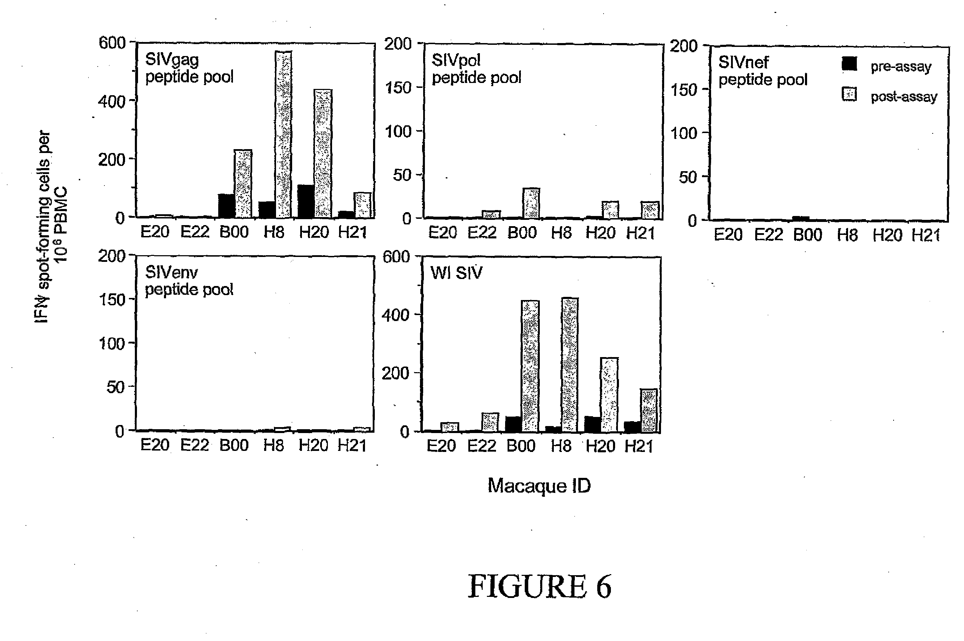

Figure 6 is a graphical representation showing INFγ ELISpot analysis 1 week following OPAL infusion at week 15. A boost in T-cell immunogenicity to SIVgag, pol, nef and HIV-1env overlapping peptide pools by OPAL infusion at week 15 was analysed 1 week later by INFγ ELISpot. Increased responses to SIVgag were detected in all four 2xDNA/FPV-immunised animals. SIVpol responses were marginally increased (or primed) in monkeys, E22, B00, H20 and H21. Increased responses to WI SIV were evident in all animals, whereas no responses were detected for SIVnef or HIV-env in any animals.

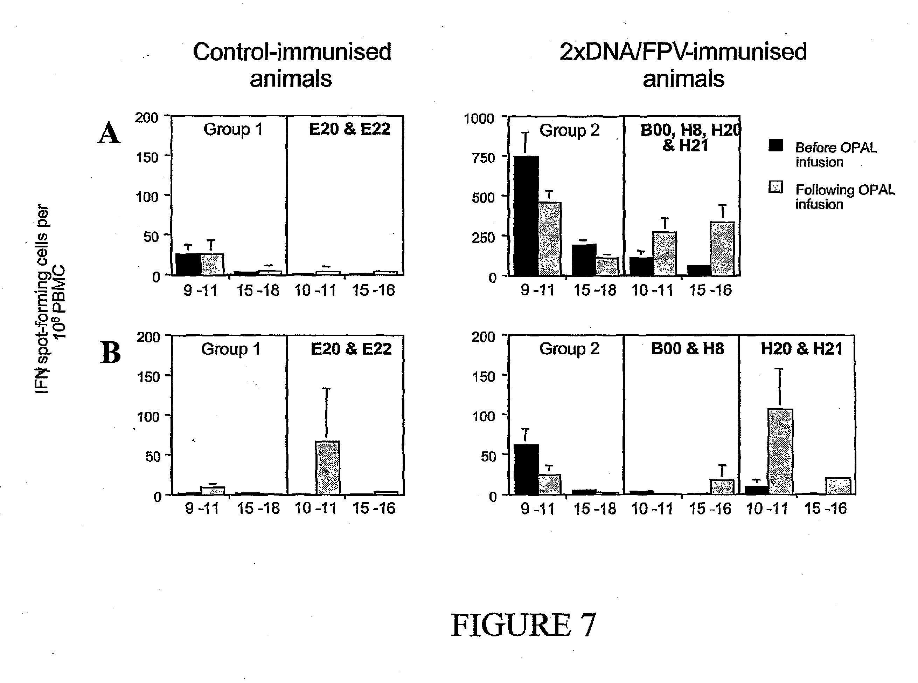

Figure 7 is a graphical representation depicting mean INFγ ELISpot of immunogenicity of OPAL infusion. Mean INFγ ELISpot responses to (A) SIVgag and (B) SIVpol overlapping peptide pool of control- and 2xDNA/FPV-immunised animals receiving OPAL infusions (bold) were compared to animals receiving equivalent immunisations but no OPAL infusions, before an after the OPAL infusions given at weeks 10 and 15 following the immunisation. For the comparison of SIVpol-specific responses, 2xDNA/FPV-immunised animals were grouped based on receiving either 1 (B00 and H8) or 2 (H20 and H21) doses ofpol-OPAL infusions.

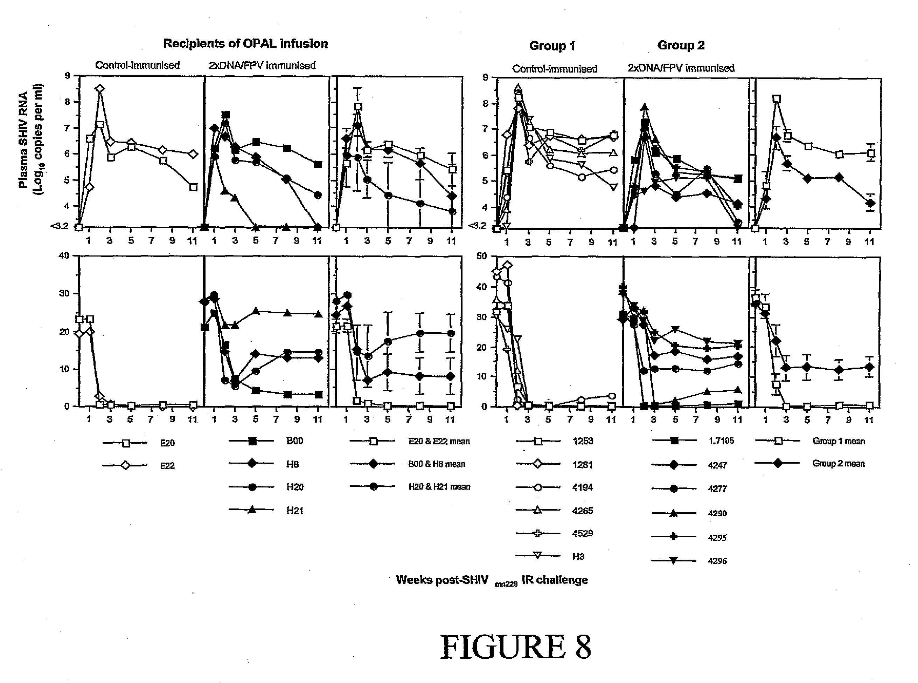

Figure 8 is a graphical representation showing the outcome of SHIV intrarectal challenge. At week 18 all control-and 2xDNA/FPV-inmunised macaques were challenged intrarectally with SHIVₘₙ₂₂₉ and were assessed for plasma SHIV RNA viral load and CD4+ T cell count over the course of the infection. Recipients of OPAL infusion were compared to their respective immunised non-OPAL recipients. Group comparisons indicate mean ± SE. 2xDNA/FPV-immunised macaques receiving OPAL infusions were further grouped based on receiving either 1 or 2 separate doses of pol-pulsed PBMC (B00 & H8, and H20 & H21, respectively).

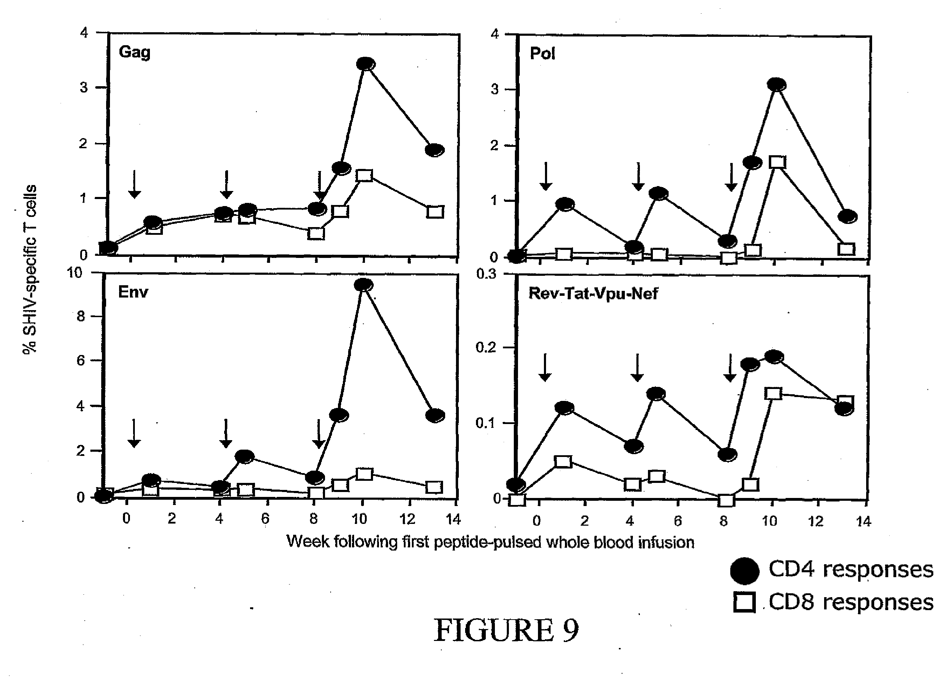

Figure 9 is a graphical representation depicting induction of CD4+ and CD8+ T cell responses to SHIV antigens in monkeys infected with SHIV utilising administration of whole blood pulsed with overlapping 15mer peptides encompassing the open reading frames of the entire SHIV genome. The whole blood pulsed peptides were administered at weeks 0, 4 and 8 (arrows) and a boost in T cell immunogenicity of both CD4+ and CD8+ T cells measured by IFNgamma production to SHIV antigens gag, pol, env and rev-tat-vpu-nef detected by ICS is seen following each time point. *Pre-OPAL T cells responses measured 1 week prior to 1^{st} OPAL (week -1).

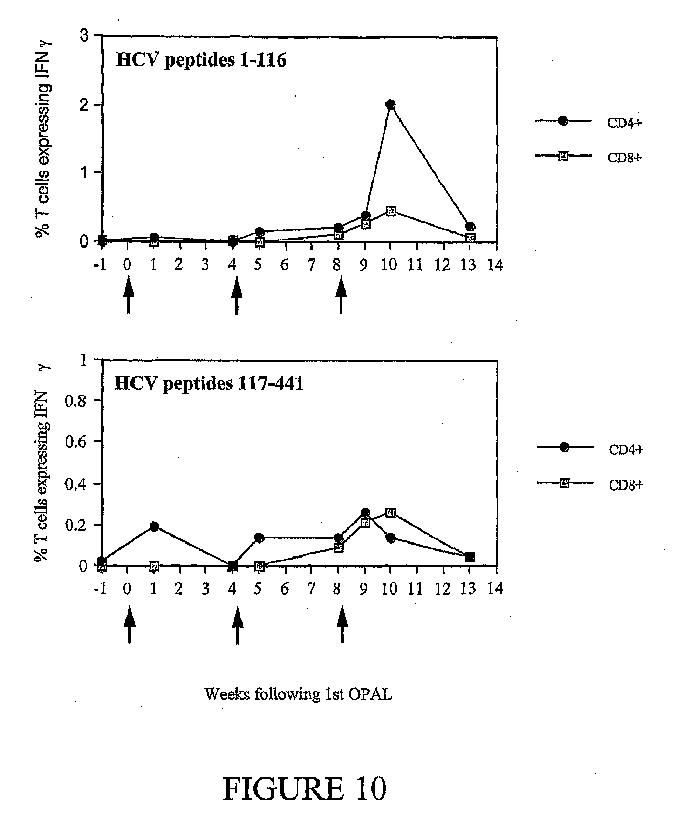

Figure 10 is a graphical representation depicting *de novo* induction of CD4+ and CD8+ T cell responses to HCV in monkeys utilising administration of whole blood pulsed with overlapping l8mer peptides encompassing the open reading frames of the entire HCV type-la H77 genome. The whole blood pulsed peptides were administered at weeks 0, 4 and 8 (arrows) in two separate pools (peptides: 1-116, and; 117-441). Induction and boosting of T cell immunogenicity of both CD4+ and CD8+ T cells measured by IFNgamma production to HCV antigens detected by ICS is seen following each time point. *Pre-OPAL T cells responses measured 1 week prior to 1^{st} OPAL (week -1).

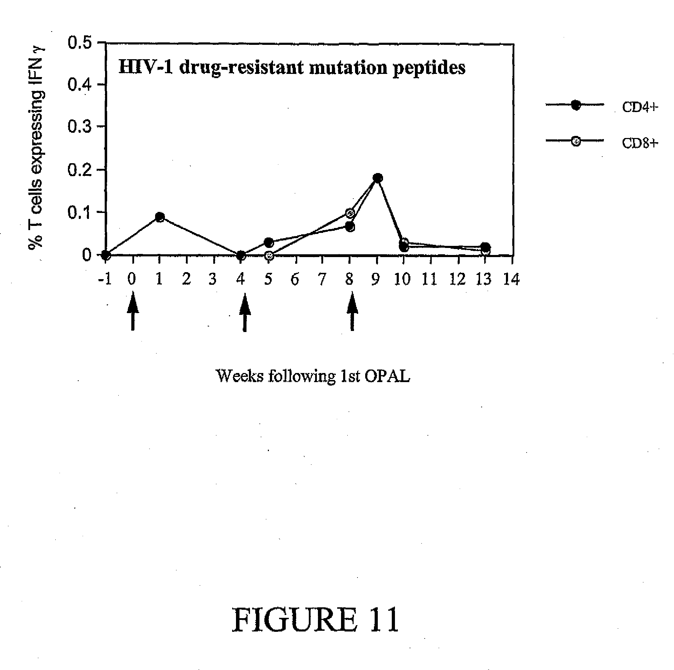

Figure 11 is a graphical representation showing *de novo* induction of CD4+ and CD8+ T cell responses to peptides representative of drug-resistant mutations in HIV-1 described in H1V-1 infected humans, in monkeys utilising administration of whole blood pulsed with 17mer peptides encompassing known sites of reverse transcriptase or protease resistance mutations. The whole blood pulsed peptides were administered at weeks 0, 4 and 8 (arrows). Induction and boosting of T cell immunogenicity of both CD4+ and CD8+ T cells measured by IF`NNgamma production to HIV-1 drug-resistant mutation peptides detected by ICS is seen following each time point. *Pre-OPAL T cells responses measured 1 week prior to 1^{st} OPAL (week -1).

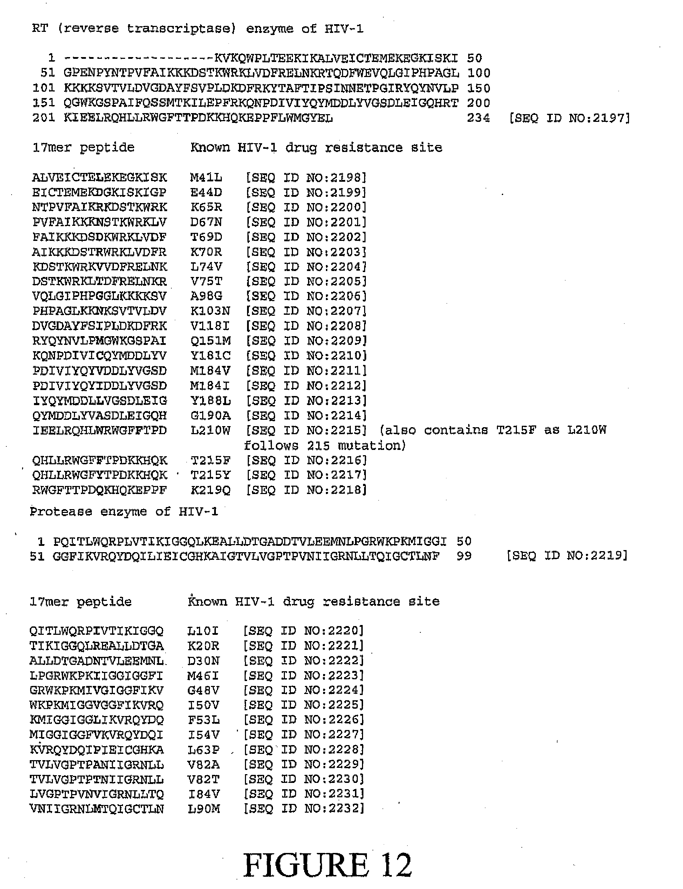

Figure 12 is a diagrammatic representation showing one embodiment of a pool of single peptides corresponding to drug-resistant mutations in the reverse transcriptase region or the protease region of wild-type HIV- described in HIV-1 humans (Mimotopes, Melbourne). 17mer peptides were designed spanning the sites of common known mutations to incorporate the resistant mutation at the 9^{th} amino acid residue (bold) on each l7mer peptide, such that every 9mer epitope (the most common length of CD8+ T cell epitopes) as a result of proteolytic cleaving *ex vivo* would encompass the mutation.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles *"a"* and *"an"* are used herein to refer to one or to more than one (i.e., to at least one) of the grammatical object of the article, By way of example, "an element" means one element or more than one element.

The term *"about"* is used herein to refer to conditions (e.g., amounts, concentrations, time etc) that vary by as much as 30%, preferably by as much as 20%, and more preferably by as much as 10% to a specified condition.

The term *"activating conditions"* refers to treatment conditions that lead to the expression of each of CD2, CD83, CD14, MHC class I, MHC class II and TNF-α at a level or functional activity that results from an activating treatment condition selected from: incubating the antigen-presenting cells or their precursors in the presence of an agent selected from cytokine (e.g., IL-4, GM-CSF or a type I interferon), chemokines, mitogens, lipopolysaccharide, or agents that induce interferon synthesis in the antigen-presenting cells or their precursors; or exposing the antigen-presenting cells or their precursors to physical stress. However, it shall be understood that the term *"activating conditions"* excludes treatment conditions that result in negligible activation of the cells, e.g., when less than about 20%, 15%, 10%, 9%, 8%, 7%, 6%, 5%, 4%, 3%, 2%, 1%, 0.5%, 0.2% or 0.1% of the cells are activated, or when each of CD2, CD83, CD14, MHC class I, MHC class II and TNF-α is expressed at a level or functional activity that is at least about 30%, 40%, 50%, 60%, 70%, 80% or 90%, or even at least about 100%, 200%, 300%, 400%, 500%, 600%, 700%, 800%, 900% or 1000% higher, or at least about 30%, 40%, 50%, 60%, 70%, 80%, 90%, 92%, 94%, 96%, 97%, 98% or 99%, or even an at least about 99.5%, 99.9%, 99.95%, 99.99%, 99.995% or 99.999% lower than its level or functional activity in antigen-presenting cells or their precursors subjected to an activating treatment condition mentioned above.

By *"antigen"* is meant all, or part of, a protein, peptide, or other molecule or macromolecule capable of eliciting an immune response in a vertebrate animal, preferably a mammal. Such antigens are also reactive with antibodies from animals immunised with said protein, peptide, or other molecule or macromolecule.

By *"antigen-binding molecule"* is meant a molecule that has binding affinity for a target antigen. It will be understood that this term extends to immunoglobulins, immunoglobulin fragments and non-immunoglobulin derived protein frameworks that exhibit antigen-binding activity.

By *"autologous"* is meant something (e.g., cells, tissues etc) derived from the same organism.

The term *"allogeneic"* as used herein refers to cells, tissues, organisms etc that are of different genetic constitution.

Throughout this specification, unless the context requires otherwise, the words *"comprise", "comprises"* and *"comprising"* will be understood to imply the inclusion of a stated step or element or group of steps or elements but not the exclusion of any other step or element or group of steps or elements.

By *"corresponds to"* or *"corresponding to"* is meant a polynucleotide (a) having a nucleotide sequence that is substantially identical or complementary to all or a portion of a reference polynucleotide sequence or (b) encoding an amino acid sequence identical to an amino acid sequence in a peptide or protein. This phrase also includes within its scope a peptide or polypeptide having an amino acid sequence that is substantially identical or similar to a sequence of amino acids in a reference peptide or protein.

As used herein, the terms *"culturing", "culture"* and the like refer to the set of procedures used *in vitro* where a population of cells (or a single cell) is incubated under conditions which have been shown to support the growth or maintenance of the cells *in vitro.* The art recognises a wide number of formats, media, temperature ranges, gas concentrations etc. which need to be defined in a culture system. The parameters will vary based on the format selected and the specific needs of the individual who practices the methods herein disclosed. However, it is recognised that the determination of culture parameters is routine in nature,

By *"effective amount",* in the context of modulating an immune response or treating or preventing a disease or condition, is meant the administration of that amount of composition to an individual in need thereof, either in a single dose or as part of a series, that is effective for that modulation, treatment or prevention. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

By *"expression vector" is* meant any autonomous genetic element capable of directing the synthesis of a protein encoded by the vector. Such expression vectors are known by practitioners in the art.

The term *"gene"* as used herein refers to any and all discrete coding regions of the cell's genome, as well as associated non-coding and regulatory regions. The gene is also intended to mean the open reading frame encoding specific polypeptides, introns, and adjacent 5' and 3' non-coding nucleotide sequences involved in the regulation of expression. In this regard, the gene may further comprise control signals such as promoters, enhancers, termination and/or polyadenylation signals that are naturally associated with a given gene, or heterologous control signals. The DNA sequences may be cDNA or genomic DNA or a fragment thereof. The gene may be introduced into an appropriate vector for extrachromosomal maintenance or for integration into the host.

A compound or composition is *"immunogenic"* if it is capable of either: a) generating an immune response against an antigen (e.g., a tumour antigen) in a naive individual; or b) reconstituting, boosting, or maintaining an immune response in an individual beyond what would occur if the compound or composition was not administered, A compound or composition is immunogenic if it is capable of attaining either of these criteria when administered in single or multiple doses.

Reference herein to *"immuno-interactive"* includes reference to any interaction, reaction, or other form of association between molecules and in particular where one of the molecules is, or mimics, a component of the immune system.

By *"isolated"* is meant material that is substantially or essentially free from components that normally accompany it in its native state.

By *"modulating"* is meant increasing or decreasing, either directly or indirectly, the immune response of an individual. In certain embodiments, "modulation" or "modulating" means that a desired/selected response is more efficient (e.g., at least 10%, 20%, 30%, 40%, 50%, 60% or more), more rapid (e.g., at least 10%, 20%, 30%, 40%, 50%, 60% or more), greater in magnitude (e.g., at least 10%, 20%, 30%, 40%, 50%, 60% or more), and/or more easily induced (e.g., at least 10%, 20%, 30%, 40%, 50%, 60% or more) than in the absence of an antigen or than if the antigen had been used i alone.

The term *"operably connected"* or *"operably linked"* as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly_{;} the preferred positioning of a regulatory sequence element with respect to a heterologous gene to be placed under its control is defined by the positioning of the element in its natural setting; i.e. the genes from which it is derived.

The terms *"patient," "subject"* and *"individual"* are used interchangeably herein to refer to any subject, particularly a vertebrate subject, and even more particularly a mammalian subject, for whom therapy or prophylaxis is desired. However, it will be understood that these terms do not imply that symptoms are present. Suitable vertebrate animals that fall within the scope of the invention include, but are not restricted to, primates, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes, reptiles, avians, fish).

By *"pharmaceutically-acceptable carrier"* is meant a solid or liquid filler, diluent or encapsulating substance that may be safely used in topical or systemic administration,

The term *"polynucleotide"* or *"nucleic acid"* as used herein designates mRNA, RNA, cRNA, cDNA or DNA. The term typically refers to oligonucleotides greater than 30 nucleotides in length.

*"Polypeptide", "peptide"* and *"protein"* are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers.

Reference herein to a *"promoter"* is to be taken in its broadest context and includes the transcriptional regulatory sequences of a classical genomic gene, including the TATA box which is required for accurate transcription initiation, with or without a CCAAT box sequence and additional regulatory elements (i.e. upstream activating sequences, enhancers and silencers) which alter gene expression in response to developmental and/or environmental stimuli, or in a tissue-specific or cell-type-specific manner. A promoter is usually, but not necessarily, positioned upstream or 5', of a structural gene, the expression of which it regulates. Furthermore, the regulatory elements comprising a promoter are usually positioned within 2 kb of the start site of transcription of the gene. Preferred promoters according to the invention may contain additional copies of one or more specific regulatory elements to further enhance expression in a cell, and/or to alter the timing of expression of a structural gene to which it is operably connected.

The term *"purified peptide"* means that the peptide is substantially free of cellular material or other contaminating proteins from the cell or tissue source from which the peptide is derived, or substantially free from chemical precursors or other chemicals when chemically synthesised. "Substantially free" means that a preparation of a peptide of the invention is at least 10% pure. In certain embodiments, the preparation of peptide has less than about 30%, 25%, 20%, 15%, 10% and desirably 5% (by dry weight), of non-peptide protein (also referred to herein as a "contaminating protein"), or of chemical precursors or non-peptide chemicals. The invention includes isolated or purified preparations of at least 0.01,0.1,1.0, and 10 milligrams in dry weight.

The term *"recombinant polynucleotide"* as used herein refers to a polynucleotide formed *in vitro* by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

By *"Recombinαnt polypeptide"* is meant a polypeptide made using recombinant techniques, i.e., through the expression of a recombinant polynucleotide.

By *"reporter inolecule"* as used in the present specification is meant a molecule that, by its chemical nature, provides an analytically identifiable signal that allows the detection of a complex comprising an antigen-binding molecule and its target antigen. The term "reporter molecule" also extends to use of cell agglutination or inhibition of agglutination such as red blood cells on latex beads, and the like.

The term *"sequence identity"* as used herein refers to the extent that sequences are identical on a nucleotide-by-nucleotide basis or an amino acid-by-amino acid basis over a window of comparison. Thus, a *"percentage of sequence' identity"* is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, *"sequence identity"* will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software.

*"Similarity"* refers to the percentage number of amino acids that are identical or constitute conservative substitutions as defined in Table B *infra,* Similarity may be determined using sequence comparison programs such as GAP (Deveraux *et al.* 1984, *Nucleic Acids Research* **12**, 387-395). In this way, sequences of a similar or substantially different length to those cited herein might be compared by insertion of gaps into the alignment, such gaps being determined, for example, by the comparison algorithm used by GAP.

Terms used to describe sequence relationships between two or more polynucleotides or polypeptides include "reference sequence", "comparison window", "sequence identity", "percentage of sequence identity" and "substantial identity". A *"reference sequence"* is at least 12 but frequently 15 to 18 and often at least 25 monomer units, inclusive of nucleotides and amino acid residues; in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A *"compαrison* window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences. Optimal alignment of sequences for aligning a comparison window may be conducted by computerised implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i,e., resulting in the highest percentage homology over the comparison window) generated by any of the various methods selected. Reference also may be made to the BLAST family of programs as for example disclosed by Altschul et *αl.,* 1997, *Nucl. Acids Res.* 25:3389. A detailed discussion of sequence analysis can be found in Unit 19.3 of *Ausubel et αl.,* "Current Protocols in Molecular Biology", John Wiley & Sons Inc, 1994-1998, Chapter 15.

By *"substantially purified populαtion"* and the like is meant that greater than about 80%, usually greater than about 90%, more usually greater than about 95%, typically greater than about 98%, and more typically greater than about 99% of the cells in the population are antigen-presenting cells of a chosen type.

The term *"uncultured"* as used herein refers to a population of cells (or a single cell), which have been removed from an animal and incubated or processed under conditions that do not result in the growth or expansion of the cells *in vitro,* or that result in negligible growth or expansion of the cells (e.g., an increase of less than about 50%, 40%, 30%, 20%, 15%, 10%, 9%, 8%, 7%, 6%, 5%, 4%, 3%, 2%, 1%, 0.5%, 0.2% or 0.1% in cell number as compared to the number of cells at the commencement of the incubation or processing), In certain desirable embodiments, the population of cells (or the single cell) is incubated or processed under conditions supporting the maintenance of the cells *in vitro.*

By *"vector"* is meant a nucleic acid molecule, preferably a DNA molecule derived, for example, from a plasmid, bacteriophage, or plant virus, into which a nucleic acid sequence may be inserted or cloned. A vector preferably contains one or more unique restriction sites and may be capable of autonomous replication in a defined host cell including a target cell or tissue or a progenitor cell or tissue thereof, or be integrable with the genome of the defined host such that the cloned sequence is reproducible. Accordingly, the vector may be an autonomously replicating vector, i.e., a vector that exists as an extrachromosomal entity, the replication of which is independent of chromosomal replication, e.g., a linear or closed circular plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. A vector system may comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. The vector may also include a selection marker such as an antibiotic resistance gene that can be used for selection of suitable transformants.

### 2. Immunomodualting sets of overlapping peptides

The present invention is predicated in part on the discovery that antigen-presenting cells contacted *ex vivo* with a set of overlapping peptides spanning a viral polypeptide antigen of interest (also referred to herein as **O**verlapping **P**eptide-pulsed **A**utologous ceLls, OPAL) are effective in producing a strong immunogenic response in an outbred population, without prior knowledge of the epitopes of the antigen. Since antigen-presenting cells form a significant part of the circulatory system, it is proposed that systemic delivery of the overlapping peptides *per se* will produce a similar protective effect. Accordingly, the present invention broadly provides a set of peptides for modulating an immune response to a polypeptide of interest, wherein individual peptides comprise different portions of an amino acid sequence corresponding to the polypeptide of interest and display partial sequence identity or similarity to at least one other peptide of the set.

The partial sequence identity or similarity is typically contained at one or both ends of an individual peptide. In one embodiment, there are at least 4, 5, 6, 7, 8, 9, 10, 11, 12, 13, 14, 15, 20, 25, 30, 40, 50 contiguous amino acid residues at one or both ends of an individual peptide, whose sequence is identical or similar to an amino acid sequence contained within at least one other of the peptides. In an alternate embodiment, there are less than 500, 100, 50, 40, 30 contiguous amino acid residues at one or both ends of an individual peptide, whose sequence is identical or similar to an amino acid sequence contained within at least one other of the peptides. Such 'sequence overlap' is advantageous to prevent or otherwise reduce the loss of any potential epitopes contained within a polypeptide of interest. In specific examples disclosed herein, the sequence overlap is 11 amino acid residues.

Typically, when peptides have partial sequence similarity, their sequences will usually differ by one or more conserved and/or non-conserved amino acid substitutions. Exemplary conservative substitutions are listed in the following table.

**TABLE A**

| Original Residue | Exemplary Substitutions | Original Residue | Exemplary Substitutions |
|---|---|---|---|
| Ala | Ser | Leu | Ile,Val |
| Arg | Lys | Lys | Arg, Gln, Glu |
| Asn | Gln,His | Met | Leu,Ile, |
| Asp | Glu | Phe | Met,Leu,Tyr |
| Cys | Ser | Ser | Thr |
| Gln | Asn | Thr | Ser |
| Glu | Asp | Trp | Tyr |
| Gly | Pro | Tyr | Trp, Phe |
| His | Asn, Gln | Vat | Ile, Leu |
| Ile | Leu, Val | | |

Conserved or non-conserved substitutions may correspond to polymorphisms in a polypeptide of interest. Polymorphic polypeptides are expressed by various pathogenic organisms and cancers. For example, the polymorphic polypeptides may be expressed by different viral strains or clades or by different cancers in distinct individuals. Thus, where polymorphic regions of a pathogen of interest are involved, it is generally desirable to use additional sets of peptides covering the variation in amino acid residue at the polymorphic site.

The peptides of the invention may be of any suitable size that can be utilised to elicit an immune response to a polypeptide of interest. A number of factors can influence the choice of peptide size. For example, the size of a peptide can be chosen such that it includes, or corresponds to the size of, CD4+ T cell epitopes, CD8+ T cell epitopes and/or B cell epitopes, and their processing requirements. Practitioners in the art will recognise that class I-restricted CD8+ T cell epitopes are typically between 8 and 10 amino acid residues in length and if placed next to unnatural flanking residues, such epitopes can generally require 2 to 3 natural flanking amino acid residues to ensure that they are efficiently processed and presented Class II-restricted CD4+ T cell epitopes usually range between 12 and 25 amino acid residues in length and may not require natural flanking residues for efficient proteolytic processing although it is believed that natural flanking residues may play a role. Another important feature of class II-restricted epitopes is that they generally contain a core of 9-10 amino acid residues in the middle which bind specifically to class II MHC molecules with flanking sequences either side of this core stabilising binding by associating with conserved structures on either side of class II MHC antigens in a sequence independent manner. Thus the functional region of class II-restricted epitopes is typically less than about 15 amino acid residues long. The size of linear B cell epitopes and the factors effecting their processing, like class II-restricted epitopes, are quite variable although such epitopes are frequently smaller in size than 15 amino acid residues. From the foregoing, it is advantageous, but not essential, that the size of the peptide is at least 6, 7, 8, 9,10,11,12,13, 14, 15, 20, 25, 30 amino acid residues. Suitably, the size of the peptide is no more than about 500, 200, 100, 80, 60, 50, 40 amino acid residues. In one embodiment, the size of the peptide is large enough to minimise loss of T cell and/or B cell epitopes. In another embodiment, the size of the peptide is sufficient for presentation by an antigen-presenting cell of a T cell and/or a B cell epitope contained within the peptide. In one example of this embodiment, the size of the peptide is about 15 amino acid residues.

The polypeptide of interest is suitably a disease- or condition-associated antigen, which may be selected from endogenous antigens produced by an individual or exogenous antigens that are foreign to the individual. Suitable endogenous antigens include, but are not restricted to, self-antigens that are targets of autoimmune responses as well as cancer or tumour antigens. Illustrative examples of self antigens useful in the treatment or prevention of autoimmune disorders include, but not limited to, diabetes mellitus, arthritis (including rheumatoid arthritis, juvenile rheumatoid arthritis, ostecarthritis, psoriasic arthritis), multiple sclerosis, myasthenia gravis, systemic lupus erythematosis, autoimmune thyroiditis, dermatitis (including atopic dermatitis and eczematous dermatitis), psoriasis, Sjögren's Syndrome, including keratoconjunctivitis sicca secondary to Sjögren's Syndrome, alopecia areata, allergic responses due to arthropod bite reactions, Crohn's disease, ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing haemorrhagic encephalopathy, idiopathic bilateral progressive sensorineural hearing loss, aplastic anaemia, pure red cell anaemia, idiopathic thrombocytopenia, polychondritis, Wegener's granulomatosis, chronic active hepatitis, Stevens-Johnson syndrome, idiopathic sprue, lichen planus, Graves ophthalmopathy, sarcoidosis, primary biliary cirrhosis, uveitis posterior, and interstitial lung fibrosis. Other autoantigens include those derived from nucleosomes for the treatment of systemic lupus erythematosus (e.g., GenBank Accession No. D28394; Bruggen et al., 1996, Ann, Med, Interne (Paris), 147:485-489) and from the 44,000 Da peptide component of ocular tissue cross-reactive with *O. volvulus* antigen (McKeclmie et αl., 1993, Ann Trop. Med. Parasitol. 87:649-652). Thus, illustrative autoantigens antigens that can be used in the compositions and methods of the present invention include, but are not limited to, at least a portion of a lupus autoantigen, Smith, Ro, La, UI-RNP, fibrillin (scleroderma), pancreatic β cell antigens, GAD65 (diabetes related), insulin, myelin basic protein, myelin proteolipid protein, histones, PLP, collagen, glucose-6-phosphate isomerase, citrullinated proteins and peptides, thyroid antigens, thyroglobulin, thyroid-stimulating hormone (TSH) receptor, various tRNA synthetases, components of the acetyl choline receptor (AchR), MOG, proteinase-3, myeloperoxidase, epidermal cadherin, acetyl choline receptor, platelet antigens, nucleic acids, nucleic acid:protein complexes, joint antigens, antigens of the nervous system, salivary gland proteins, skin antigens, kidney antigens, heart antigens, lung antigens, eye antigens, erythrocyte antigens, liver antigens and stomach antigens.

Non-limiting examples of cancer or tumour antigens include antigens from a cancer or tumour selected from ABL1 protooncogene, AIDS Related Cancers, Acoustic Neuroma, Acute Lymphocytic Leukaemia, Acute Myeloid Leukaemia, Adenocystic carcinoma, Adrenocortical Cancer, Agnogenic myeloid metaplasia, Alopecia, Alveolar soft-part sarcoma, Anal cancer, Angiosarcoma, Aplastic Anaemia, Astrocytoma, Ataxia-telangiectasia, Basal Cell Carcinoma (Skin), Bladder Cancer, Bone Cancers, Bowel cancer, Brain Stem Glioma, Brain and CNS Tumours, Breast Cancer, CNS tumours, Carcinoid Tumours, Cervical Cancer, Childhood Brain Tumours, Childhood Cancer, Childhood Leukaemia, Childhood Soft Tissue Sarcoma, Chondrosarcoma, Choriocarcinoma, Chronic Lymphocytic Leukaemia, Chronic Myeloid Leukaemia, Colorectal Cancers, Cutaneous T-cell Lymphoma, Dermatofibrosarcoma-protuberans, Desmoplastic-Small-Round-Cell-Tumour, Ductal Carcinoma, Endocrine Cancers, Endometrial Cancer, Ependymoma, Esophageal Cancer, Ewing's Sarcoma, Extra-Hepatic Bile Duct Cancer, Eye Cancer, Eye: Melanoma, Retinoblastoma, Fallopian Tube cancer, Fanconi Anaemia, Fibrosarcoma, Gall Bladder Cancer, Gastric Cancer, Gastrointestinal Cancers, Gastrointestinal-Carcinoid-Tumour, Genitourinary Cancers, Germ Cell Tumours, Gestational-Trophoblastic-Disease, Glioma, Gynaecological Cancers, Haematological Malignancies, Hairy Cell Leukaemia, Head and Neck Cancer, Hepatocellular Cancer, Hereditary Breast Cancer, Histiocytosis, Hodgkin's Disease, Human Papillomavirus, Hydatidiform mole, Hypercalcemia, Hypopharynx Cancer, Intraocular Melanoma, Islet cell cancer, Kaposi's sarcoma, Kidney Cancer, Langerhan's-Cell-Histlocytosis, Laryngeal Cancer, Leiomyosarcoma, Leukaemia, Li-Fraumeni Syndrome, Lip Cancer, Liposarcoma, Liver Cancer, Lung Cancer, Lymphedema, Lymphoma, Hodgkin's Lymphoma, Non-Hodgkin's Lymphoma, Male Breast Cancer, Malignant-Rhabdoid-Tumour-of-Kidney, Medulloblastoma, Melanoma, Merkel Cell Cancer, Mesothelioma, Metastatic Cancer, Mouth Cancer, Multiple Endocrine Neoplasia, Mycosis Fungoides, Myelodysplastic Syndromes, Myeloma, Myeloproliferative Disorders, Nasal Cancer, Nasopharyngeal Cancer, Nephroblastoma, Neuroblastoma, Neurofibromatosis, Nijmegen Breakage Syndrome, Non-Melanoma Skin Cancer, Non-Small-Cell-Lung-Cancer-(NSCLC), Ocular Cancers, Oesophageal Cancer, Oral cavity Cancer, Oropharynx Cancer, Osteosarcoma, Ostomy Ovarian Cancer, Pancreas Cancer, Paranasal Cancer, Parathyroid Cancer, Parotid Gland Cancer, Penile Cancer, Peripheral-Neuroectodcrmal-Tumours, Pituitary Cancer, Polycythemia vera, Prostate Cancer, Rare-cancers-and-associated-disorders, Renal Cell Carcinoma, Retinoblastoma, Rhabdomyosarcoma, Rothmund-Thomson Syndrome, Salivary Gland Cancer, Sarcoma, Schwannoma, Sezary syndrome, Skin Cancer, Small Cell Lung Cancer (SCLC), Small Intestine Cancer, Soft Tissue Sarcoma, Spinal Cord Tumours, Squamous-Ceii-Carcinoma-(skin), Stomach Cancer, Synovial sarcoma, Testicular Cancer, Thymus Cancer, Thyroid Cancer, Transitional-Cell-Cancer-(bladder), Transitional-Cell-Cancer-(renal-pelvis-/- ureter), Trophoblastic Cancer, Urethral Cancer, Urinary System Cancer, Uroplakins, Uterine sarcoma, Uterus Cancer, Vaginal Cancer, Vulva Cancer, Waldenstrom's-Macroglobulinemia, Wilms' Tumour. In certain embodiments, the cancer or tumour relates to melanoma. Illustrative examples of melanoma-related antigens include melanocyte differentiation antigen (e.g., gp100, MART, TRP-1, Tyros, TRP2, MC1R, MUC1F, MUC1R or a combination thereof) and melanoma-specific antigens (e.g., BAGE, GAGE-1, gpl00In4, MAGE-1 (e.g., GenBank Accession No. X54I56 and AA494311), MAGE-3, MAGE4, FRAME, TRP2IN2, NYNSO1a, NYNSO1b, LAGE1, p97 melanoma antigen (e.g., GenBank Accession No. M12154) or a combination thereof. Other tumour-specific antigens include the Ras peptide and p53 peptide associated with advanced cancers, MUCl-KLH antigen associated with breast carcinoma (e.g., GenBank Accession No. J03651), CEA (carcinoembryonic antigen) associated with colorectal cancer (e.g., GenBank Accession No. X98311), gp100 (e.g., GenBank Accession No. 573003) and the PSA antigen with prostate cancer (e.g., GenBank Accession No. X14810). The p53 gene sequence is known (See e.g., Harris et. αl., 1986 Mol, Cell. Biol. 6:4650-4656) and is deposited with GenBank under Accession No. M14694.

Foreign antigens are suitably selected from transplantation antigens, allergens as well as antigens from pathogenic organisms. Transplantation antigens can be derived from donor cells or tissues from e.g., heart, lung, liver, pancreas, kidney, neural graft components, or from the donor antigen-presenting cells bearing MHC loaded with self antigen in the absence of exogenous antigen.

Non-limiting examples of allergens include Fel d 1 (i.e., the feline skin and salivary gland allergen of the domestic cat *Felis domestics,* the amino acid sequence of which is disclosed International Publication WO 91/06571), Der p I, Der p II, Der fI or Der fII (i.e., the major protein allergens from the house dust mite dermatophagoides, the amino acid sequence of which is disclosed in International Publication WO 94/24281). Other allergens may be derived, for example from the following: grass, tree and weed (including ragweed) pollens; fungi and moulds; foods such as fish, shellfish, crab, lobster, peanuts, nuts, wheat gluten, eggs and milk; stinging insects such as bee, wasp, and hornet and the chirnomidae (non-biting midges); other insects such as the housefly, fruitfly, sheep blow fly, screw worm fly, grain weevil, silkworm, honeybee, non-biting midge larvae, bee moth larvae, mealworm, cockroach and larvae of *Tenibrio molitor* beetle; spiders and mites, including the house dust mite; allergens found in the dander, urine, saliva, blood or other bodily fluid of mammals such as cat, dog, cow, pig, sheep, horse, rabbit, rat, guinea pig, mouse and gerbil; airborne particulates in general; latex; and protein detergent additives.

Exemplary pathogenic organisms include, but are not limited to, viruses, bacteria, fungi parasites, algae and protozoa and amoebeae. Illustrative examples of viruses include viruses responsible for diseases including, but not limited to, measles, mumps, rubella, poliomyelitis, hepatitis A, B (e.g., GenBank Accession No. E02707), and C (e.g., GenBank Accession No. E06890), as well as other hepatitis viruses, influenza, adenovirus (e.g., types 4 and 7), rabies (e.g., GenBank Accession No. M34678), yellow fever, Epstein-Barr virus and other herpesviruses such as papillomavirus, Ebola virus, influenza virus, Japanese encephalitis (e.g., GenBank Accession No. E07883), dengue (e.g., GenBank Accession No. M24444), hantavirus, sendai virus, respiratory syncytial virus, othromyxoviruses, vesicular stomatitis virus, visna virus, cytomegalovirus and human immunodeficiency virus (HIV) (e.g., GenBank Accession No. U18552). Any suitable antigen derived from such viruses are useful in the practice of the present invention. For example, illustrative retroviral antigens derived from HIV include, but are not limited to, antigens such as gene products of the *gag, pol,* and *env* genes, the Nef protein, reverse transcriptase, and other HIV components. Illustrative examples of hepatitis viral antigens include, but are not limited to, antigens such as the S, M, and L proteins of hepatitis B virus, the pre-S antigen of hepatitis B virus, and other hepatitis, e.g., hepatitis A, B, and C, viral components such as hepatitis C viral RNA. Illustrative examples of influenza viral antigens include; but are not limited to, antigens such as hemagglutinin and neuraminidase and other influenza viral components. Illustrative examples of measles viral antigens include, but are not limited to, antigens such as the measles virus fusion protein and other measles virus components. Illustrative examples of rubella viral antigens include, but are not limited to, antigens such as proteins E1 and E2 and other rubella virus components; rotaviral antigens such as VP7se and other rotaviral components. Illustrative examples of cytomegaloviral antigens include, but are not limited to, antigens such as envelope glycoprotein B and other cytomegaloviral antigen components. Non-limiting examples of respiratory syncytial viral antigens include antigens such as the RSV fusion protein, the M2 protein and other respiratory syncytial viral antigen components. Illustrative examples of herpes simplex viral, antigens include, but are not limited to, antigens such as immediate early proteins, glycoprotein D, and other herpes simplex viral antigen components. Non-limiting examples of varicella zoster viral antigens include antigens such as 9PI, gpII, and other varicella zoster viral antigen components. Non-limiting examples of Japanese encephalitis viral antigens include antigens such as proteins E, M-E, M-E-NS 1, NS 1, NS 1-NS2A, 80%E, and other Japanese encephalitis viral antigen components. Illustrative examples of rabies viral antigens include, but are not limited to, antigens such as rabies glycoprotein, rabies nucleoprotein and other rabies viral antigen components. Illustrative examples of papillomavirus antigens include, but are not limited to, the L1 and L2 capsid proteins as well as the E6/E7 antigens associated with cervical cancers, See Fundamental Virology, Second Edition, eds. Fields, B.N. and Knipe, D.M., 1991, Raven Press, New York, for additional examples of viral antigens.

Illustrative examples of fungi include *Acremonium* spp., *Aspergillus* spp., *Basidiobolus* spp., *Bipolaris* spp., *Blastomyces dermatidis, Candida spp., Cladophialophora carrionii, Coccoidiodes immitis, Conidiobolus* spp., *Cryptococcus* spp., *Curvularia* spp., *Epidermophyton* spp., *Exophiala jeanselmei, Exserohilum* spp., *Fonsecaea compacta, Fonsecaea pedrosoi, Fusarium oxysporum, Fusarium solani, Geotrichum candidum, Histoplasma capsulatum* var. *capsulatum, Histoplasma capsulatum* var. *duboisii, Hortaea werneckii, Lacazia loboi, Lasiodiplodia theobromae, Leptosphaeria senegalensis, Madurella grisea, Madurella mycetomatis, Malassezia furfur, Microsporum* spp., *Neotestudina rosatii, Onychocola canadensis, Paracoccidioides brasiliensis, Phialophora verrucosa, Piedraia hortae, Piedra iahortae, Pityriasis versicolor, Pseudallesheria boydii, Pyrenochaeta romeroi, Rhizopus arrhizus, Scopulariopsis brevicaulis, Scytalidium dimidiatum, Sporothrix schenckii, Trichophyton* spp., *Trichosporon* spp., Zygomcete fungi, *Absidia corymbifera, Rhizomucor pusillus* and *Rhizopus arrhizus.* Thus, illustrative fungal antigens that can be used in the compositions and methods of the present invention include, but are not limited to, candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.

Illustrative examples of bacteria include bacteria that are responsible for diseases including, but not restricted to, diphtheria (e.g., *Corynebacterium diphtheria*), pertussis (e.g., *Bordetella pertussis,* CenBank Accession No. M35274), tetanus (e.g., *Clostridium tetani,* GenBank Accession No. M64353), tuberculosis (e.g., *Mycobacterium tuberculosis),* bacterial pneumonias (e.g., *Haemophilus influenzae.),* cholera (e.g., *Vibrio cholerae),* anthrax (e.g., *Bacillus anthracis),* typhoid, plague, shigellosis (e.g., *Shigella dysenteriae),* botulism (e.g., *Clostridium botulinum),* salmonellosis (e.g., GenBank Accession No. L03833), peptic ulcers (e.g., *Helicobacter pylori),* Legionnaire's Disease, Lyme disease (e,g., GenBank Accession No. U59487), Other pathogenic bacteria include *Escherichia coli, Clostridium perfringens, Pseudomonas aeruginosa, Staphylococcus aureus* and *Streptococcus pyogenes,* Thus, bacterial antigens which can be used in the compositions and methods of the invention include, but are not limited to: pertussis bacterial antigens such as pertussis toxin, filamentous hemagglutinin, pertactin, F M2, FIM3, adenylate cyclase and other pertussis bacterial antigen components; diphtheria bacterial antigens such as diphtheria toxin or toxoid and other diphtheria bacterial antigen components; tetanus bacterial antigens such as tetanus toxin or toxoid and other tetanus bacterial antigen components, streptococcal bacterial antigens such as M proteins and other streptococcal bacterial antigen components; gram-negative bacilli bacterial antigens such as lipopolysaccharides and other gram-negative bacterial antigen components; *Mycobacterium tuberculosis* bacterial antigens such as mycolic acid, heat shock protein 65 (HSP65), the 30kDa major secreted protein, antigen 85A and other mycobacterial antigen components; *Helicobacter pylori* bacterial antigen components, pneumococcal bacterial antigens such as pneumolysin, pneumococcal capsular polysaccharides and other pnermiococcal bacterial antigen components; *Haemophilus influenza* bacterial antigens such as capsular polysaccharides and other *Haemophilus influenza* bacterial antigen components; antbrax bacterial antigens such as anthrax protective antigen and other anthrax bacterial antigen components; rickettsiae bacterial antigens such as rompA and other rickettsiae bacterial antigen component. Also included with the bacterial antigens described herein are any other bacterial, mycobacterial, mycoplasmal, rickettsial, or chlamydial antigens.

Illustrative examples of protozoa include protozoa that are responsible for diseases including, but not limited to, malaria (e.g., GenBank Accession No. X53832), hookworm, onchocerciasis (e.g., GenBank Accession No. M27807), schistosomiasis (e.g., GenBank Accession No. LOS 198), toxoplasmosis, trypanosomiasis, leishmaniasis, giardiasis (GenBank Accession No. M33641), amoebiasis, filariasis (e.g., GenBank Accession No. J03266), borreliosis, and trichinosis. Thus, protozoal antigens which can be used in the compositions and methods of the invention include, but are not limited to: plasmodium falciparum antigens such as merozoite surface antigens, sporozoite surface antigens, circumsporozoite antigens, gametocyte/gamete surface antigens, blood-stage antigen pf 155/RESA and other plasmodial antigen components; toxoplasma antigens such as SAG-1, p30 and other toxoplasmal antigen components; schistosomae antigens such as glutathione-S-transferase, paramyosin, and other schistosomal antigen components; leishmania major and other leishmaniae antigens such as gp63, lipophosphoglycan and its associated protein and other leishmanial antigen components; and trypanosoma cruzi antigens such as the 75-77kDa antigen, the 56kDa antigen and other trypanosomal antigen components.

The present invention also contemplates toxin components as antigens. Illustrative examples of toxins include, but are not restricted to, staphylococcal enterotoxins, toxic shock syndrome toxin; retroviral antigens (e.g., antigens derived from HIV), streptococcal antigens, staphylococcal enterotoxin-A (SEA), staphylococcal enterotoxin-B (SEB), staphylococcal enterotoxin₁₋₃ (SE₁₋₃), staphylococcal enterotoxin-D (SED), staphylococcal enterotoxin-E (SEE) as well as toxins derived from mycoplasma, mycobacterium, and herpes viruses.

In one example of the present invention, the size of individual peptides is about 14 or 15 amino acid residues and the sequence overlap at one or both ends of an individual peptide is about 11 amino acid residues. However, it will be understood that other suitable peptide sizes and sequence overlap sizes are contemplated by the present invention, which can be readily ascertained by persons of skill in the art.

It is advantageous but not necessary to utilise the entire sequence of a polypeptide of interest for producing a set of overlapping peptides. Typically, at least 30%, 40%, 50%, 60%, 70%, 80% 90%, 91%, 92%, 93%, 94%, 95%, 96%, 97%, 98%, 99% of the sequence corresponding to a polypeptide of interest is used to produce the overlapping peptides of the invention. However, it will be understood that the more sequence information from a polypeptide of interest that is utilised to produce the overlapping peptides, the greater the outbred population coverage will be of the overlapping peptides as an immunogen. Suitably, no sequence information from the polypeptide of interest is excluded (e.g., because of an apparent lack of immunological epitopes, since more rare or subdominant epitopes may be inadvertently missed). If required, hypervariable sequences within a polypeptide of interest can be either excluded from the construction of an overlapping set of peptides, or additional sets of peptides covering the polymorphic regions can be constructed and administered.. Peptide sequences may include additional sequences that are not derived from a polypeptide of interest. These additional sequences may have various functions, including improving solubility, stability or immunogenicity or facilitating purification. Typically, such additional sequences are contained at one or both ends of a respective peptide.

Persons of skill in the art will appreciate that when preparing a set of overlapping peptides according to the invention, it may be advantageous to use sequence information from a plurality of different polypeptides produced by a pathogenic organism or expressed in a cancer. Accordingly, in certain embodiments, at least 2, 3, 4, 5, 6, 7, 9, 10, 15, 20 other sets of peptides are used for the production of the immunomodulating compositions of the invention, wherein the sequences of a respective other set of peptides are derived from a distinct polypeptide of interest and wherein individual peptides of the respective other set display partial sequence identity or similarity to at least one other peptide of a corresponding set of peptides. It is advantageous in this respect to utilise as many polypeptides as possible from, or in relation to, a particular source in the construction of sets of overlapping peptides. Suitably, at least about 30%, 40%, 50%, 60%, 70%, 80% 90%, 91%, 92%, 93%, 94%, 95%, 96%, 97%, 98%, 99%, and desirably 100%, of the polypeptides expressed by the source is used in the construction of the corresponding sets of overlapping peptides. Exemplary viral polypeptides that can be used for such construction include, but are not restricted to, latent polypeptides, regulatory polypeptides or polypeptides expressed early during their replication cycle. Suitably, polypeptides from a protozoan, bacterium, mycoplasma, fungus or helminth include, but are not restricted to, secretory polypeptides, regulatory polypeptides and polypeptides expressed on the surface of these organisms. Polypeptides from a cancer or tumour, which can be used for the construction of overlapping peptide sets, are suitably cancer-specific polypeptides.

Representative overlapping peptide sets for modulating the immune response to simian immunodeficiency virus (SIV) and/or the chimeric SIV-HIV-1 (SHIV), both of which are known to be suitable models for the pathogenic HIV-1 virus in humans, can be based on one or more polypeptides selected from SIV gag, pol, nef or SHIV env as for example presented in Tables 1 to 4, Illustrative overlapping peptide sets for modulating the immune response to HIV-1 can be based on one or more polypeptides selected from HIV Gag, Nef, Pol, Rev, Tat, Vif, Vpr and Vpu as for example set forth in Tables 5 to 12. An illustrative overlapping peptide set for modulating the immune response to HCV 1a can be based on the HCV 1a H77 polyprotein sequence as for example set forth in Table 13. An illustrative overlapping peptide set for modulating the immune response to HBV Genotype A can be based on all proteins expressed by this genotype and on some portions of proteins expressed from Genotypes B/C/D, which display significant variability from Genotype A sequence, as for example set forth in Table 14.

The overlapping peptide sets of the invention may be prepared by any suitable procedure known to those of skill in the art. For example, the peptide sets can be synthesised conveniently using solution synthesis or solid phase synthesis as described, for example, in Chapter 9 of Atherton and Shephard (1989, Solid Phase Peptide Synthesis: A Practical Approach. IRL Press, Oxford) and in Roberge *et al* (1995, *Science* **269**: 202). Syntheses may employ, for example, either *t-*butyloxycarbonyl (*t*-Boc) or 9-fluorenylmethyloxycarbonyl (Fmoc) chemistries (see Chapter 9.1, of Coligan *et al.,* CURRENT PROTOCOLS IN PROTEIN SCIENCE, John Wiley & Sons, Inc. 1995-1997; Stewart and Young, 1984, Solid Phase Peptide Synthesis, 2nd ed. Pierce Chemical Co., Rockford, III; and Atherton and Shephard, *supra),* In specific embodiments, the individual peptides are solubilized in DMSO (e.g., 100% pure DMSO) at high concentration (1 mg peptide/10-30 µL DMSO) so that large pools of peptides do not contain excessive amounts of DMSO when pulsed onto cells. In certain advantageous embodiments, one or more peptide sets of the invention, in soluble form, are placed into a single container for convenient administration (e.g. a blood tube or vial for ready re-infusion) to a subject and such containers are also contemplated by the present invention,

Alternatively, individual peptides may be prepared by a procedure including the steps of: (a) preparing a synthetic construct including a synthetic polynucleotide encoding an individual peptide of an overlapping set of peptides, wherein the synthetic polynucleotide is operably linked to a regulatory polynucleotide; (b) introducing the synthetic construct into a suitable host cell; (c) culturing the host cell to express the synthetic polynucleotide; and (d) isolating the individual peptide. The synthetic construct is preferably in the form of an expression vector. For example, the expression vector can be a self-replicating extra-chromosomal vector such as a plasmid, or a vector that integrates into a host genome. Typically, the regulatory polynucleotide includes, but is not limited to, promoter sequences, leader or signal sequences, ribosomal binding sites, transcriptional start and stop sequences, translational start and termination sequences, and enhancer or activator sequences. Constitutive or inducible promoters as known in the art are contemplated by the invention. The promoters may be either naturally occurring promoters, or hybrid promoters that combine elements of more than one promoter. The regulatory polynucleotide will generally be appropriate for the host cell used for expression. Numerous types of appropriate expression vectors and suitable regulatory polynucleotides are known in the art for a variety of host cells. In certain embodiments, the expression vector contains a selectable marker gene to allow the selection of transformed host cells. Selection genes are well known in the art and will vary with the host cell used. In other embodiments, the expression vector also includes a nucleic acid sequence that codes for a fusion partner so that an individual peptide is expressed as a fusion polypeptide with the fusion partner. The main advantage of fusion partners is that they assist identification and/or purification of the fusion polypeptide. Exemplary fusion partners include, but are not limited to, glutathione-S-transferase (GST), Fc portion of human IgG, maltose binding protein (MBP) and hexahistidine (HIS₆), which are particularly useful for isolation of the fusion polypeptide by affinity chromatography, For the purposes of fusion polypeptide purification by affinity chromatography, relevant matrices for affinity chromatography are glutathione-, amylose-, and nickel- or cobalt-conjugated resins respectively. Many such matrices are available in "kit" form, such as the QIAexpress™ system (Qiagen) useful with (HIS₆) fusion partners and the Pharmacia GST purification system, In a preferred embodiment, the recombinant polynucleotide is expressed in the commercial vector pFLAG™. Advantageously, the fusion partners also have protease cleavage sites, such as for Factor X_{E}, Thrombin and inteins (protein introns), which allow the relevant protease to partially digest the fusion polypeptide of the invention and thereby liberate the recombinant polypeptide of the invention therefrom. The liberated peptide can then be isolated from the fusion partner by subsequent chromatographic separation. Fusion partners according to the invention also include within their scope "epitope tags", which are usually short peptide sequences for which a specific antibody is available. Well known examples of epitope tags for which specific monoclonal antibodies are readily available include c-Myc, influenza virus, haemagglutinin and FLAG tags.

The step of introducing the synthetic construct into the host cell may be achieved using any suitable technique including transfection, and transformation, the choice of which will be dependent on the host cell employed. Such methods are well known to those of skill in the art. The peptides of the invention may be produced by culturing a host cell transformed with the synthetic construct. The conditions appropriate for protein expression will vary with the choice of expression vector and the host cell. This is easily ascertained by one skilled in the art through routine experimentation. Suitable host cells for expression may be prokaryotic or eukaryotic. One preferred host cell for expression of a polypeptide according to the invention is a bacterium. The bacterium used may be *Escherichia coli.* Alternatively, the host cell may be an insect cell such as, for example, *SF9* cells that may be utilised with a baculovirus expression system.

The amino acids of the peptides can be any non-naturally occurring or any naturally occurring amino acid. Examples of unnatural amino acids and derivatives during peptide synthesis include but are not limited to, use of 4-amino butyric acid, 6-aminohexanoic acid, 4-amino-3-hydroxy-5-phenylpentanoic acid, 4-amino-3-hydroxy-6-methylheptanoic acid, t-butylglycine, norleucine, norvaline, phenylglycine, ornithine, sarcosine, 2-thienyl alanine and/or D-isomers of amino acids. A list of unnatural amino acids contemplated by the present invention is shown in TABLE B.

**TABLE B**

| Non-conventional amino acid | Non-conventional amino acid. |
|---|---|
| α-aminobutyric acid | L-N-methylalanine |
| α-amino-α-methylbutyrate | L-N-metlylarginine |
| aminocyclopropane-carboxylate | L-N-methylasparagine |
| aminoisobutyric acid | L-N-methylaspartic acid |
| aminonorbornyl-carboxylate | L-N-methylcysteine |
| cyclohexylalanine | L-N-methylglutamine |
| cyclopentylalanine | L-N-methylglutamic acid |
| L-N-methylisoleucine | L-N-methylhistidine |
| D-alanine | L-N-mefhylleucine |
| D-arginine | L-N-methyllysine |
| D-aspartic acid | L-N-methylmethionine |
| D-cysteine | L-N-methylnorleucine |
| D-glutamate | L-N-methylnorvaline |
| D-glutamic acid | L-N-methylornithine |
| D-histidine | L-N-methylphenylalanine |
| D-isoleucine | L-N-methylproline |
| D-leucine | L-N-medlylserine |
| D-lysine | L-N-methylthteonine |
| D-methionine | L-N-methyltryptophan |
| D-ornithine | L-N-methyltyrosine |
| D-phenylalanine | L-N-methylvaline |
| D-proline | L-N-methylethylglycine |
| D-serine | L-N-methyl-t-butylglycine, |
| D-threonine | L-norleucine |
| D-tryptophan | L-norvaline |
| D-tyrosine | α-methyl-aminoisobutyrate |
| D-valine | α-methyl-γ-aminobutyrate |
| D-α-methylalanine | α-methylcyolohexylalanine |
| D-α-methylarginine | α-methylcylcopentylalanine |
| D-α-methylasparagine | α-methyl-α-napthylalanine |
| D-α-methylaspartate | α-methylpenicillamine |
| D-α-methylcysteine | N-(4-aminobutyl)glycine |
| D-α-methylglutamine | N-(2-aminoethyl)glycine |
| D-α-methylhistidine | N-(3-aminoprapyl)glycine |
| D-α-methylisoleucine | N-amino-α-methylbutyrate |
| D-α-methylleucine | α-napthylalanine |
| D-α-methyllysine | N-benzylgiycine |
| D-α-methylmethionine | N-(2-carbamylediyl)glycine |
| D-α-methylornithiine | N-(carbamylmethyl)glycine |
| D-α-methylphenylalanine | N-(2-carboxyethyl)glycine |
| D-α-methylproline | N-(carboxymethyl)glycine |
| D-α-methylserine | N-cyclobutylglycine |
| D-α-methylthreonine | N-cycloheptylglycine |
| D-α-methyltryptophan | N-cyclohexylglycine |
| D-α-methyltyrosine | N-cyclodecylglycine |
| L-α-methylleucine | L-α-methyllysine |
| L-α-methylmethionine | L-α-methylnorleucine |
| L-α-methylnorvatine | L-α-methylornithine |
| L-α-methylphenylalanine | L-α-methylproline |
| L-α-methylserine | L-α-methylthreonine |
| L-α-methyltryptophan | L-α-methyltyrosine |
| L-α-methylvaline | L-N-methylhomophenylalanine |
| N-(N-(2,2-diphenylethyl | N-(N-(3,3-diphenylpropyl |
| carbamylmethyl)glycine | carbamylmethyl)glycine |
| 1-carboxy-1-(2,2-dipbenyl-ethyl | |
| amino)cyclopropane | |

The invention also contemplates modifying the peptides of the invention using ordinary molecular biological techniques so as to alter their resistance to proteolytic degradation or to optimise solubility properties or to render them more suitable as an immunogenic agent.

### 3. Antigen-presenting cell embodiments

The present invention also discloses the discovery that antigen-presenting cells which have been contacted with overlapping peptide sets as described in Section 2 are potent modulators of immune responses and are especially useful for raising strong immunogenic responses that can prevent or ameliorate the symptoms of a disease or condition of interest. Accordingly, the invention provides a process for producing antigen-specific antigen-presenting cells, comprising contacting antigen-presenting cells or their precursors with one or more sets of peptides as broadly described above for a time and under conditions sufficient for the peptides or processed forms thereof to be presented by the antigen-presenting cells or their precursors, and in the case of precursors, culturing the precursors for a time and under conditions sufficient to differentiate antigen-presenting cells from the precursors.

The present inventors have also found unexpectedly that, in contrast to current dogma, it is not necessary to culture or activate purified antigen-presenting cells to increase their number or efficiency before loading them with antigen for effective modulation of an immune response to the antigen in a recipient of those cells. Instead, the present inventors have discovered that an uncultured population of antigen-presenting cells or their precursors, which have not been subjected to activating conditions, when contacted with an antigen that corresponds to a target antigen of interest is sufficient to effectively modulate an immune response to the target antigen in a recipient of the contacted population. Accordingly, in another aspect, the present invention provides a process for producing antigen-specific antigen-presenting cells, comprising contacting an uncultured population of antigen-presenting cells or their precursors, which have not been subjected to activating conditions, with an antigen corresponding to the target antigen for a time and under conditions sufficient for the antigen-presenting cells or their precursors to express a processed or modified form of the antigen. Illustrative examples of the uncultured population of antigen-presenting cells or their precursors include whole blood, fresh blood, or fractions thereof such as but not limited to peripheral blood mononuclear cells (PMBC), buffy coat fractions of whole blood, packed red cells, irradiated blood, dendritic cells, monocytes, macrophages, neutrophils, lymphocytes, natural killer cells and natural killer T cells. In specific embodiments, the uncultured population of antigen-presenting cells is selected from freshly isolated blood or PMBC. In other embodiments, the uncultured population of antigen-presenting cells is a necrotic or apoptotic population. Thus, the uncultured population of cells may be contacted with antigen and subsequently subjected to necrotic conditions, which lead to irreversible trauma to cells (e.g., osmotic shock or exposure to chemical poison such as glutaraldehyde), wherein the cells are characterised by marked swelling of the mitochondria and cytoplasm, followed by cell destruction and autolysis. Alternatively, the uncultured cell population is subjected may be contacted with antigen and subsequently subjected to apoptotic conditions.Cells expressing or presenting antigen can be induced to undergo apoptosis *in vitro* or *in vivo* using a variety of methods known in the art including, but not limited to, viral infection, irradiation with ultraviolet light, gamma radiation, steroids, fixing (e.g., with glutaraldehyde), cytokines or by depriving donor cells of nutrient's in the cell culture medium. Time course studies can establish incubation periods sufficient for optimal induction of apoptosis in a population of cells. For example, monocytes infected with influenza virus begin to express early markers for apoptosis by 6 hours after infection. Examples of specific markers for apoptosis include Annexin V, TUNEL+ cells, DNA laddering and uptake of propidium iodide.

According to this aspect of the present invention, the antigen used to contact the population is not limited to the overlapping set of peptides described in Section 2 above but instead encompasses antigens of any biological type including, for example, simple intermediary metabolites, sugars, lipids, and hormones as well as macromolecules such as complex carbohydrates, phospholipids, nucleic acid molecules and proteinaceous molecules. In illustrative examples, the antigen corresponding to the target antigen is selected from whole protein antigens, cellular material (e,g., live or inactivated cancer cells), particulate matter such as, but not limited to, cell debris, apoptotic cells, lipid aggregates such as liposomes, membranous vehicles, microspheres, heat aggregated proteins, virosomes, virus-like particles and whole organisms including, for example, bacteria, mycobacteria, viruses, fungi, protozoa or parts thereof.

Target antigens may be selected from endogenous antigens produced by a host or exogenous antigens that are foreign to the host, as described for example in Section 2. In certain embodiments, the antigen corresponding to the target antigen is a proteinaceous antigen. Such antigens may be isolated from a natural source or may be prepared by recombinant techniques as known in the art. Alternatively, crude antigen preparations can be produced by isolating a sample of a cell population or tissue for which a modified immune response is desired, and either lysing the sample or subjecting the sample to conditions that will lead to the formation of apoptotic cells (e.g., irradiation with ultra violet or with gamma rays, viral infection, cytokines or by depriving cells of nutrients in the cell culture medium, incubation with hydrogen peroxide, or with drugs such as dexamethasone, ceramide chemotherapeutics and anti-hormonal agents such as Lupron™ or Tamoxifen™). The lysate or the apoptotic cells can then be used as a source of crude antigen for use in soluble form or for contact with antigen-presenting cells as described in more detail below.

### 3.1 Sources of antigen-presenting cells

The antigen-presenting cells suitably encompass both professional and facultative types of antigen-presenting cells. For example, professional antigen-presenting cells include, but are not limited to, macrophages, monocytes, cells of myeloid lineage, including monocytic-granulocytic-DC precursors, marginal zone Kupffer cells, microglia, T cells, B cells Langerhans cells and dendritic cells including interdigitating dendritic cells and follicular dendritic cells. Examples of facultative antigen-presenting cells include but are not limited to activated T cells, astrocytes, follicular cells, endothelium and fibroblasts. In a preferred embodiment, the antigen-presenting cells are selected from monocytes, macrophages, cells of myeloid lineage, dendritic cells or Langerhans cells.

Antigen-presenting cells or their precursors can be isolated by methods known to those of skill in the art. The source of antigen-presenting cell or precursor may differ depending upon the antigen-presenting cell required for modulating a specified immune response. In this context, the antigen-presenting cell can be selected from dendritic cells, macrophages, monocytes and other cells of myeloid lineage. Typically, precursors of antigen-presenting cells can be isolated from any tissue, but are most easily isolated from blood, cord blood or bone marrow (Sorg et al., 2001, Exp Hematol 29: 1289-1294; Zheng et al., 2000, J Hematother Stem Cell Res 9: 453-464), It is also possible to obtain suitable precursors from diseased tissues such as rheumatoid synovial tissue or fluid following biopsy or joint tap (Thomas et al., 1994, J Immunol 152: 2613-2623; Thomas et al., 1994, J Immunol 153: 4016-4028). Other examples include, but are not limited to liver, spleen, heart, kidney, gut and tonsil (Lu et al., 1994, Transplantation 64: 1808-1815; McIlroy et al., 2001, Blood 97: 3470-3477; Vremec et al., 2000, J Immunol 164: 2978-2986; Hart and Fabre, 1981, J Exp Med 154(2): 347-361; Hart and McKenzie, 1988, J Exp Med 168(1): 157-170; Pavli et al., 1990, Immunology 70(1): 40-47).

Leukocytes isolated directly from tissue provide a major source of antigen-presenting cell precursors. Typically, these precursors can only differentiate into antigen-presenting cells by culturing in the presence or absence of various growth factors *ex vivo* for at least about 6-9 days. However, in some advantageous embodiments of the present invention, antigen-presenting cells or their precursors (e.g., in the form of freshly isolated blood or PMBC) are simply isolated from an individual and incubated in the presence of antigen and preferably one or more growth factors for much shorter periods, e.g., less than about 48, 36, 24, 12, 8, 7, 6, 5, 4, 3 or 2 hours or even less that about 60, 50,40, 30, 20, 15, 10, 9, 8, 7, 6, 5, 4, 3 or 2 minutes, to produce antigen-specific antigen-presenting cells that are effective in raising an immunogenic response to that antigen.

In some embodiments, antigen-presenting cell precursors may be differentiated from crude mixtures or from partially or substantially purified preparations of precursors. Leukocytes can be conveniently purified from blood or bone marrow by density gradient centrifugation using, for example, Ficoll Hypaque which eliminates neutrophils and red cells (peripheral blood mononuclear cells or PBMCs), or by ammonium chloride lysis of red cells (leukocytes or white blood cells). Many precursors of antigen-presenting cells are present in peripheral blood as non-proliferating monocytes, which can be differentiated into specific antigen-presenting cells, including macrophages and dendritic cells, suitably by incubating the precursor in the presence of one or more specific cytokines.

Tissue-derived precursors such as unfractionated lymph node-derived mononuclear cells, precursors of tissue dendritic cells or of Langerhans cells are typically obtained by mincing tissue (e.g., basal layer of epidermis) and digesting it with collagenase or dispase followed by density gradient separation, or selection of precursors based on their expression of cell surface markers. For example, Langerhans cell precursors express CD1. molecules as well as HLA-DR and can be purified on this basis.

In some embodiments, the antigen-presenting cell precursor is a precursor of macrophages. Generally these precursors can be obtained from monocytes of any source and can be differentiated into macrophages by prolonged incubation in the presence of medium and macrophage colony stimulating factor (M-CSF) (Erickson-Miller et al., 1990, lnt J Cell Cloning 8: 346-356; Metcalf and Burgess, 1982, J Cell Physiol 111: 275-283).

In other embodiments, the antigen presenting cell precursor is a precursor of Langerhans cells. Usually, Langerhans cells can be generated from human monocytes or CD34⁺ bone marrow precursors in the presence of granulocyte/macrophage colony-stimulating factor (GM-CSF), IL-4/TNFα and TGFβ (Geissmann et al., 1998, J Exp Med 187: 961-966; Strobl et al., 1997, Blood 90: 1425-1434 Strobl et al., 1997, Adv Exp Med Biol 417: 161-165; Strobl et al., 1996, J Immunol 157: 1499-1507).

In some embodiments, the antigen-presenting cell precursor is a precursor of dendritic cells. Several potential dendritic cell precursors can be obtained from peripheral blood, cord blood or bone marrow. These include monocytes, CD34⁺ stem cells, granulocytes, CD33⁺CD11c⁺ DC precursors, and committed myeloid progenitors - described below.

**Monocytes.** Monocytes can be purified by adherence to plastic for 1-2 h in the presence of tissue culture medium (e.g., RPMI) and serum (e.g., human or foetal calf serum), or in serum-free medium (Anton et al., 1998, Scand J Immunol 47: 116-121.; Araki et al., 2001, Br J Haematol 114: 681-689; Mackensen et al., 2000, Int J Cancer 86: 385-392; Nestle et al., 1998, Nat Med 4: 328-332; Romani et al., 1996, J Immunol Meth 196: 137-151; Thurner et al., 1999, J Immunol Methods 223: 1-15). Monocytes can also be elutriated from peripheral blood (Garderet et al., 2001, J Hematother Stem Cell Res 10: 553-567). Monocytes can also be purified by immunoaffinity techniques, including immunomagnetic selection, flow cytometric sorting or panning (Araki *et al.,* 2001, *supra*; Battye and Shortman, 1991, Curr. Opin, Immunol. 3: 238-241), with anti-CD14 antibodies to obtain CD14^{hi} cells. The numbers (and therefore yield) of circulating monocytes can be enhanced by the *in vivo* use of various cytokines including GM-CSF (Groopman et al., 1987, N Engl J Med 317: 593-598; Hill et al., 1995, J Leukoc Biol 58: 634-642). Monocytes can be differentiated into dendritic cells by prolonged incubation in the presence of GM-CSF and IL-4 (Romani et al., 1994, J Exp Med 180: 83-93; Romani *et al.,* 1996, *supra).* A combination of GM-CSF and IL-4 at a concentration of each at between about 200 to about 2000 U/mL, more preferably between about 500 to about 1000 U/mL and even more preferably between about 800 U/mL (GM-CSF) and 1000 U/mL (IL-4) produces significant quantities of immature dendritic cells, i.e., antigen-capturing phagocytic dendritic cells. Other cytokines which promote differentiation of monocytes into antigen-capturing phagocytic dendritic cells include, for example, IL-13.

**CD34⁺ stem cells.** Dendritic cells can also be generated from CD34⁺ bone marrow derived precursors in the presence of GM-CSF, TNFα ± stem cell factor (SCF, o-kitL), or GM-CSF, IL-4 ± flt3L (Bai et al., 2002, Int J Oncol 20: 247-53; Chen et al., 2001, Clin Immunol 98: 280-292; Loudovaris et al., 2001, J Hematother Stem Cell Res 10: 569-578). CD34⁺ cells can be derived from a bone marrow aspirate or from blood and can be enriched as for monocytes using, for example, immunomagnetic selection or immunocolumns (Davis et al., 1994, J Immunol Meth 175: 247-257). The proportion of CD34⁺ cells in blood can be enhanced by the *in vivo* use of various cytokines including (most commonly) G-CSF, but also flt3L and progenipoietin (Fleming et al., 2001, Exp Hematol 29: 943-951; Pulendran et al., 2000, J Immunol 165: 566-572; Robinson et al., 2000, J Hematother Stem Cell Res 9: 711-7 20).

**Other Myeloid progenitors.** DC can be generated from committed early myeloid progenitors in a similar fashion to CD34⁺ stem cells, in the presence of GM-CSF and IL-4/TNF. Such myeloid precursors infiltrate many tissues in inflammation, including rheumatoid arthritis synovial fluid (Santiago-Schwarz et al., 2001, J Immunol 167(3): 1758-68). Expansion of total body myeloid cells including circulating dendritic cell precursors and monocytes, can be achieved with certain cytokines, including flt-3 ligand, granulocyte colony-stimulating factor (G-CSF) or progenipoietin (pro-GP) (Fleming *et al.,* 2001, *supra;* Pulendran *et al.,* 2000, *supra;* Robinson *et al.,* 2000, *supra).* Administration of such cytokines for several days to a human or other mammal would enable much larger numbers of precursors to be derived from peripheral blood or bone marrow for *in vitro* manipulation. Dendritic cells can also be generated from peripheral blood neutrophil precursors in the presence of GM-CSF, IL-4 and TNFα (Kelly et al., 2001, Cell Mol Biol (Noisy-le-grand) 47(1): 43-54; Oehler et al., 1998, J Exp Med. 187(7):1019-28). It should be noted that dendritic cells can also be generated, using similar methods, from acute myeloid leukemia cells (Oehler et al., 2000, Ann Hematol 79(7): 355-62).

**Tissue DC precursors and other sources of APC precursors.** Other methods for DC generation exist from, for example, thymic precursors in the presence of IL-3 +/- GM-CSF, and liver DC precursors in the presence of GM-CSF and a collagen matrix. Transformed or immortalised dendritic cell lines may be produced using oncogenes such as *v-myc* as for example described by (Paglia et al., 1993, J Exp Med 178(6): 1893-901) or by myb (Banyer and Hapel, 1999, J Leukoc Biol 66(2): 217-223; Gonda et al., 1993, Blood 82(9): 2813-2822).

**Circulating DC precursors.** These have been described in human and mouse peripheral blood. One can also take advantage of particular cell surface markers for identifying suitable dendritic cell precursors. Specifically, various populations of dendritic cell precursors can be identified in blood by the expression of CD11c and the absence or low expression of CD14, CD19, CD56 and CD3 (O'Doherty et al., 1994, Immunology 82: 487-493; O'Doherty et al., 1993, J Exp Med 178: 1067-1078). These cells can also be identified by the cell surface markers CD13 and CD33 (Thomas et al., 1993, J Immunol 151(12); 6840-6852). A second subset, which lacks CD14, CD19, CD56 and CD3, known as plasmacytoid dendritic cell precursors, does not express CD11c, but does express CD123 (IL-3R chain) and HLA-DR (Farkas et al., 2001, Am J Pathol 159: 237-243; Grouard et al., 1997, J Exp Med 185: 1101-1111; Rissoan et al., 1999, Science 283: 1183-1186). Most circulating CD11c⁺ dendritic cell precursors are HLA-DR⁺, however some precursors may be HLA-DR-. The lack of MHC class II expression has been clearly demonstrated for peripheral blood dendritic cell precursors (del Hoyo et al., 2002, Nature 415: 1043-1047).

Optionally, CD33⁺CD14^{-/lo} or CD11c⁺HLA-DR⁺, lineage marker-negative dendritic cell precursors described above can be differentiated into more mature antigen-presenting cells by incubation for 18-36 h in culture medium or in monocyte conditioned medium (Thomas et al., 1993, J Immunol 151(12): 6840-6852; Thomas and Lipsky, 1994, J Immunol 153: 4016-4028; O'Doherty *et al.,* 1993, *supra).* Alternatively, following incubation of peripheral blood non-T cells or unpurified PBMC, the mature peripheral blood dendritic cells are characterised by low density and so can be purified on density gradients, including metrizamide and Nycodenz (Freudenthal and Steinman, 1990, Proc Natl Acad Sci U S A 87: 7698-7702; Vremec and Shortman, 1997, J Immunol 159: 565-573), or by specific monoclonal antibodies, such as but not limited to the CMRF-44 mAb (Fearnley et al., 1999, Blood 93, 728-736; Vuckovic et al., 1998, Exp Hematol 26: 1255-1264)*.* Plasmacytoid dendritic cells can be purified directly from peripheral blood on the basis of cell surface markers, and then incubated in the presence of IL-3 (Grouard *et al.,* 1997, *supra;* Rissoan *et al.,* 1999, *supra).* Alternatively, plasmacytoid DC can be derived from density gradients or CMRF-44 selection of incubated peripheral blood cells as above.

In general, for dendritic cells generated from any precursor, when incubated in the presence of activation factors such as monocyte-derived cytokines, lipopolysaccharide and DNA containing CpG repeats, cytokines such as TNF-α, IL-6, IFN-α, IL-1β, necrotic cells, readherence, whole bacteria, membrane components, RNA or polyIC, immature dendritic cells will become activated (Clark, 2002, J Leukoc Biol 71: 388-400; Hacker et al., 2002, Immunology 105: 245-25 1; Kaisho and Akira, 2002, Blochim Biophys Acta 1589: 1-13; Koski et al., 2001, Crit Rev Immunol 21: 179-189).

Other methods for isolation, expansion and/or maturation of dendritic cells are described for example by Takamizawa et al. (1997, J Immunol, 158(5): 2134-2142), Thomas and Lipsky (1994, J Immunol, 153(9): 4016-4028), O'Doherty et al. (1994, Immunology, 82(3): 487-93), Fearnley et al. (1997, Blood, 89(10): 3708-3716), Weissman et al. (1995, Proc Natl Acad Sci USA, 92(3): 826-830), Freudenthal and Steinman (1990, Proc Natl Acad Sci U S A, 87(19): 7698-7702), Romani et al. (1996, J Immunol Methods, 196(2): 137-151), Reddy et al. (1997, Blood, 90(9): 3640-3646), Thurnher et al. (1997, Exp Hematol, 25(3): 232-237), Caux et al, (1996, J Exp Med, 184(2): 695-706; 1996, Blood, 87(6): 2376-85), Luft et al. (1998, Exp Hematol, 26(6): 489-500; 1998, J Immunol, 161(4): 1947-1953), Cella et al. (1999, J Exp Med, 189(5): 821-829; 1997, Nature, 388(644): 782-787; 1996, J Exp Med, 184(2): 747-572), Ahonen et al. (1999, Cell Immunol, 197(1): 62-72) and Piemonti et al. (1999, J Immunol, 162(11): 6473-6481).

In certain embodiments, the antigen-presenting cells or their precursors are in the form of a substantially purified population of cells. In other embodiments, the antigen-presenting cells or their precursors are in the form of a heterogenous pool of cells. Suitably, the substantially purified or heterogenous population used to contact an antigen is in cultured or uncultured form as defined herein. In certain advantageous embodiments employing an uncultured population of antigen-presenting cells or their precursors, the population can be incubated for short time periods (e.g., as low as about 5, 10, 15, 20, 20, 40, 50, 60 min) and the contacted population can be infused directly into a recipient without further culturing of the cells. This further shortens the processing time to permit potentially the harvesting of autologous or syngeneic antigen-presenting cells, treatment of those cells with antigen and infusion of the antigen-contacted cells into a patient in a single sitting or day.

### 3.2 Delivery of antigen to antigen-presenting_cells

The delivery of exogenous antigen to antigen presenting cells can be enhanced by methods known to practitioners in the art. For example, several different strategies have been developed for delivery of exogenous antigen to the endogenous processing pathway of antigen-presenting cells, especially dendritic cells. These methods include insertion of antigen into pH-sensitive liposomes (Zhou and Huang, 1994, Immunomethods, 4:229-235), osmotic lysis of pinosomes after pinocytic uptake of soluble antigen *{*Moore et al. , 1988, Cell, 54:777-785), coupling of antigens to potent adjuvants *(*Aichele et al., 1990, J. Exp. Med., 171: 1815-1820; Gao et al., 1991, J. Immunol, 147: 3268-3273; Schulz et al., 1991, Proc. Natl. Acad. Sci. USA, 88:991-993; Kuzu et al., 1993, Euro, J. Immunol., 23: 1397-1400; and Jondal et al., 1996, Immunity 5: 295-302) and apoptotic cell delivery of antigen *(*Albert et al. 1998, Nature 392:86-89; Albert et al. 1998, Nature Med. 4:1321-1324; and in International Publications WO 99142564 and WO 01/85207). Recombinant bacteria (eg. E. *coli)* or transfected host mammalian cells may be pulsed onto dendritic cells (as particulate antigen, or apoptotic bodies respectively) for antigen delivery. Recombinant chimeric virus-like particles (VLPs) have also been used as vehicles for delivery of exogenous heterologous antigen to the MHC class I processing pathway of a dendritic cell line (Bachmann et al., 1996, Eur. J. Immunol., 26(11): 2595-2600). In some embodiments, solubilized antigen (e.g., in DMSO) is incubated with antigen-presenting cells.

Alternatively, or in addition, an antigen (e.g., a peptide antigen) may be linked to, or otherwise associated with, a cytolysin to enhance the transfer of the peptide into the cytosol of an antigen-presenting cell of the invention for delivery to the MHC class I pathway. Exemplary cytolysins include saponin compounds such as saponin-containing Immune Stimulating Complexes (ISCOMs) (see e.g., Cox and Coulter, 1997, Vaccine 15(3): 248-256 and U.S. Patent No. 6,352,697), phospholipases (see, e.g., Camilli et al., 1991, J. Exp. Med. 173: 751-754), pore-forming toxins (e.g., an alpha-toxin), natural cytolysins of gram-positive bacteria, such as listeriolysin O (LLO, e.g., Mengaud et al., 1988, Infect. Immun. 56: 766-772 and Portnoy et al., 1992, Infect. Immun, 60: 2710-2717), streptolysin O (SLO, e.g., Palmer et al., 1998, Biochemistry 37(8): 2378-2383) and perfringolysin O (PFO, e.g., Rossjohn et al., Cell 89(5): 685-692). Where the antigen-presenting cell is phagosomal, acid activated cytolysins may be advantageously used. For example, listeriolysin exhibits greater pore-forming ability at mildly acidic pH (the pH conditions within the phagosome), thereby facilitating delivery of vacuole (including phagosome and endosome) contents to the cytoplasm (see, e.g., Portnoy et al., Insect. Immun. 1992,60: 2710-2717).

The amount of antigen to be placed in contact with antigen-presenting cells can be determined empirically by persons of skill in the art. The antigen-presenting cells should be exposed to the antigen for a period of time sufficient for those cells to present the peptides or processed forms thereof for the modulation of T cells. In some advantageous embodiments the antigen presenting cells are incubated in the presence of antigen for less than about 48, 36,24, 12, 8, 7, 6, 5, 4, 3 or 2 hours or even for less that about 60, 50, 40, 30,20,15,10, 9, 8, 7,6, 5,4, 3 or 2 minutes). The time and dose of peptides necessary for the cells to optionally process and present the peptides or their processed forms may be determined using pulse-chase protocols in which exposure to peptides is followed by a washout period and exposure to a read-out system e.g., antigen reactive T cells. Once the optimal time and dose necessary for cells to express the peptides or their processed forms on their surface is determined, a protocol may be used to prepare cells and peptides for inducing immunogenic responses. Those of skill in the art will recognise in this regard that the length of time necessary for an antigen-presenting cell to present an antigen on its surface may vary depending on the antigen or form of antigen employed, its dose, and the antigen-presenting cell employed, as well as the conditions under which antigen loading is undertaken. These parameters can be determined by the skilled artisan using routine procedures. Efficiency of priming of the antigen-presenting cells can be determined by assaying T cell cytotoxic activity *in vitro* or using antigen-presenting cells as targets of CTLs. Other methods known to practitioners in the art, which can detect the presence of antigen on the surface of antigen-presenting cells after exposure to one or more of the modified and unmodified antigens, are also contemplated by the presented invention.

Usually, about 0.1 to 20 µg/mL of antigen *(e.g.,* peptide antigen) to about 1-10 million antigen-presenting cells is suitable for producing primed antigen-specific antigen-presenting cells. Typically antigen-presenting cells are incubated with antigen for about 1 to 6 hr at 37° C, although it is also possible to expose antigen-presenting cells to antigen for the duration of incubation with one or more growth factors. As discussed above, the present inventors have shown that successful presentation of antigen (e.g., peptide antigen) or their processed forms can be achieved using much shorter periods of incubation (e.g., about 5, 10, 15, 20, 30, 40, 50 minutes) using antigen at a concentration of about 10-20 µg/mL.

If desired, all or a portion of the antigen-presenting cells can be frozen in an appropriate cryopreservative solution, until required. For example, the cells may be diluted in an appropriate medium, such as one containing 10% of autologous serum + 10% of dimethylsulfoxide in a phosphate buffer saline. In certain embodiments, the cells are conserved in a dehydrated form.

### 4. Lymphocyte embodiments

The antigen-presenting cells of the invention may be obtained or prepared to contain and/or express one or more antigens by any number of means, such that the antigen(s) or processed form(s) thereof, is (are) presented by those cells for potential modulation of other immune cells, including T lymphocytes and B lymphocytes, and particularly for producing T lymphocytes and B lymphocytes that are primed to respond to a specified antigen or group of antigens. In some embodiments, the subject antigen-presenting cells are useful for producing primed T lymphocytes to an antigen or group of antigens. The efficiency of inducing lymphocytes, especially T lymphocytes, to exhibit an immune response to a specified antigen can be determined by any suitable method including, but not limited to, assaying T lymphocyte cytolytic activity *in vitro* using for example antigen-specific antigen-presenting cells as targets of antigen-specific cytolytic T lymphocytes (CTL); assaying antigen-specific T lymphocyte proliferation (see, e.g., Vollenweider and Groseurth, 1992, J. Immunol. Meth. 149: 133-135), measuring B cell response to the antigen using, for example, ELISPOT assays, and ELISA assays; interrogating cytokine profiles; or measuring delayed-type hypersensitivity (DTH) responses by test of skin reactivity to a specified antigen (see, e.g., Chang et al. (1993, Cancer Res. 53: 1043-1050). Other methods known to practitioners in the art, which can detect the presence of antigen on the surface of antigen-presenting cells after exposure to the antigen, are also contemplated by the present invention.

Accordingly, the present invention also provides antigen-specific B or T lymphocytes, especially T lymphocytes, which respond in an antigen-specific fashion to representation of the antigen. In some embodiments, antigen-specific T lymphocytes are produced by contacting an antigen-presenting cell as defined above with a population of T lymphocytes, which may be obtained from any suitable source such as spleen or tonsil/lymph nodes but is preferably obtained from peripheral blood. The T lymphocytes can be used as crude preparations or as partially purified or substantially purified preparations, which are suitably obtained using standard techniques as, for example, described in "Immunochemical Techniques, Part G: Separation and Characterization of Lymphoid Cells" (Meth. in Enzymol. 108, Edited by Di Sabato et al., 1984, Academic Press). This includes rosetting with sheep red blood cells, passage across columns of nylon wool or plastic adherence to deplete adherent cells, immunomagnetic or flow cytometric selection using appropriate monoclonal antibodies is known in the art.

The preparation of T lymphocytes is contacted with the antigen-presenting cells of the invention for an adequate period of time for priming the T lymphocytes to the antigen or antigens presented by those antigen-presenting cells. This period will preferably be at least about 1 day, and up to about 5 days.

In some embodiments, a population of antigen-presenting cells is cultured in the presence of a heterogeneous population of T lymphocytes, which is suitably obtained from peripheral blood, together with a set of peptides of the invention corresponding to an antigen to which an immune response is required. These cells are cultured for a period of time and under conditions sufficient for the peptides, or their processed forms, to be presented by the antigen-presenting cells; and the antigen-presenting cells to prime a subpopulation of the T lymphocytes to respond to the antigen.

### 5. Cell based therapy or prophylaxis

The antigen-presenting cells described in Section 3 and the lymphocytes described in Section 4 can be administered to a patient, either by themselves or in combination, for modulating an immune response, especially for modulating an immune response to one or more cognate antigens. These cell based compositions are useful, therefore, for treating or preventing a disease or condition as noted above. The cells of the invention can be introduced into a patient by any means (e.g., injection), which produces the desired immune response to an antigen or group of antigens. The cells may be derived from the patient (i.e., autologous cells) or from an individual or individuals who are MHC matched or mismatched (i.e., allogeneic) with the patient, Typically, autologous cells are injected back into the patient from whom the source cells were obtained. The injection site may be subcutaneous, intraperitoneal, intramuscular, intradermal, intravenous or intralymphoid. The cells may be administered to a patient already suffering from a disease or condition or who is predisposed to a disease or condition in sufficient number to treat or prevent or alleviate the symptoms of the disease or condition. The number of cells injected into the patient in need of the treatment or prophylaxis may vary depending on *inter alia,* the antigen or antigens and size of the individual. This number may range for example between about 10³ and 10¹¹, and usually between about 10⁵ and 10⁷ cells (e.g., in the form blood, PMBC or purified dendritic cells or T lymphocytes). Single or multiple (2, 3, 4 or 5} administrations of the cells can be carried out with cell numbers and pattern being selected by the treating physician. The cells should be administered in a pharmaceutically acceptable carrier, which is non-toxic to the cells and the individual. Such carrier may be the growth medium in which the cells were grown, or any suitable buffering medium such as phosphate buffered saline. The cells may be administered alone or as an adjunct therapy in conjunction with other therapeutics known in the art for the treatment or prevention of unwanted immune responses for example but not limited to glucocorticoids, methotrexate, D-penicillamine, hydroxychloroquine, gold salts, sulfasalazine, TNF-alpha or interleukin-1 inhibitors, and/or other forms of specific immunotherapy.

### 6. Compositions

The overlapping sets of peptides described in Sections 2 and the antigen-primed antigen-presenting cells described in Section 3 or the lymphocytes described in Section 4 (therapeutic/prophylactic agents) can be used singly or together as active ingredients for the treatment or prophylaxis of various conditions associated with the presence of one or more target polypeptide antigens. These therapeutic/prophylactic agents can be administered to a patient either by themselves, or in compositions where they are mixed with a suitable pharmaceutically acceptable carrier and/or diluent, or an adjuvant.

The invention also encompasses a method for stimulating a patient's immune system, and preferably for eliciting a humoral and/or cellular immune response to a polypeptide of interest, by administering to the patient a therapeutic agent or composition as described above. Such stimulation may be utilised for the treatment and/or prophylaxis of a disease or condition including, but not restricted to, a pathogenic infection (e,g., viral, bacterial, fungal, protozoan) or a cancer. Accordingly, the invention contemplates a method for treatment and/or prophylaxis of a disease or condition, comprising administering to a patient in need of such treatment a therapeutically/prophylactically effective amount of a therapeutic agent or composition as broadly described above.

Depending on the specific conditions being treated, therapeutic/prophylactic agents may be formulated and administered systemically or locally. Techniques for formulation and administration may be found in "Remington's Pharmaceutical Sciences," Mack Publishing Co., Easton, Pa., latest edition. Suitable routes may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. For injection, which constitutes one desirable embodiment of the present invention, the therapeutic agents of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art, Intra-muscular and subcutaneous injection is appropriate, for example, for administration of immunogenic compositions, vaccines and DNA vaccines. In certain embodiments of the present invention, the immunogenic compositions are administered intravenously.

The therapeutic/prophylactic agents can be formulated readily using pharmaceutically acceptable carriers well known in the art into dosages suitable for oral administration. Such carriers enable the compounds of the invention to be formulated in dosage forms such as tablets, pills, capsules, liquids, gels, syrups, slurries, suspensions and the like, for oral ingestion by a patient to be treated. These carriers may be selected from sugars, starches, cellulose and its derivatives, malt, gelatine, talc, calcium sulphate, vegetable oils, synthetic oils, polyols, alginic acid, phosphate buffered solutions, emulsifiers, isotonic saline, and pyrogen-free water.

Pharmaceutical compositions suitable for use in the present invention include compositions wherein the active ingredients are contained in an effective amount to achieve its intended purpose. The dose of agent administered to a patient should be sufficient to effect a beneficial response in the patient over time such as a reduction in the symptoms associated with the condition. The quantity of the therapeutic/prophylactic agent(s) to be administered may depend on the subject to be treated inclusive of the age, sex, weight and general health condition thereof. In this regard, precise amounts of the therapeutic/prophylactic agent(s) for administration will depend on the judgement of the practitioner. In determining the effective amount of the agent to be administered in the treatment or prophylaxis of the condition, the physician may evaluate tissue levels of a target antigen, and progression of the disease or condition. In any event, those of skill in the art may readily determine suitable dosages of the therapeutic agents of the invention.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes, Aqueous injection suspensions may contain substances which increase the viscosity of the suspension, such as sodium carboxymethyl cellulose, sorbitol, or dextran. Optionally, the suspension may also contain suitable stabilisers or agents which increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as., for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Such compositions may be prepared by any of the methods of pharmacy but all methods include the step of bringing into association one or more therapeutic agents as described above with the carrier which constitutes one or more necessary ingredients, In general, the pharmaceutical compositions of the present invention may be manufactured in a manner that is itself known, e.g., by means of conventional mixing, dissolving, granulating, dragee-making, levitating, emulsifying, encapsulating, entrapping or lyophilising processes.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterise different combinations of active compound doses.

Pharmaceutical which can be used orally include push-fit capsules made of gelatin, as well as soft, sealed capsules made of gelatin and a plasticiser, such as glycerol or sorbitol. The push-fit capsules can contain the active ingredients in admixture with filler such as lactose, binders such as starches, and/or lubricants such as talc or magnesium stearate and, optionally, stabilisers. In soft capsules, the active compounds may be dissolved or suspended in suitable liquids, such as fatty oils, liquid paraffin, or liquid polyethylene glycols. In addition, stabilisers may be added.

Dosage forms of the therapeutic agents of the invention may also include injecting or implanting controlled releasing devices designed specifically for this purpose or other forms of implants modified to act additionally in this fashion. Controlled release of an agent of the invention may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, controlled release may be effected by using other polymer matrices, liposomes and/or microspheres.

Therapeutic agents of the invention may be provided as salts with pharmaceutically compatible counterions. Pharmaceutically compatible salts may be formed with many acids, including but not limited to hydrochloric, sulphuric, acetic, lactic, tartaric, malic, succinic, etc. Salts tend to be more soluble in aqueous or other protonic solvents that are the corresponding free base forms.

For any compound used in the method of the invention, the effective dose can be estimated initially from cell culture assays. For example, a dose can be formulated in animal models to achieve a circulating concentration range that includes the IC50 as determined in cell culture (e.g., the concentration of a test agent, which achieves a half-maximal reduction in target antigen). Such information can be used to more accurately determine useful doses in humans,

Toxicity and therapeutic efficacy of the compounds of the invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. The data obtained from these cell culture assays and animal studies can be used in formulating a range of dosage for use in human. The dosage of such compounds lies preferably within a range of circulating concentrations that include the ED50 with little or no toxicity. The dosage may vary within this range depending upon the dosage form employed and the route of administration utilised. The exact formulation, route of administration and dosage can be chosen by the individual physician in view of the patient's condition. (See for example Fingl *et al.,* 1975, in "The Pharmacological Basis of Therapeutics", Ch. 1 pl).

Dosage amount and interval may be adjusted individually to provide plasma levels of the active compound(s) which are sufficient to maintain target antigen-reducing effects or effects that ameliorate the disease or condition. Usual patient dosages for systemic administration range from 1-2000 mg/day, commonly from 1-250 mg/day, and typically from 10-150 mg/day. Stated in terms of patient body weight, usual dosages range from 0.02-25 mg/kg/day, commonly from 0.02-3 mg/kg/day, typically from 0.2-1.5 mg/kg/day. Stated in terms of patient body surface areas, usual dosages range from 0.5-1200 mg/m²/day, commonly from 0.5-150 mg/m²/day, typically from 5-100 mg/m²/day.

Alternately, one may administer the agent in a local rather than systemic manner, for example, *via* injection of the compound directly into a tissue, often in a depot or sustained release formulation. Furthermore, one may administer the agent in a targeted drug delivery system, for example, in a liposome coated with tissue-specific antibody. The liposomes will be targeted to and taken up selectively by the tissue.

From the foregoing, it will be appreciated that the agents of the invention may be used as therapeutic or prophylactic immunomodulating compositions or vaccines. Accordingly, the invention extends to the production of immunomodulating compositions containing as active compounds one or more of the therapeutic/prophylactic agents of the invention. Any suitable procedure is contemplated for producing such vaccines. Exemplary procedures include, for example, those described in NEW GENERATION VACCINES (1997, Levine *et al.,* Marcel Dekker, Inc. New York, Basel Hong Kong).

Immunomodulating compositions according to the present invention can contain a physiologically acceptable diluent or excipient such as water, phosphate buffered saline and saline. They may also include an adjuvant as is well known in the art. Suitable adjuvants include, but are not limited to: surface active substances such as hexadecylamine, octadecylamine, octadecyl amino acid esters, lysolecithin, dimethyldioctadecylammonium bromide, N, N-dicoctadecyl-N', N'bis(2-hydroxyethyl-propanediamine), methoxyhexadecylglycerol, and pluronic polyols; polyamines such as pyran, dextransulfate, poly IC carbopol; peptides such as muramyl dipeptide and derivatives, dimethylglycine, tuftsin; oil emulsions; and mineral gels such as aluminum phosphate, aluminum hydroxide or alum; lymphokines, QuilA and immune stimulating complexes (ISCOMS).

The antigen-primed antigen-presenting cells of the invention and antigen-specific T lymphocytes generated with, these antigen-presenting cells, as described *supra,* can be used as active compounds in immunomodulating compositions for prophylactic or therapeutic applications. In some embodiments, the antigen-primed antigen-presenting cells of the invention are useful for generating large numbers of CD8⁺ or CD4+ CTL, for adoptive transfer to immunosuppressed individuals who are unable to mount normal immune responses. For example, antigen-specific CD8⁺ CTL can be adoptively transferred for therapeutic purposes in individuals afflicted with HIV infection (Koup et al., 1991, J. Exp. Med., 174: 1593-1600; Carmichael et al., 1993, J. Exp. Med., 177: 249-256; and Johnson et al., 1992, J. Exp. Med., 175: 961-971), malaria (Hill et al., 1992, Nature, 360: 434-439) and malignant tumours such as melanoma (Van der Brogen et al., 1991, Science, 254: 1643-1647; and Young and Steinman, 1990, J, Exp. Med., 171: 1315-1332).

In other embodiments, the immunomodulating composition of the invention is suitable for treatment or prophylaxis of a cancer. Cancers which could be suitably treated in accordance with the practices of this invention include cancers associated with a viral infection such as cervical cancer (e,g., papillomavirus infection) and Burkitt's lymphoma (e.g., Epstein Barr virus infection). Other virus associated cancers include, but are not restricted to, HTLV1 associated leukemia, Non Hodgkins lymphoma (EBV), anal cancer, skin cancer (HPV), hepatocellular carcinoma (HBV) and Kaposis sarcoma (HHV8). Alternatively, the cancer may be a non-virally associated cancer such as but not limited to melanoma, lung cancer, breast cancer, prostate cancer, colon cancer, pancreatic cancer, stomach cancer, bladder cancer, kidney cancer, post transplant lymphoproliferative disease (PTLD), Hodgkin's Lymphoma and the like.

In still other embodiments, the immunomodulating composition is suitable for treatment or prophylaxis of a viral, bacterial or protozoan infection. Viral infections contemplated by the present invention include, but are not restricted to, infections caused by HIV, Hepatitis, Influenza, Japanese encephalitis virus, Epstein-Barr virus and respiratory syncytial virus. Bacterial infections include, but are not restricted to, those caused by *Neisseria* species, *Meningococcal* species, *Haemophilus* species *Salmonella* species, *Streptococcal* species, *Legionella* species and *Mycobacterium* species. Protozoan infections encompassed by the invention include, but are not restricted to, those caused by *Plasmodium* species (e.g., malaria), *Schistosoma* species (e.g., schistosomiasis), *Leishamania* species, *Trypanosoma* species, *Toxoplasma* species and *Giardia* species.

### 7. Methods for assessing immunomodulation

The effectiveness of the immunisation may be assessed using any suitable technique. An individual's capacity to respond to foreign or disease-specific antigens (e.g., viral antigens and cancer antigens) may be determined by assessing whether those cells primed to attack such antigens are increased in number, activity, and ability to detect and destroy those antigens. Strength of immune response is measured by standard tests including: direct measurement of peripheral blood lymphocytes by means known to the art; natural killer cell cytotoxicity assays (see, e.g,, Provinciali M. et al (1992, J. Immunol. Meth. 155: 19-24), cell proliferation assays (see, e.g., Vollenweider, I. and Groseurth P. J, (1992, J. Immunol. Meth. 149: 133-135), immunoassays of immune cells and subsets (see, e.g., Loeffler, D. A., et al. (1992, Cytom. 13: 169-174); Rivoltini, L., et al. (1992, Can. Immnunol. Immunother. 34: 241-251); or skin tests for cell-mediated immunity (see, e.g., Chang, A. E. et al (1993, CancerRes. 53: 1043-1050). Alternatively, the efficacy of the immunisation may be monitored using one or more techniques including, but not limited to, HLA class I tetramer staining - of both fresh and stimulated PBMCs (see for example Allen *et al., .supra),* proliferation assays (Allen *et al., supra),* ELISPOT assays and intracellular cytokine staining (Allen *et al., supra),* ELISA Assays - for linear B cell responses; and Western blots of cell sample expressing the synthetic polynucleotides. Particularly relevant will be the cytokine profile of T cells activated by antigen, and more particularly the production and secretion of IFNγ, IL-2, IL4, IL5, IL-10, TGFβ and TNF α.

The cytotoxic activity of T lymphocytes, and in particular the ability of cytotoxic T lymphocytes to be induced by antigen-presenting cells, may be assessed by any suitable technique known to those of skill in the art. For example, a sample comprising T lymphocytes to be assayed for cytotoxic activity is obtained and the T lymphocytes are then exposed to antigen-primed antigen-presenting cells, which have been caused to present antigen. After an appropriate period of time, which may be determined by assessing the cytotoxic activity of a control population of T lymphocytes which are known to be capable of being induced to become cytotoxic cells, the T lymphocytes to be assessed are tested for cytotoxic activity in a standard cytotoxic assay.

The method of assessing CTL activity is particularly useful for evaluating an individual's s capacity to generate a cytotoxic response against cells expressing tumour or viral antigens. Accordingly, this method is useful for evaluating an individual's ability to mount an immune response to a cancer or virus. For example, CTL lysis assays may be employed using stimulated splenocytes or peripheral blood mononuclear cells (PBMC) on peptide coated or recombinant virus infected cells using ⁵¹Cr labelled target cells. Such assays can be performed using for example primate, mouse or human cells *(*Allen et al., 2000, J Immunol, 164(9): 4968-4978 also Woodberry *et al., infra),* In addition, CTL activity can be measured in outbred primates using the *in vivo* detection method described in Figure 1.. In this method, autologous cells (e.g., PMBC) are labelled with an optically detectable label (e.g., a fluorescent, chemiluminescent or phosphorescent or visual label or dye) and are contacted with one ore more peptide sets as disclosed herein. The peptide sets are chosen so that they correspond to an antigen which is the subject of a CTL response under test in a subject. The autologous cells are infused into the subject and lymphocytes from the subject are harvested after a suitable period to permit the subject's immune system sufficient time to respond to the autologous cells (e.g., 10 minutes to 24 hours post infusion). The harvested lymphocytes are then analysed to identify the number or proportion of lymphocytes which contain or otherwise carry the optically detectable label, which represents a measure of the *in vivo* CTL response to the antigen in the subject.

In order that the invention may be readily understood and put into practical effect, particular preferred embodiments will now be described by way of the following non-limiting examples.

### EXAMPLES

### EXAMPLE 1

### In vivo cytotoxic T-lymphocyte killing

The standard measure of virus-specific CTL effector is measured via the release of a radioisotope ⁵¹Cr from target cells, an assay that is tedious and poorly sensitive. By pulsing dye-labelled autologous macaque PBMC with large pools of SIV and SHIV overlapping peptides (OPAL) and infusing the cells back into the same animal, the inventors were able to kinetically show SHIV-specific killing in blood sampled at various time-points following the infusion of OPAL by flow cytometry.

Two weeks after full immunisation (week 10), three of four immunised animals displayed moderate to large (11.4-76%) killing of gag-pulsed PBMC by 16 hours post-OPAL infusion, whereas control-immunised monkeys displayed <7% gag-specific killing. One immunised animal, monkey H20, demonstrated vigorous gag-specific killing (27.3%) as early as 4 hours post-infusion (Figure 2). These data were consistent with T cell responses induced by the vaccines as analysed by IFNγ ELISpot and ICS (data not shown), indicating the usefulness of OPAL to measure effective CTL effector responses primed by the DNA and FPV vaccines.

Shortly (2 weeks) after SHIV intrarectal challenge all four immunised animals exhibited large degrees of gag-specific killing (65-98.3%) 16 hours post-OPAL infusion, and two of four (monkeys H20 and H21) further demonstrated >99% pol-specific killing (Figure 3). In comparison with control-immunised animals, monkey E20 displayed <6% killing of both gag- and pol-pulsed PBMC whereas monkey E22 showed >90% and 31.9% of gag- and pol-pulsed PBMC, respectively. Interestingly, the animals that displayed moderate to high degrees of pol-specific killing (monkeys H20, H21 and E22) were also the only animals that had previously received 2 doses of infused pol-pulsed PBMC (weeks 10 and 15), whereas monkeys B00, H8 and E20 received pol-pulsed PBMC only once prior. This observation suggests that the infusion of OPAL may have: (a) boosted pol-specific T cell responses primed by the vaccines that were weakly or not detected by IFNγ ELISpot and ICS (data not shown), and; (b) induced pol-specific immunity in naïve animals evident post-SHIV challenge.

### EXAMPLE 2

### Analysis of the immunogenicity induced by infusing peptide-pulsed autologous cells.

It seemed plausible that if *in vivo* CTL killing could be efficiently measured by OPAL infusion, this method may be able to either prime a new or boost an existing immune response. IFNγ ELISpot and ICS assays were therefore performed prior to- and one week following each OPAL infusion assay to analyse whether there would be an increase in T cell immunogenicity previously primed by the vaccines or by the OPAL infusion method itself (Figure 4).

Following the first OPAL infusion performed at week 10, a 3- to 16-fold increase in IFNγ -secreting cells to SIV gag peptide pool was detected in monkeys H20 and H21, measuring up to 430 spot-forming cells (Figure 5). Monkey H8 measured a 54% increase to 215 spot-forming cells, whereas no increase was measured in control-immunised animals. Analysis of monkeys B00 (post-OPAL infusion) and E20 (pre-OPAL infusion) for all antigens analysed were excluded due to developmental problems of the assay. Of the four animals that received pol-pulsing at week 10, monkeys H20, H21 and E22, displayed increased pol responses by up to 140 spot-forming cells post-OPAL infusion, whereas no significant ELISpot responses were detected in monkey E20. No nef-specific T cell was in all animals apparent before or after OPAL-infusion. These results suggest a boosting effect in T cell immunogenicity following gag- and pol-peptide pulsing in the animals previously primed for SIVgag/pol responses, and furthermore indicate priming for SIVpol in a naïve animal (monkey E22).

At week 15, 8 weeks following full immunisation, a second OPAL infusion assay was performed in the six animals. ELISpot analyses revealed increased responses to gag peptide pool by up to 500 spot-forming cells from approximately 50 or less spot-forming cells prior to OPAL infusion in the four animals pre-immunised with DNA and FPV vaccines, In control-immunised animals, no gag-specific T cells were measured before or after the assay (Figure 6). In comparison, a slight increase in pol-specific responses (up to 40 spot-forming cells) from baseline was measured in only a few animals. Large increased responses to WI SIV were measured in all pre-immunised animals (up to 450 spot-forming cells), whereas control-immunised animals displayed modest or no increases (up to 50 spot-forming cells). All responses to SIV nef and SHIV env were minimal or undetected in all animals prior to and after OPAL infusion.

Following SHIV intrarectal challenge, all animals except monkey E20 displayed increased gag responses measuring between 50-600 spot-forming cells. Similar responses were observed for WI SIV but to levels up to 200 spot-forming cells, whereas pol responses above 50 spot-forming cells were only evident in monkey H20.

The immunogenicity of OPAL infusion was further verified by comparison to animals that received the same immunisation regimen but did not receive OPAL infusion (Figure 7). No rise in SIV gag, pol or WI SIV-specific T cells were detected in groups 1 (control-immunised) and 2 (2x DNA/FPV-immunised) from weeks 9 to 11 and 15 to 18. Responses from weeks 20 to 21 increased slightly the groups, attributable to responses enhanced by SHIV challenge at week 18.

The experiments performed on macaques infused with peptide pulsed whole blood also demonstrated a boost in CD4+ and CD8+ T cell responses to both (a) several parts of SHIV in recipients of SHIV-peptide pulsed blood (Figure 9), (b) 2 pools of HCV peptides spanning the entire HCV genome in recipients of HCV-peptide pulsed blood (Figure 10), and (c) a pool of peptides spanning known HIV-1 drug resistant mutations in recipients of autologous blood pulsed with HIV- I resistant peptides (Figure 11).

### EXAMPLE 3

### Outcome of SHIV ₘₙ₂₂₉ intrarectal challenge

The highly pathogenic SHIVₘₙ229 challenge stock was inoculated intrarectally into all macaques 10 weeks after full immunisation at a dose of 10⁵ TCID₅ₒ**.** Plasma SHIV RNA and CD4+ T cell counts were followed in all control-and 2xDNA/FPV-immunised animals (Figure 8).

Control-immunised monkeys E20 and E22 exhibited peak viral loads of 7.8±4,7 log₁₀ copies/mL at 2 weeks following challenge. The peak viral load of monkey E20 may have occurred between week 1 and 2, however, set-point levels of both monkeys (measured 5 to 11 Weeks post challenge) remained high at 5.9±0.3 log₁₀ copies/mL. Conversely at week 2, CD4+ T cell counts dropped dramatically to 1.6+1.1% of total lymphocytes, and set-point levels were steady at 0.3±0.2%. Monkeys that received the same immunisation but no OPAL infusions (group 1) performed only marginally worse than monkeys E20 and E22 in terms of peak and set-point viral loads (8.2±0.1 log₁₀ o copies/mL and 6.2±0.3 log₁₀ copies/mL), as well as CD4+ counts (set-point a.5±0.3%).
Based on the enhanced pol-specific killing that may have been attributed to 2 separate OPAL infusions, the SHIV viral loads and CD4+ T cell counts of monkeys H20 and H21 were compared to monkeys 500 and H8 that received only 1 dose of pol-OPAL infusions. Peak viral load of monkeys H20 and H21 (receiving 2 pol-OPAL infusions) was at least 10-fold lower than monkeys B00 and H8 (5.9±1.3 vs. 7.1±0.4 log₁₀ copies/mL, *p*=0.08), and set-point viral load showed a trend towards being lower (4.1±0.9 vs. 5.4±0.7 log₁₀ copies/mL, *P*=0.08, student's *t* test). Incidentally, set-point CD4+ T cell count for monkeys H20 and H21 was significantly greater than monkeys B00 and H8 (18.9±6.1% vs. 8.4%, *P=0.02).* Although statistically insignificant in comparison with group 2 animals who received the same immunisations but no OPAL infusions (*P*=0.12), monkeys H20 and H21 that received multiple pol-OPAL infusions displayed a trend towards the retainment of CD4+ T cells although viral loads were relatively similar, indicative of viral challenge protection. Set-point CD4+ T cell count and viral load of group 2 were 13.0±3.7% and 4.8± 0.2 log₁₀ copies/mL, respectively.

In comparison to control-immunised monkeys E20 and E22, both set-point viral load and CD4+ T cell count of monkeys H20 and H21 were significantly different (*P*=0.0*1, P*=0.00). The set-point viral load of monkeys B00 and H8, on the other hand, was not significantly lower than monkeys E20 and E22 (*P*=0.37) despite significant set-point levels of CD4+ T cells (*P*=0.01). Note that monkey H20 had completely cleared plasma viral RNA from week 5 and onwards and retained CD4+ T cells at normal levels.

### DISCUSSION OF THE EXAMPLES

The vital role for HIV-1-specific CD4+ T-helper (Th) and CD8+ CTL responses in controlling HIV-1 replication is the focus of many current vaccine concepts. The infusion of autologous PBMC pulsed with large overlapping sets of SHIV 15mer peptides (OPAL) was surprisingly immunogenic in its ability to boost SHIV-specific immune responses as analysed by IFNγ ELISpot and ICS assays. This finding forms the potential basis of a novel vaccine or immunotherapeutic strategy as described herein.

The evidence for this immunogenicity of peptide-pulsed fresh PBMC was five-fold: (a) Increases in SIV gag-specific IFNγ ELISpot responses were observed one week after each of the 3 SIV gag OPAL infusions (week 10, 15, and 20) in all vaccinated monkeys. In contrast, at week 10 and 15, SIVgag responses in equivalently immunised animals (group 2) not receiving the OPAL infusion significantly declined, (b) Increases in SIV pol-specific IPNγ ELISpot responses were observed in immunised animals one week following the SIV pol infusion at week 10 and 20, Interestingly this was observed in only the two monkeys H20 and H21 that received multiple SIV pol OPAL infusions prior to SHIV challenge (weeks 10 and 15) and not in animals receiving SIV pol peptide pulsed cells at week 15. This is of particular interest since the pol-specific T cell responses to the DNA and FPV vaccines alone were modest or undetectable by ELISpot and ICS, (c) High levels of SIV pol-specific *in vivo* killing were also seen in the two monkeys that received 2 prior infusions of SIV pol OPAL infusions, (d) This immunogenicity data was further confirmed by high levels of SIV pol-specific IFN γ intracellular cytokine responses in the two immunised animals receiving the multiple SIV pol OPAL infusions. (e) There was a trend towards greater protection from SHIV challenge in animals receiving multiple OPAL infusions. Together, these results suggest that pulsing autologous PBMC ex *vivo* with pools of overlapping peptides is an effective method for boosting immune responses. In addition, data show that peptide pulsed whole blood can both stimulate T cell responses to several parts of SHIV in recipients of SHIV-peptide pulsed blood, as well as induce *de novo* T cell responses to (a) 2 pools of HCV peptides spanning the entire HCV genomic in recipients of HCV-peptide pulsed blood and (b) a pool of peptides spanning known HIV-1 drug resistant mutations in recipients of autologous blood pulsed with HIV-1 resistant peptides.

There is a body of data ascertaining the use of pulsing autologous or syngeneic cells with defined peptide epitopes or whole antigen for the induction (or 'cross-priming') of immune responses (22, 23, 27, 34, 35). The use of specialised antigen presenting cells such as monocyte-derived dendritic cells pulsed with, for example, single tumour antigens or whole inactivated SIV has also been studied extensively as an immunotherapeutic tool (36-39). However, to the inventors' knowledge this is the first report of utilising large peptide pools spanning an entire protein (125 SIV gag l5mers or 263 SIV pol 15mers) and the use of whole PBMC cultured for short periods ex *vivo,* as a method of boosting immune responses.

In one control-immunised animal, monkey E22, which received multiple infusions of PMBC pulsed with SIV pol (and SIV gag), a modest induction of SIV gag and SIV pol-specific IFNγ ELISpot responses was detected. This animal subsequently had high levels of SIV gag- and pol-specific killing analysed at week 20, presumably from the boosting effect of the SHIV challenge. The efficiency of priming an immune response by OPAL infusion therefore seems feasible. These data were confirmed when whole blood was pulsed with HCV or HIV-1 drug resistant peptides, which efficiently induced high levels of CD4+ and CD8+ T cell responses as assessed by ICS. These data also demonstrate the feasibility of using whole blood as an antigen-presenting cell (APC) source, which would be more practical than PBMC or other more complex APC preparations (such as monocyte-derived dendritic cells) in the field.

Further modifications to the OPAL technique, such as the enrichment for APC and/or dendritic cells (DC) (40), would potentially enhance the immunogenicity of OPAL infusion as a therapeutic vaccine since DC cultured from PBMC of HIV-infected patients (41, 42) and SIV-infected animals (40) can elicit potent T-cell responses. Alternatively, the prospect of using whole blood rather than PBMC fractions as a means of delivering OPAL will certainly benefit a clinical setting, particularly for HIV-infected persons. Furthermore, a smaller whole blood sample may not require as high a concentration of peptide since 1 µg/mL is effective *in vitro* for whole blood analysis by ICS. It is also conceivable that direct intravenous infection of pooled peptides would mimic the immunogenicity of the OPAL effect The use of consensus HIV-1 clade peptide sets of gag and pol offers the broad epitopic breadth desired of an effective therapeutic vaccine for humans,. The immunogenicity of antigens that regulate viral replication, such as rev, tat, vpu, vif and vpr, which are poorly immunogenic by current vaccine approaches, should also be improved using this strategy. In addition, the general method of using blood or PBMC or other uncultured APC-containing fraction directly as an APC source immediately suggests the possibility of pulsing other sources of antigen (including but not limited to whole protein, DNA, live vector vaccines or cancer cell preparations) onto such APC populations prior to infusion. It is believed that such antigen-loaded cell APC populations could be more immunogenic (presumably by binding directly to abundant APCs) than administering the antigen by other common methods such as intramuscularly (where few APCs exist).

### EXAMPLE 4

### MATERIALS AND METHODS

### Animals

Male juvenile, colony-bred pigtailed macaques *(Macaca nemestrina,* aged 2-4 years) were studied. All animals were housed under PC3 biosafety conditions by trained animal technicians at the CSIRO Australian Animal Health Laboratory, Geelong. Prior to all procedures, animals were anaesthetised with ketamine (10 mg/kg, intramuscularly). Health and weight were routinely monitored. All conditions and protocols were approved by the CSIRO animal health and the University of Melbourne animal ethics committees.

### Pre-immunisations

To evaluate whether the OPAL method could boost T cell responses in animals with pre-primed responses. T cell responses were induced in macaques by administering 2 DNA vaccines expressing HIV or SIV structural genes followed by a FPV boost vaccine expressing similar HIV or SIV genes as previously described (16). DNA vaccines in saline were administered twice intramuscularly (0.5 mL to each anterior quadracep) at a dose of 1mg/dose. FPV boosts were delivered intramuscularly a dose of 5x10⁷ pfu.

### Isolation of plasma and peripheral blood mononuclear cells (PBMC) from whole blood

Blood was collected in 9 mL Na+ Heparin and 3 mL EDTA vacutainers from the femoral vein of each animal on study weeks prior to and after vaccination and SHIV challenge. Plasma samples were removed following centrifugation (800xg, room temperature, RT, 8 min; Beckman Coulter) and stored in 3x1.5-mL tubes at -70° C. Plasma collected in EDTA-anticoagulated blood was used for RNA extraction. Media (RPMI-1640 supplemented with penicillin, streptomycin and glutamine; Invitrogen) equal to the volume of plasma collected was added to the blood and mixed prior to PBMC isolation on Ficoll-Paque, used according to the manufacturer's instructions (Amersham Pharmacia). PBMC were washed twice (500xg, 10° C, 6min) and resuspended in 1 mL media for counting (Beckman Coulter Counter®) in preparation of immunological assays.

### Overlapping peptides

15-mer peptides (>80% purity) overlapping by 11 amino acids spanning the entire gag (125 peptides), pol (260 peptides) and nef (21 peptides) of SIV_{mac239} and env (211 peptides) protein of SHIV_{SF162P3} (NIH AIDS Research and Reference Reagent depository) (Tables 1-4) were pooled for each protein by solubilising each lmg peptide aliquot in 10-40 µL of DMSO to final concentrations: SIV_{mac239} gag (670 µg/mL or 730 µg/mL); pol (304 µg/mL), and; nef (4.762 mg/mL), and; SHIV_{SF162P3} env (330 µg/mL), stored at -70° C until use. 18mer peptides overlapping by 11 amino acids spanning the entire HCV open reading frames (NIH AIDS Research and Reference Reagent depository) were pooled into 2 pools (HCV1 and HCV2) encompassing the structural and regulatory genes of HCV. Non-overlapping 17mer peptides spanning known sites of HIV-1 drug resistance mutations were specifically designed and purchased from Mimotopes Australia (Figure 12).

### SIV antigens for in vitro analyses

Whole inactivated SIV (WI SIV) and its control (supernatant from Hut78-CLE cell-line used to culture the WI SIV) (AIDS Vaccine Program, National Cancer Institute, MD) were stored at - 70° C until use.

### In vivo cytotoxic T lymphocyte killing

At weeks 10, 15 and 20 following the initial vaccination, PBMC from the macaques were isolated from 40-50 mL blood, as described above. 25 mL sterile injectable saline was infused into the animals immediately after blood sampling to prevent hypovolemia. PBMC were resuspended in PBS and divided into 3 or 4 equal volumes, 0.5 mL. Cells were pulsed with SIVgag, pol, nef or SHIVenv peptide pools (10 µg/mL) or DMSO (volume of equal to the volume of SIVgag), in PBS for 90 min at 37° C, or on ice, with regular mixing. To subsequently track each peptide-pulsed cell population by flow cytometry, each peptide/DMSO-pulsed population was then labelled with a concentration of CFSE or SNARF (Molecular Probes). 5 mM CFSE stock in DMSO at-20° C was thawed and diluted in PBS. Neat SNARF stock was dissolved in 83 µLDMSO to make 1mM and diluted in PBS. Table 1 shows the final concentrations of each dye, Cells were mixed thoroughly and stained for 10 min in a 37° C waterbath, followed by one wash in RF5 then PBS (500xg, 10°C, 6min). All peptide/DMSO-pulsed cells for each animal were pooled in 1.5 mL saline for re-infusion into the femoral vein. 3 mL blood was sampled from the opposite femoral vein at 5 min, and at 4 and 16hr following infusion. Red blood cells were lysed with 10 L FACS Lysing Solution (BD Biosciences), incubated for 10min at room temp. Cells were pelleted and washed twice with PBS (800xg, RT, 7min), and fixed with 1-2 mL 2% paraformaldehyde (Figure 1).

To determine whether cell populations were being selectively killed, 10⁶ events gated live lymphocytes were collected by flow cytometry (FACSort Calibre, BD). CFSE and SNARF fluorescence were detected by FL1 and FL2 channels, respectively. For analysis, killing was expressed as the percentage of target versus control peptide-pulsed cell clearance. In the event of acquiring unequal labelled populations by flow cytometry at 5 minutes post-OPAL infusion, the degree of killing was subsequently scaled with respect to the initial population ratios obtained at 5 minutes. PBMC were also analysed prior to, and following, OPAL-infusion by 1FNγELISpot and ICS to detect whether T cell immune responses were enhanced.

### SHIV challenge of macaques

To assess the efficacy to the vaccines, each macaque was inoculated intrarectally with SHIV.ₘₘ₂₂₉ (5×10⁴ TCID₅₀mL on CD8-depleted *M. nemestrina* PBMC) in 0.5 mL doses over 2 days (total 10⁵ TCID50/mL) 18 weeks after the initial immunisation, as previously described (32).

### Quantification of viral SHIV RNA by reverse-transcriptase real-time PCR

*RNA extraction:* To detect SHIV RNA in macaques following SHIV challenge, total RNA was initially extracted from stored plasma samples from anti-coagulated blood collected in EDTA with QIAamp® Viral RNA commercial kit (Qiagen) as previously described (32). Briefly, plasma samples were centrifuged (500×g, RT, 10min) to remove cells (preventing DNA contamination). 140 µL plasma RNA coupled to Carrier RNA in Buffer AVL and 96-100% ethanol was centrifuged and bound to a filter membrane. 60 µL RNA was eluted with Buffer AW1 and AW2 through a spin column. All reagents except ethanol supplied by kit.

*Reverse-transcriptase PCR:* 10 µL RNA was then reverse transcribed into _{c}DNA, in duplicate, with the reaction mixture (20 µL): 2.9 µL RNAse/DNAse-free water (Promega); 3 µL 10x TaqMan buffer A (Applied Biosystems); 6 µL MgC1₂ (25nM) (Applied Biosystems); 1.5 µL Random Hexamers (diluted 1/2; Applied Biosystems); 6µl dNTPs (2.SnM; Promega); 1.5 µL; Promega); 0.5 µ L Rnasin (40 U/mL; Promega); 0.1 µL MMLV-RT superscript (200U/mL; Invitrogen), for one thermal cycle: 25° C (15min) → 42° C (40min) → 75° C (5min) (GeneAmp PCR System 9700, Applied Biosystems). A third test per sample was set up to assess the presence of SHIV DNA contamination, using the same reaction mix excluding MMLV-RT superscript. SIV RNA standards (33) were serially diluted and reverse-transcribed in duplicate (limit of detection, 1500 copies/mL) .

*Real-time PCR:* cDNA was amplified with reaction mixture (20 µl): 141µl RNAse/DNAse-ftee water (Promega); 2µL 10× PCR buffer II (Applied Biosystems); 1 [µL MgC1₂ (Applied Biosystems); 1 µL SL03 SIVgag (20pmo1/µL); 1µL SL04 SIVgag (20 pmo1/□L); 0.3 _{µ}L SL07 molecular beacon 0.5µL Tag Gold (Applied Biosystems) as previously described (33). Reaction temperature was initially raised and held at 95 °C for 10 min to activate Tag Gold enzyme, followed by 45 thermal cycles: 95° C (15 sec) → 55° C (30 sec) → 72 °C (30 sec). Real-time analysis was performed on amplicon detection at 55° C (30 sec) stages by Sequence Detector software v.1.6.3 (Applied Biosystems).

### CD4+T cell counts

To assess the depletion of CD4+ T cells following SHIV challenge, 200 µL whole blood was incubated with 5 µL PE-conjugated anti-human CD3, 5 µL FITC-conjugated anti-human CD4, 5 µL PerCP-conjugated anti-human CD8 (clone SP34; L200, and; Leu-2a, respectively; BD Pharmingen) monoclonal antibodies for 20 min in dark, RT. Red blood cells were lysed with 2 mL FACS Lysing Solution (BD Biosciences) and fixed as described in method 2.8. 50,000 total events were collected by 3-colour FACScan Calibre® and CD4+ and CD8+ T cell counts expressed as the percentage of gated lymphocytes.

### Analysis of stimulation or Induction of SHIV, HCV and peptides derived from resistant HIV-1 strains by the whole blood OPAL techniques

in a separate experiment to assess (a) whether peptide-pulsed whole blood (as compared to PBMC which had be used previously) could be effectively used as an immune stimulant and (b) whether the OPAL technique could stimulate *de novo,* un-primed, immune responses, selected SHTV-infected macaques were infused with either whole blood pulsed at 5 µg/mL for 1hr with either a series of overlapping 15mer SHIV peptides (3 pools) or a series of overlapping 18mer HCV peptides (2 pools) and a series of non-overlapping 17mer peptides encompassing known mutations induced by HIV-1 drugs as illustrated in Figures 9-12.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

### TABLES

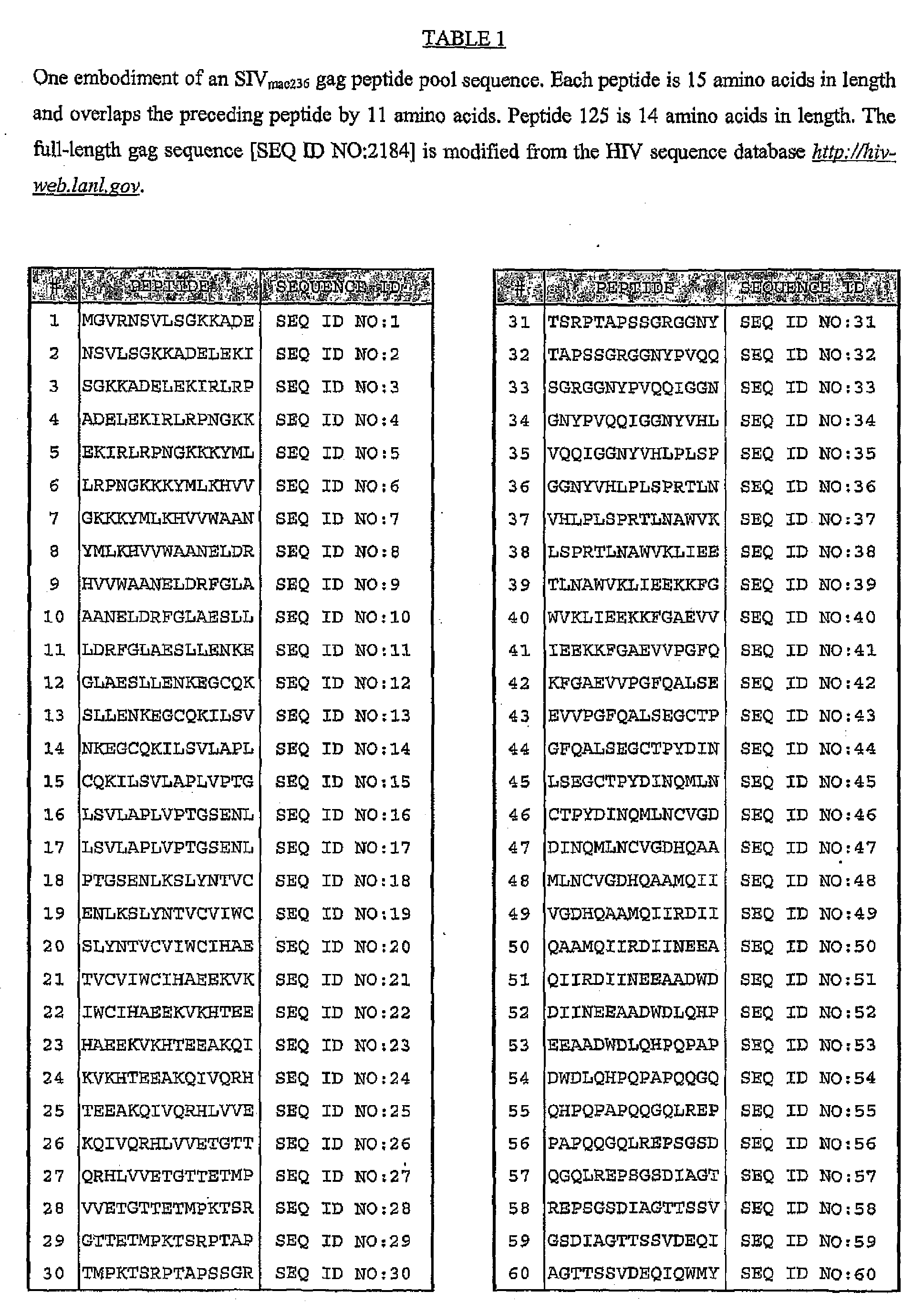

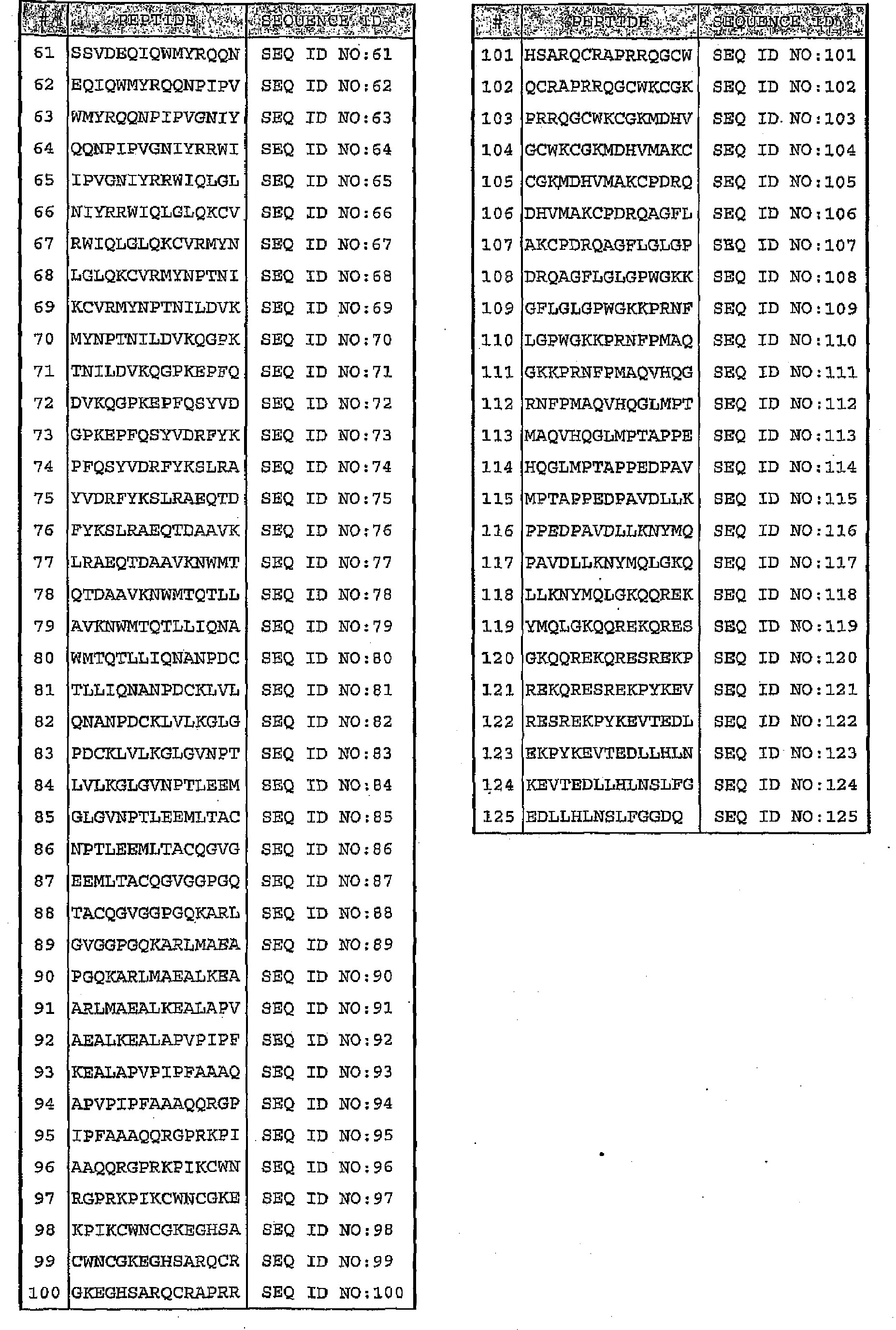

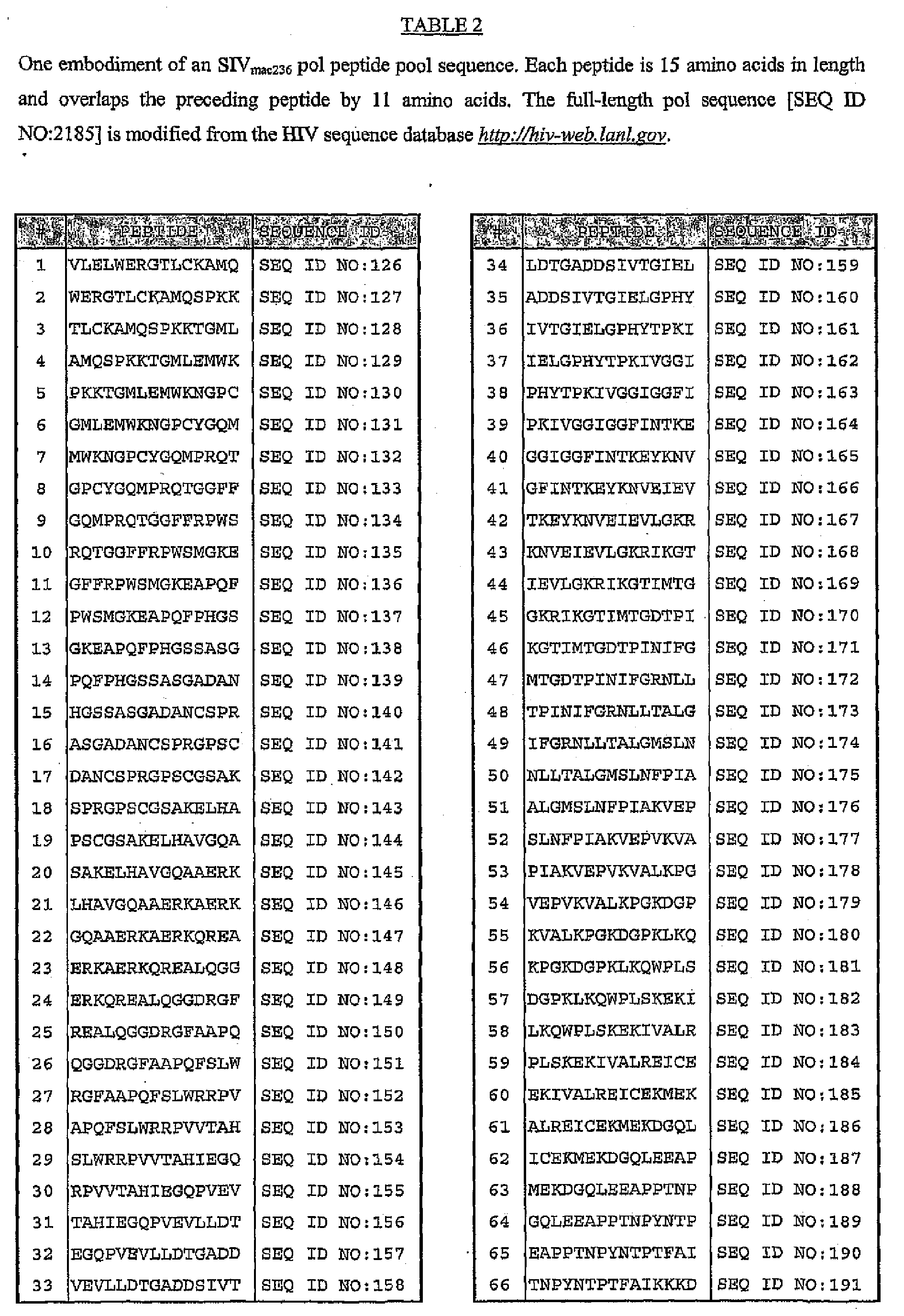

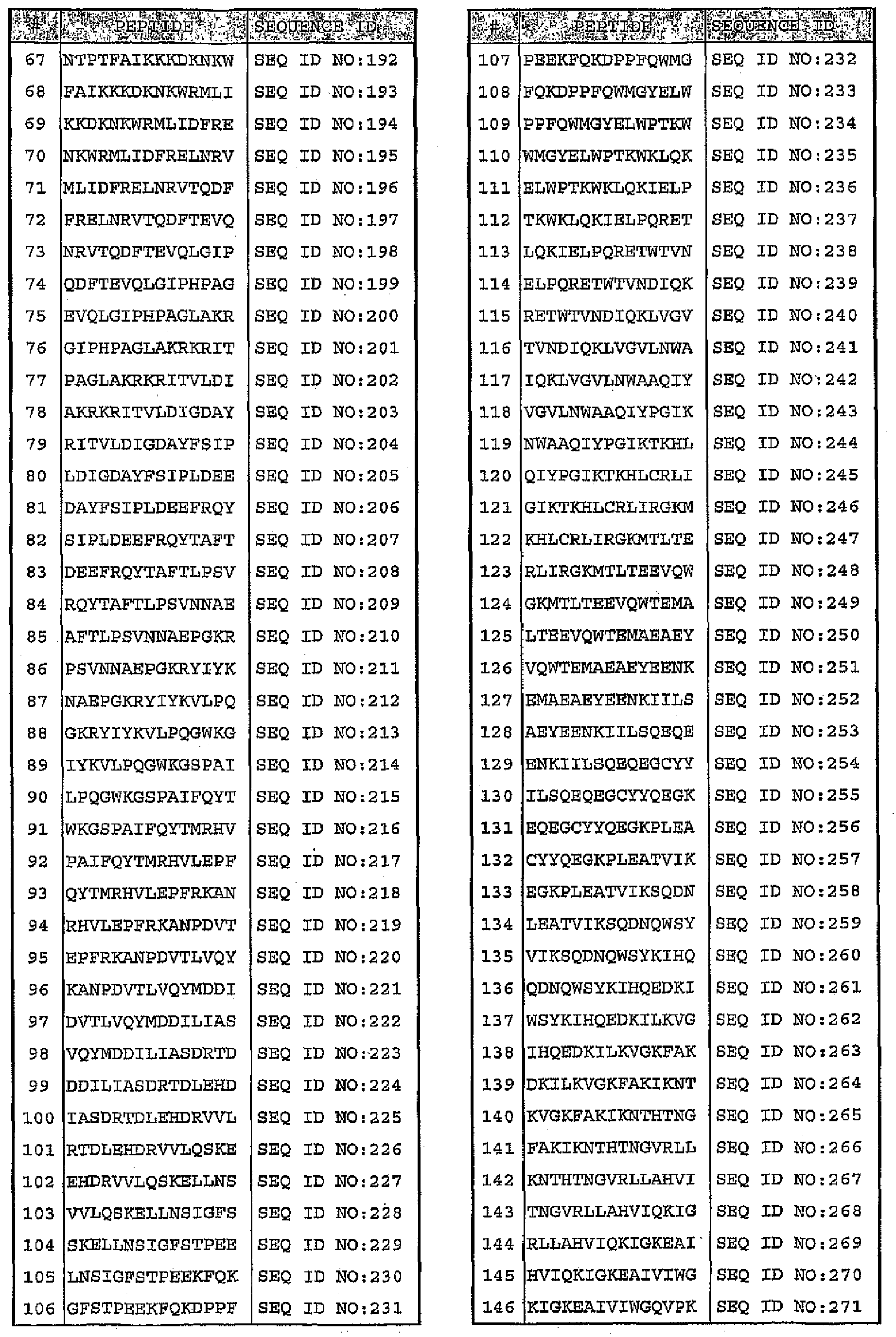

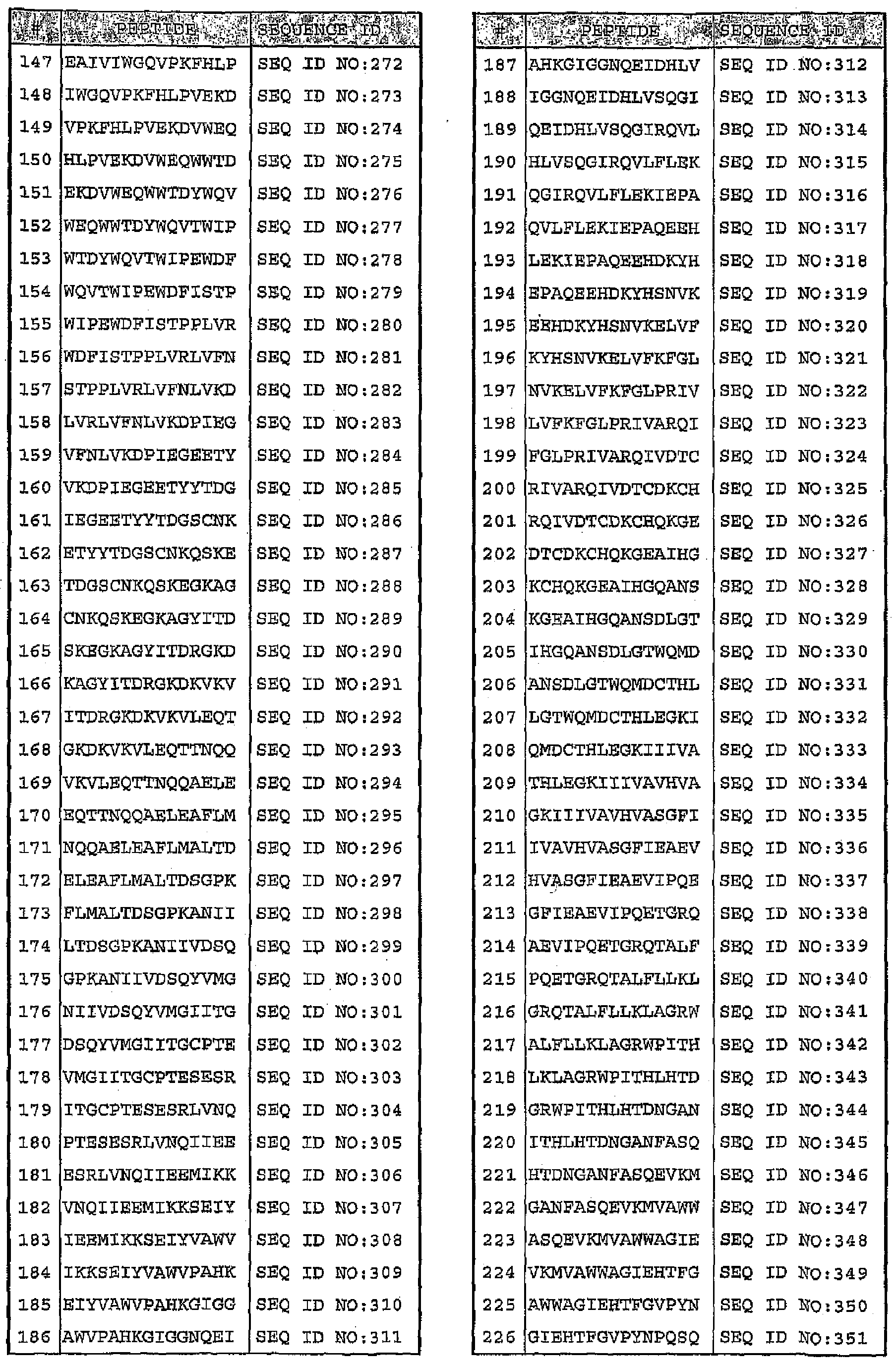

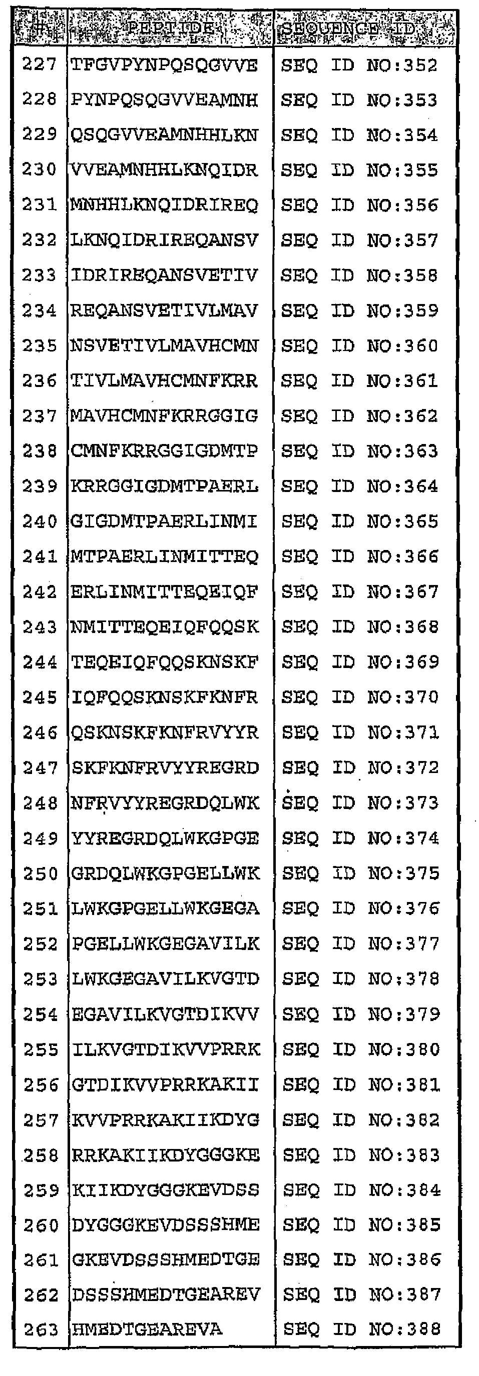

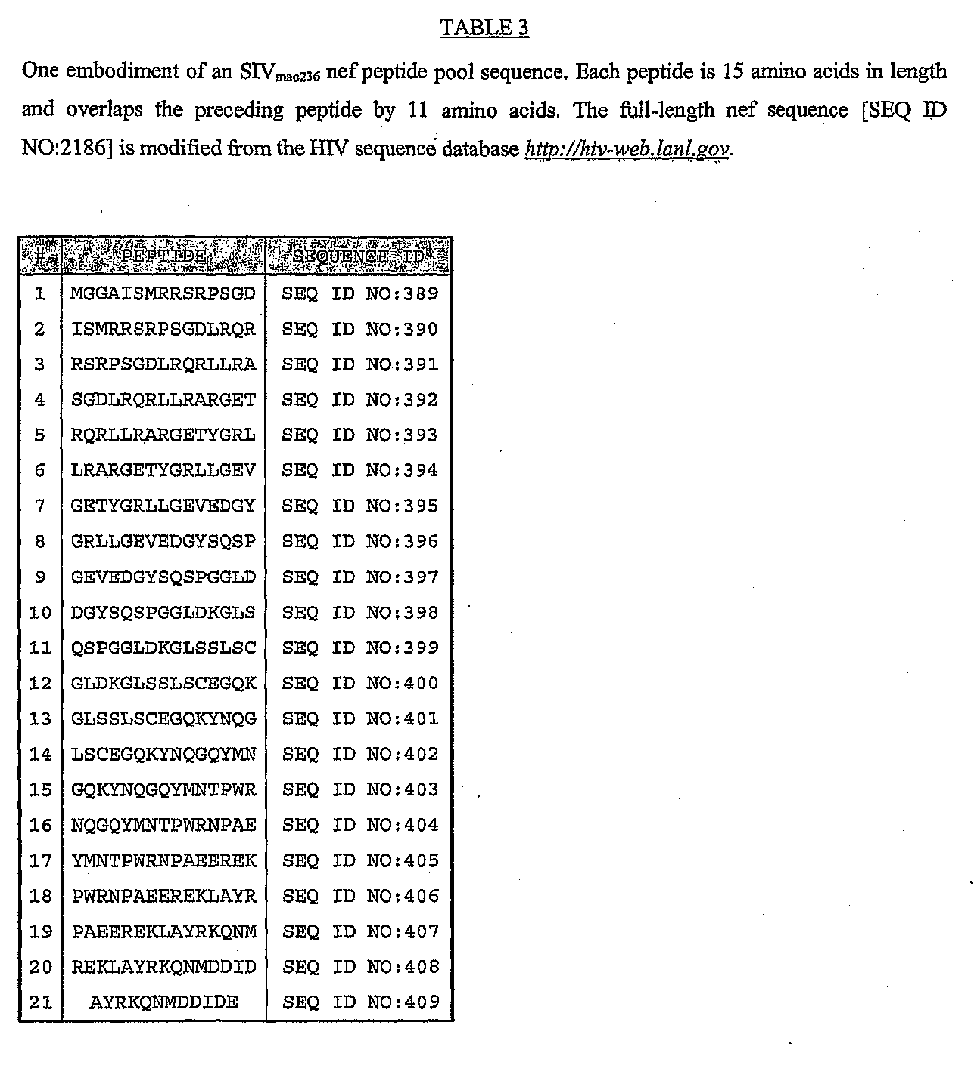

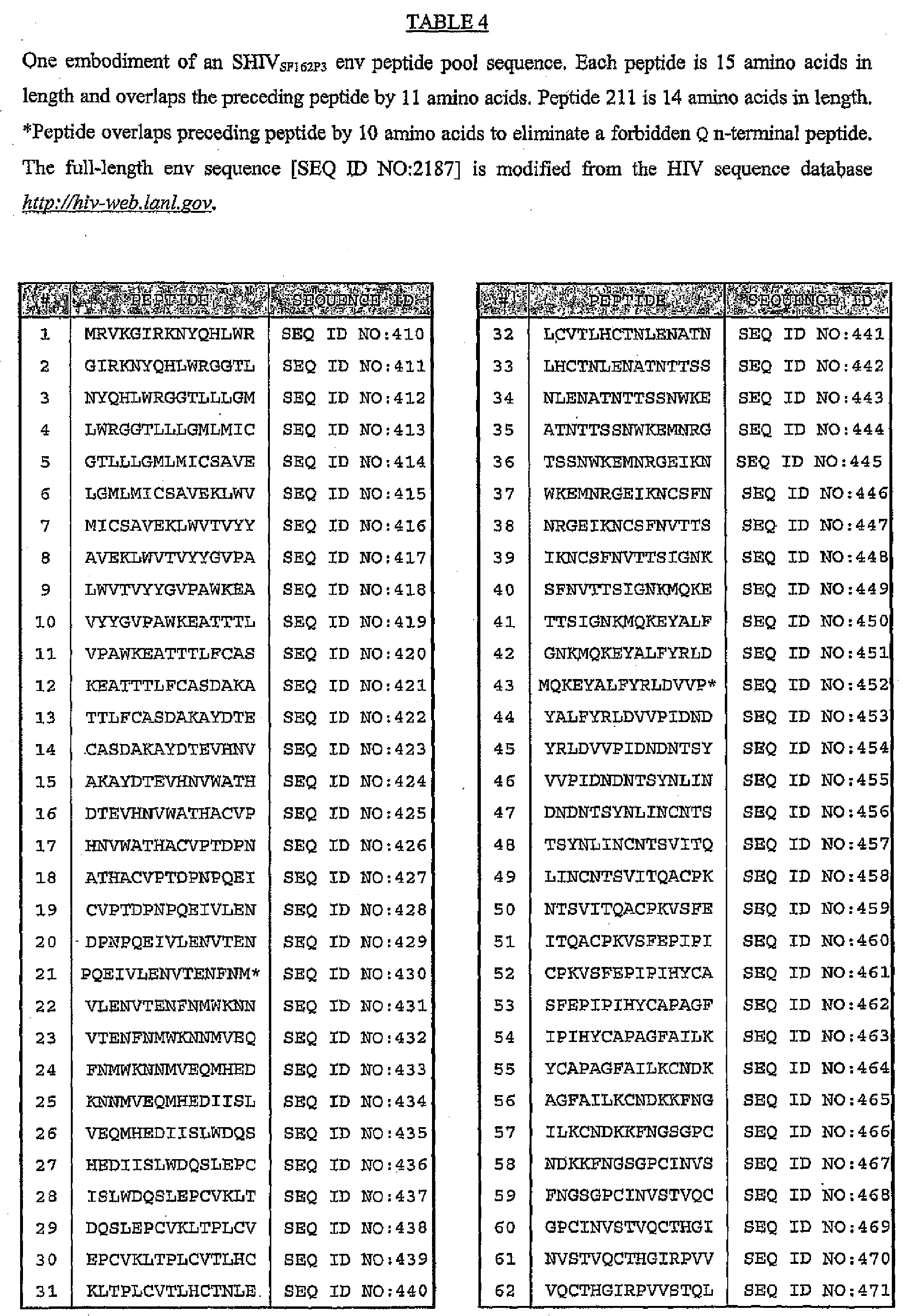

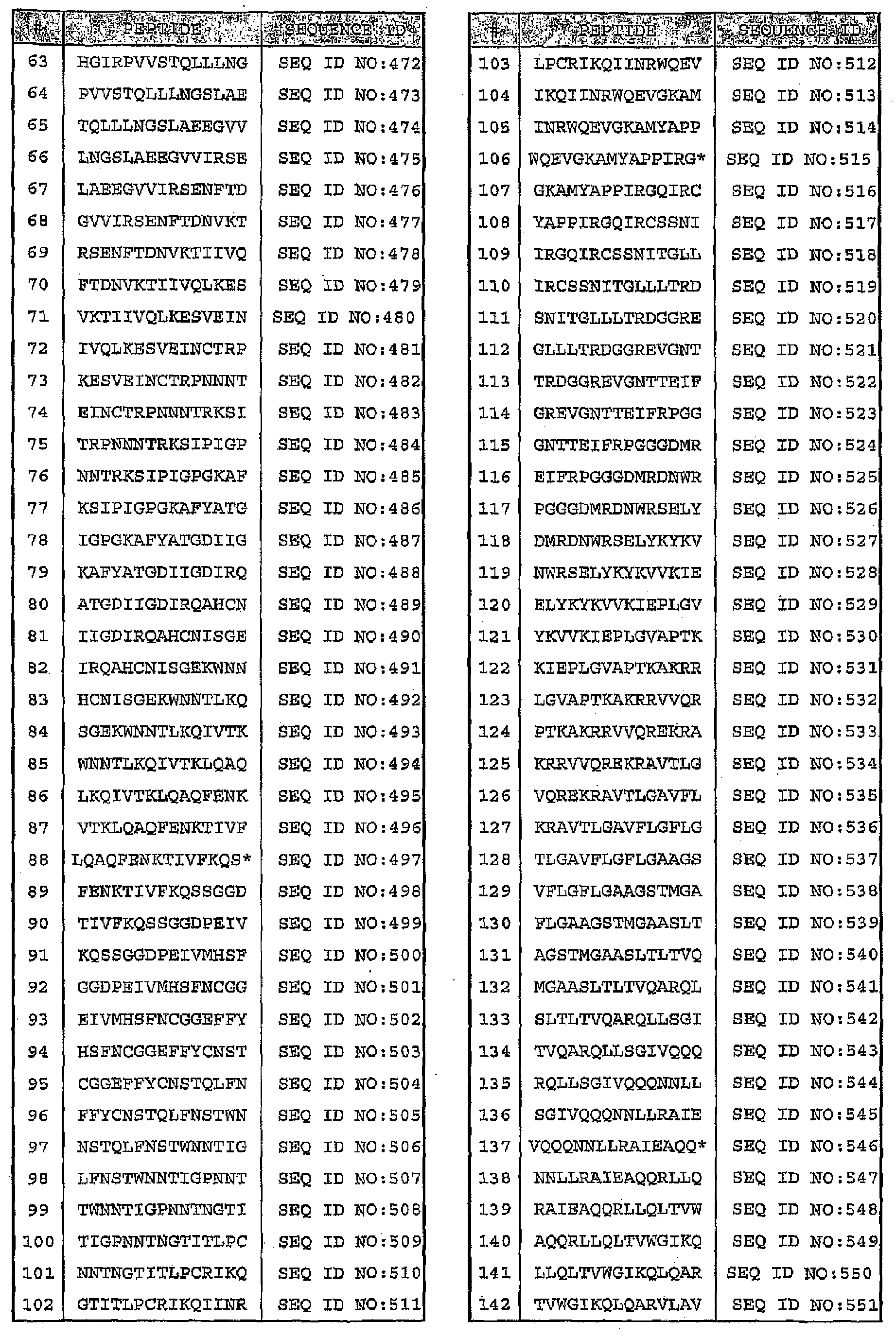

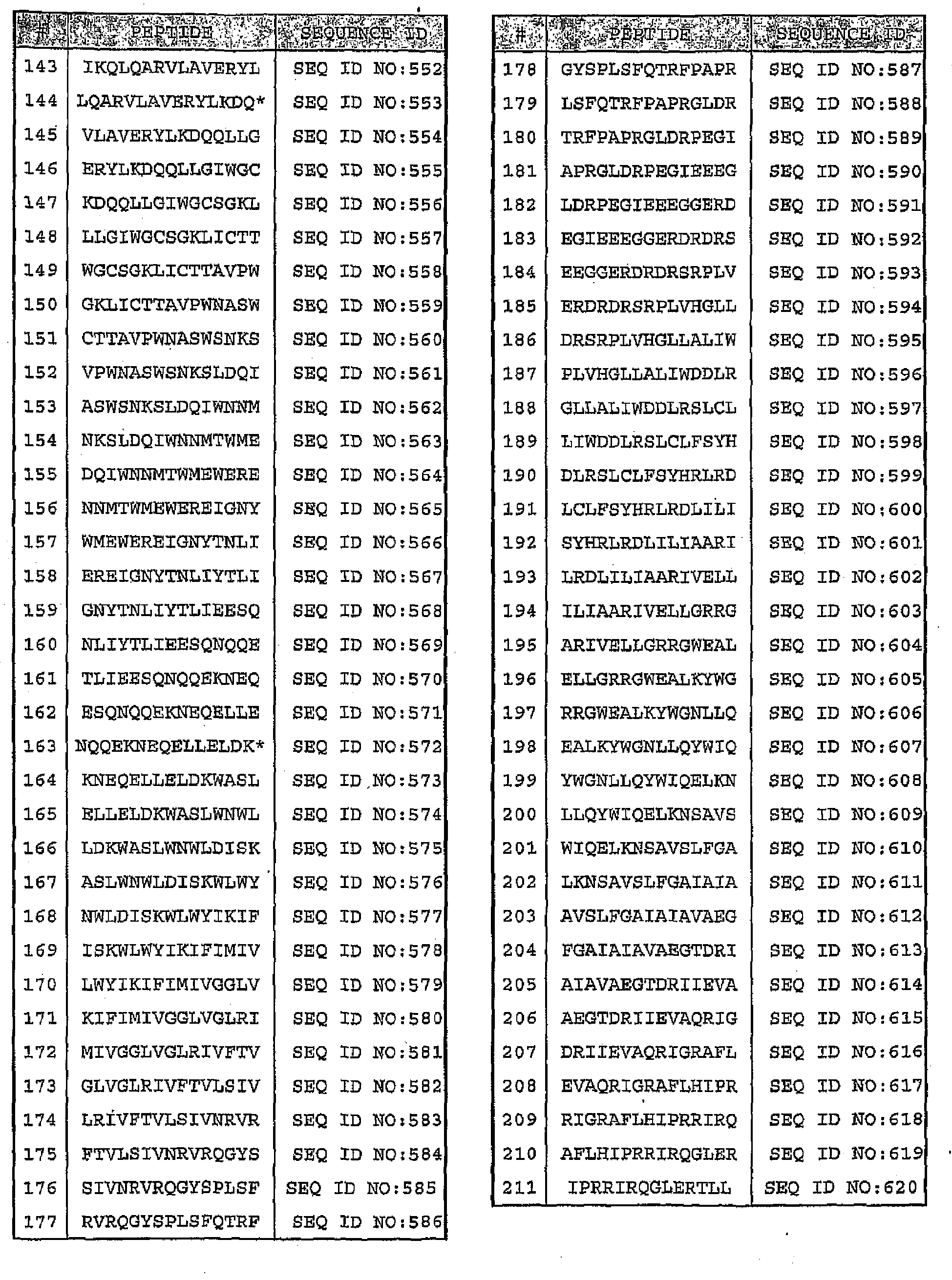

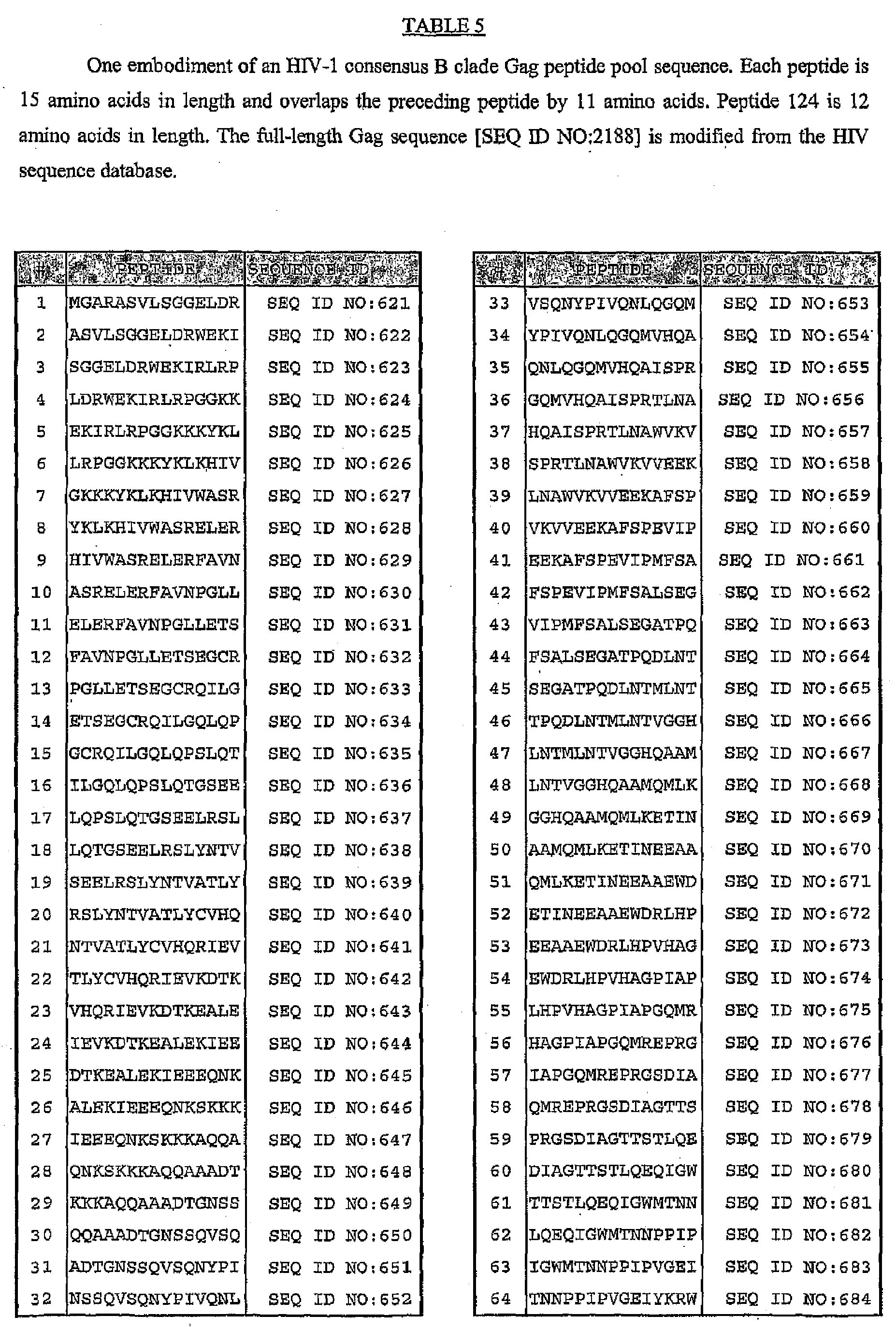

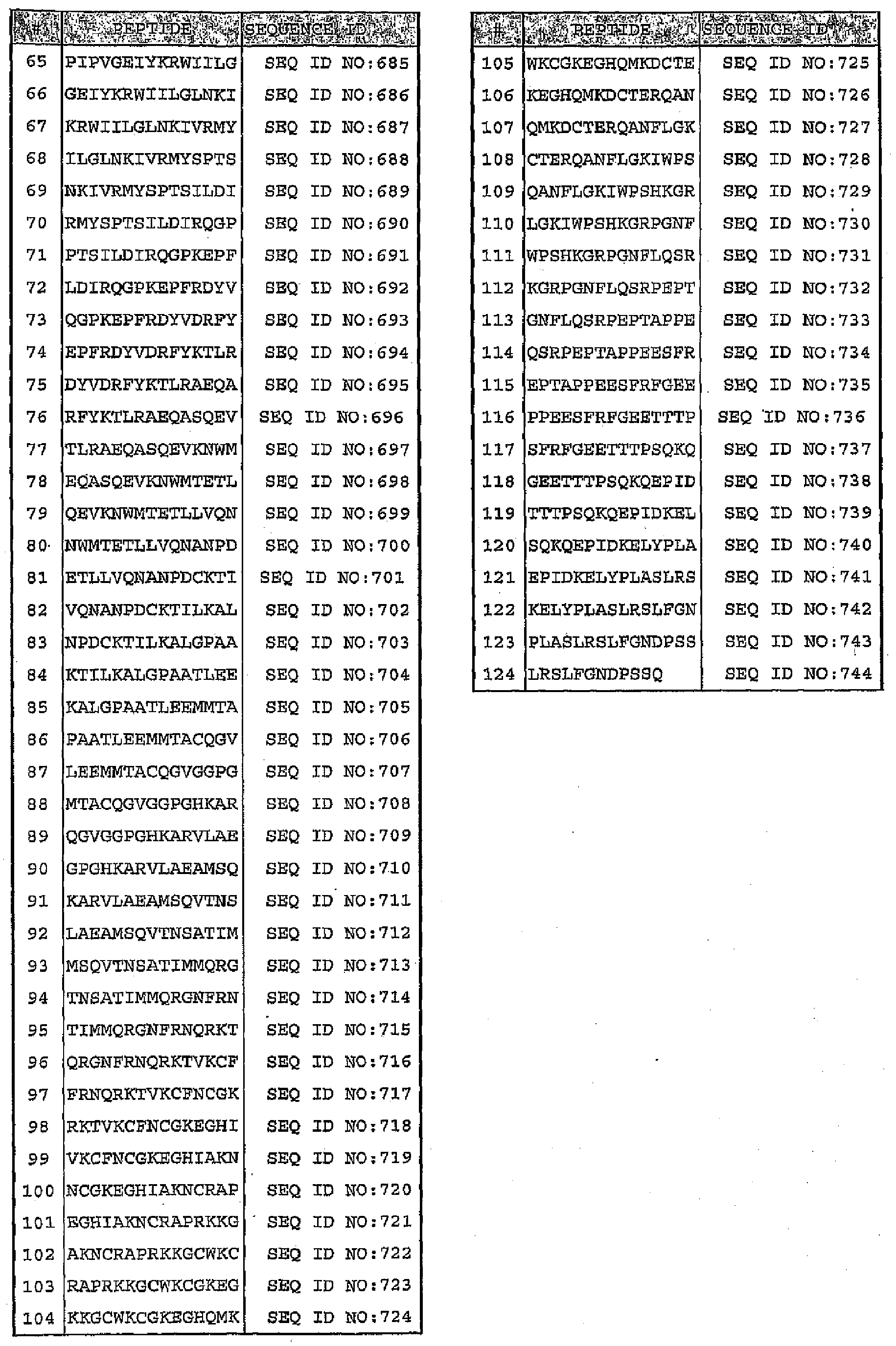

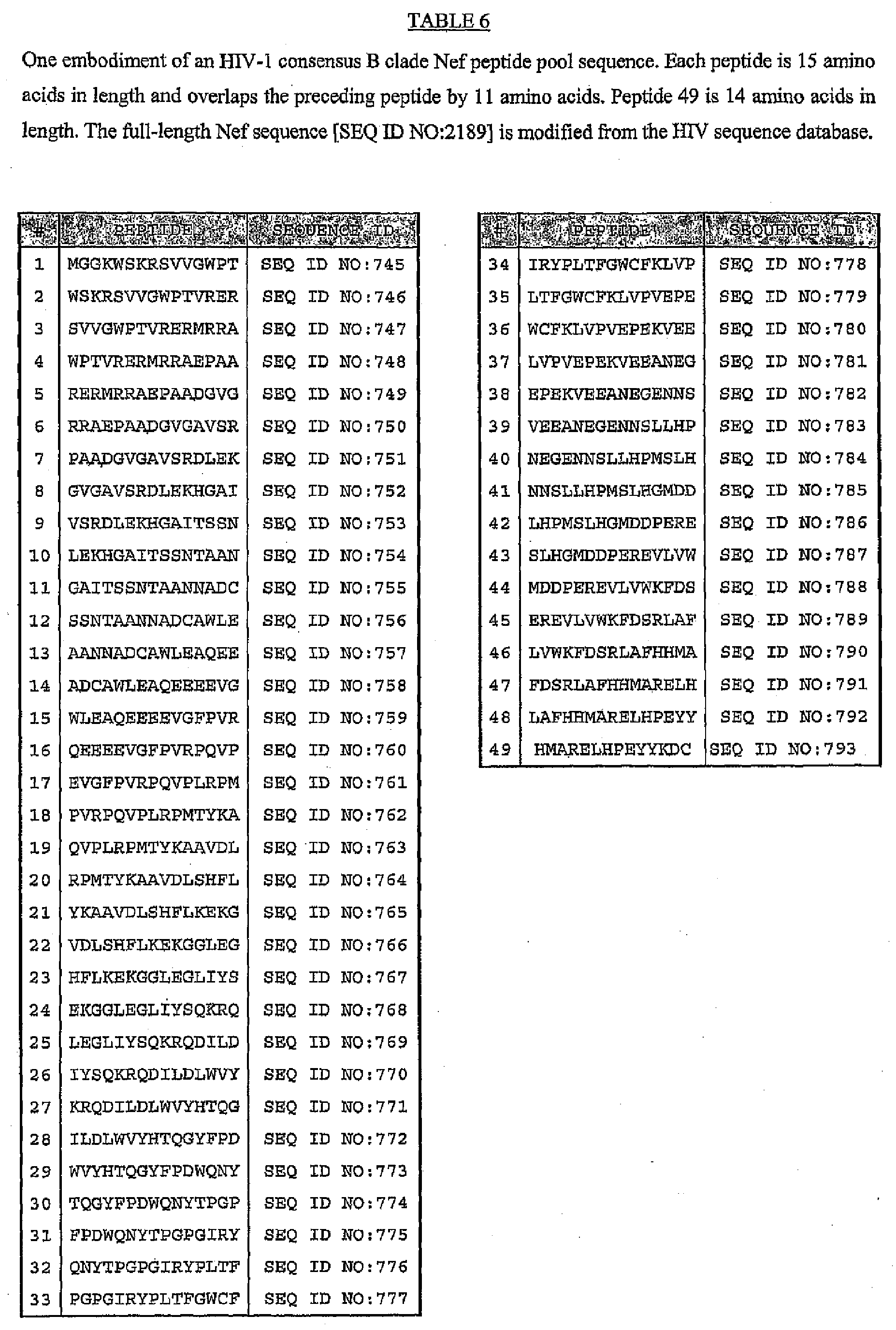

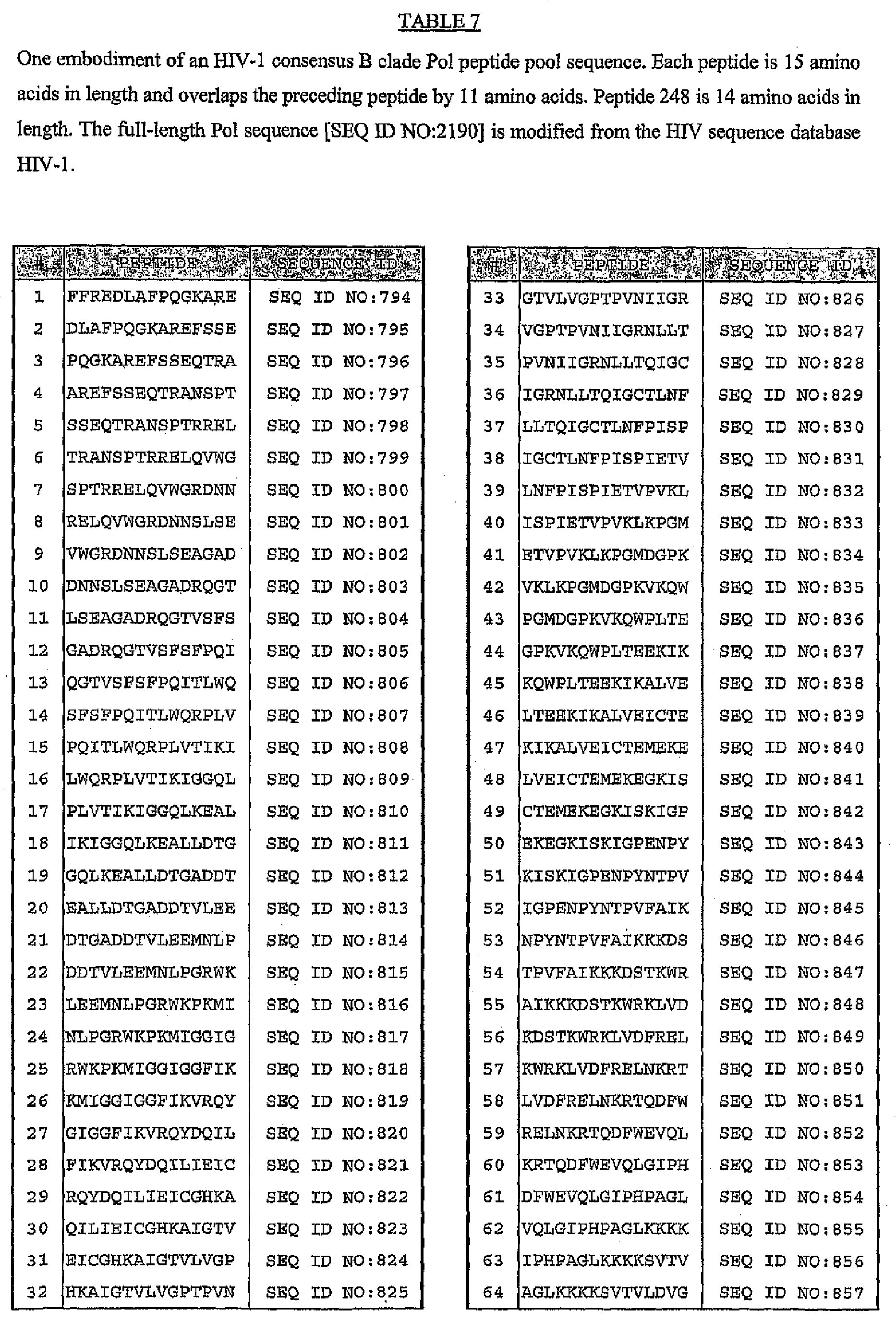

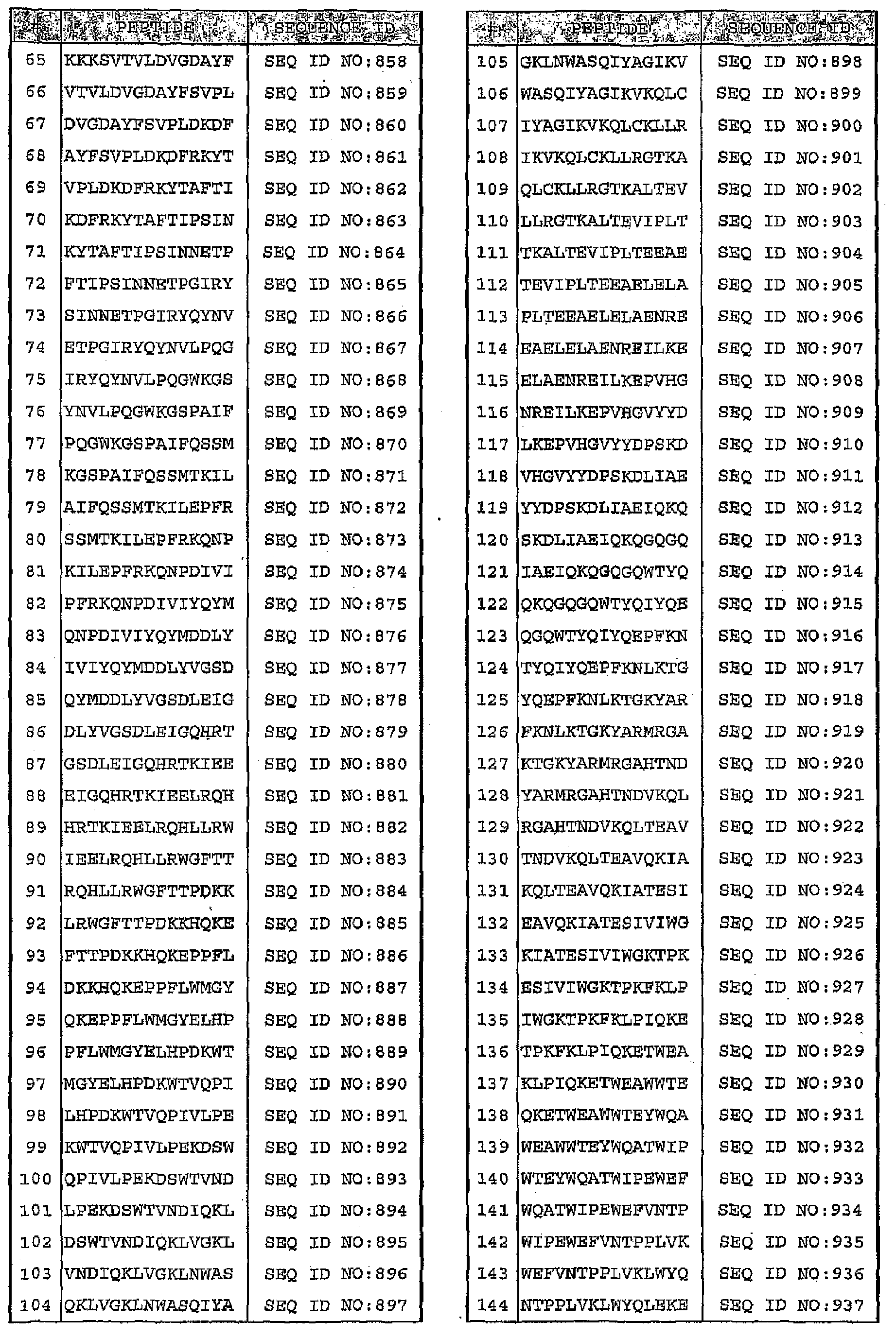

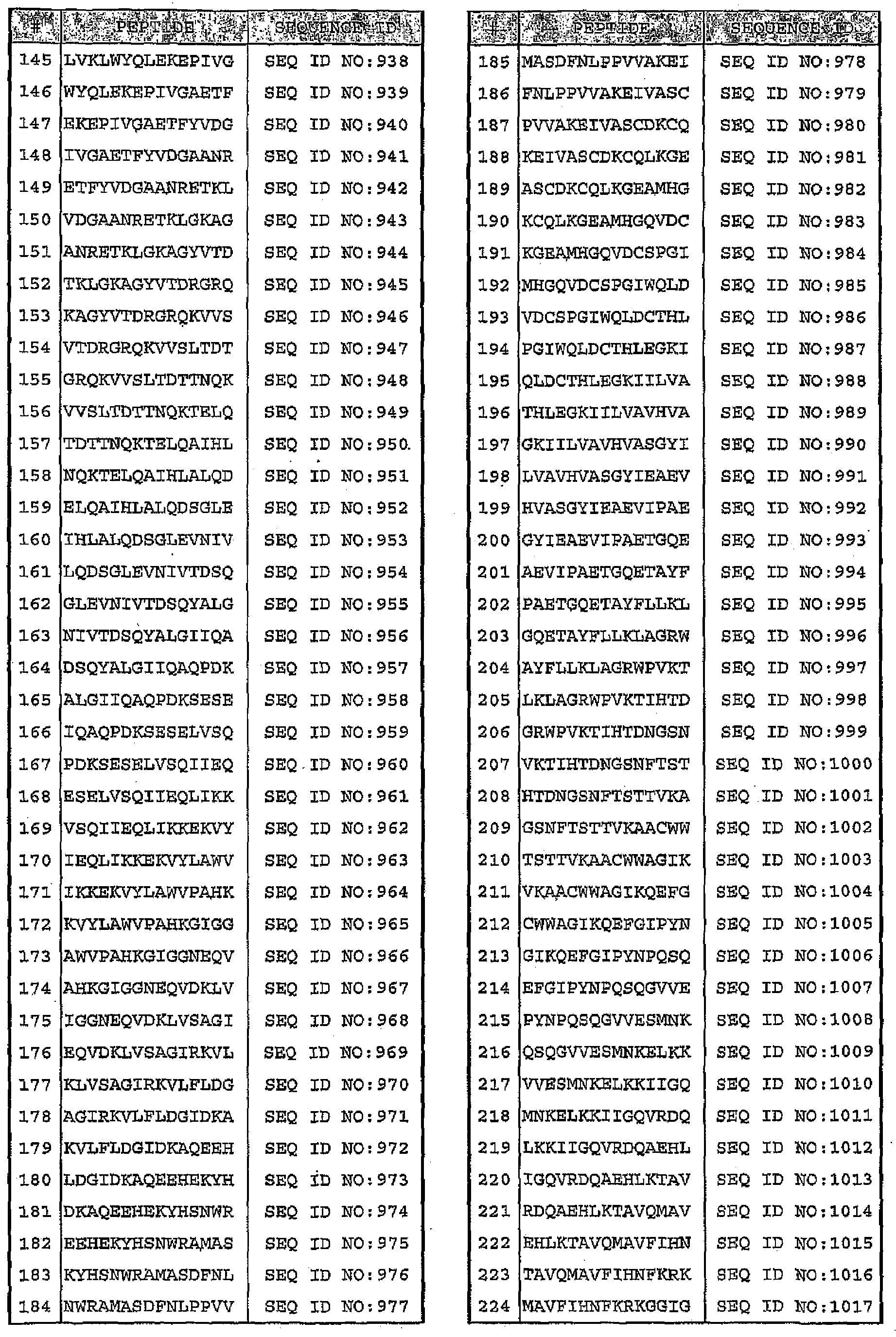

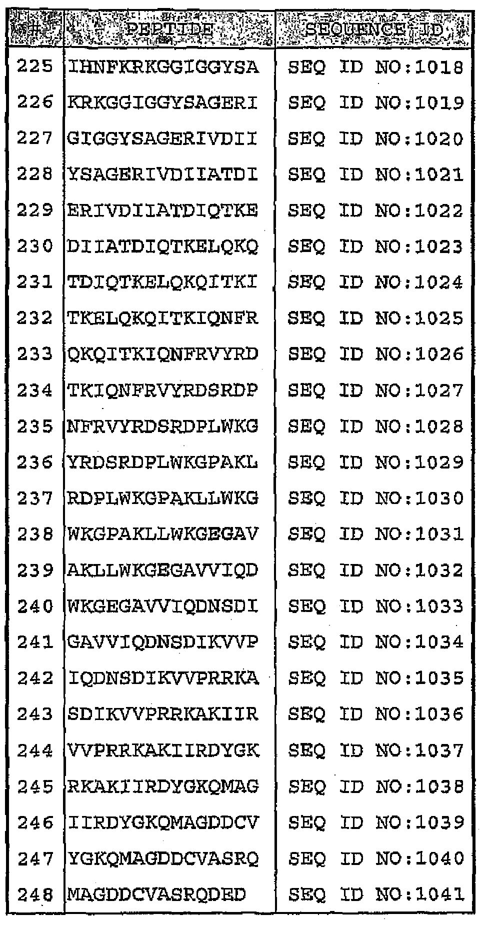

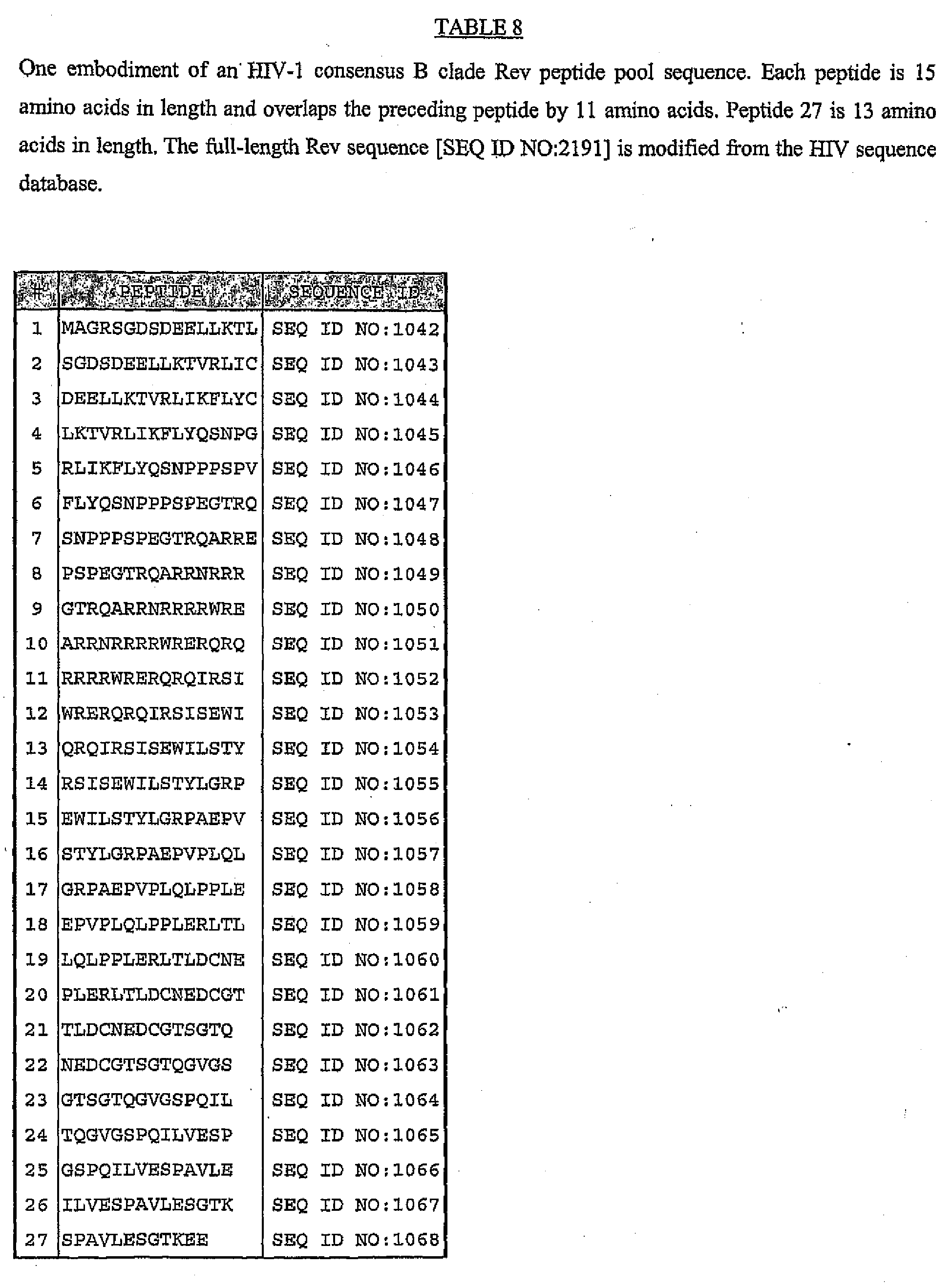

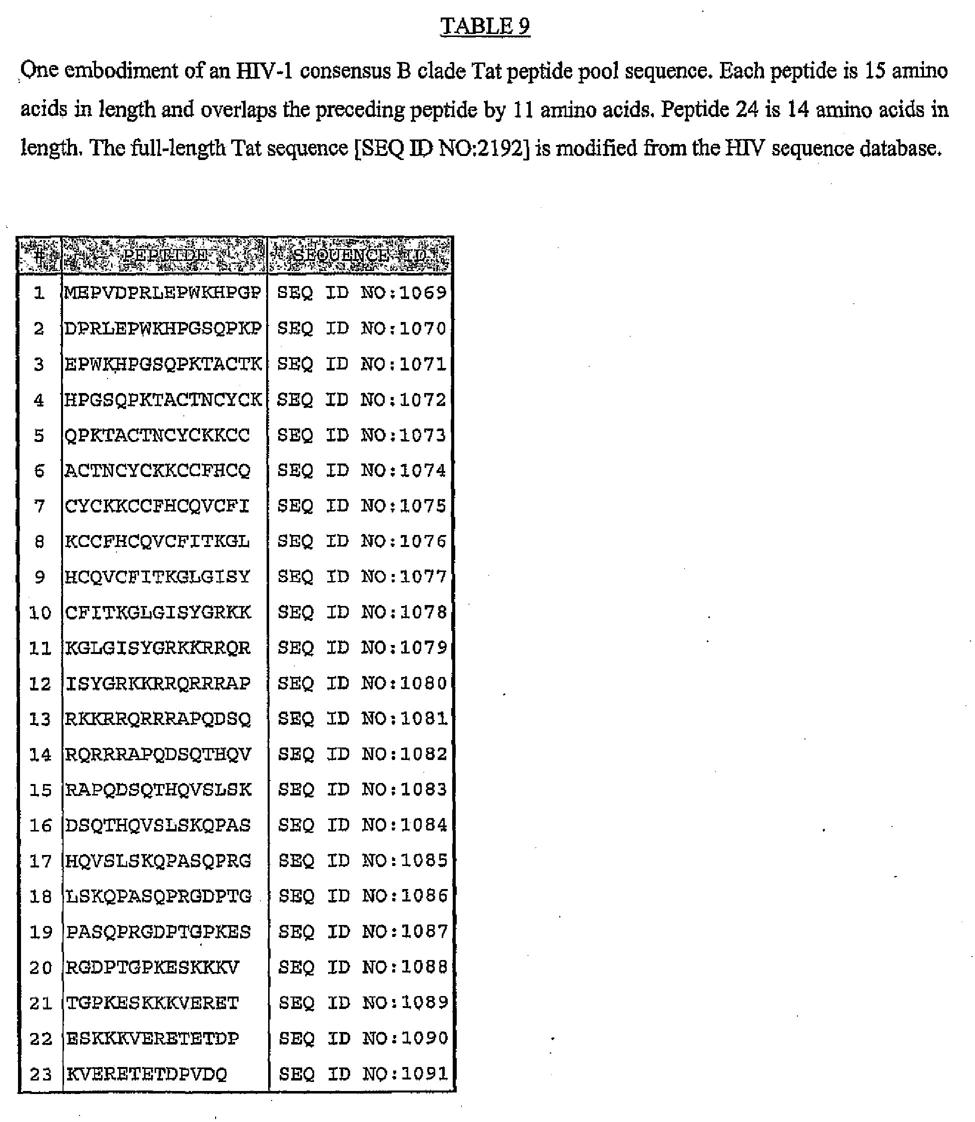

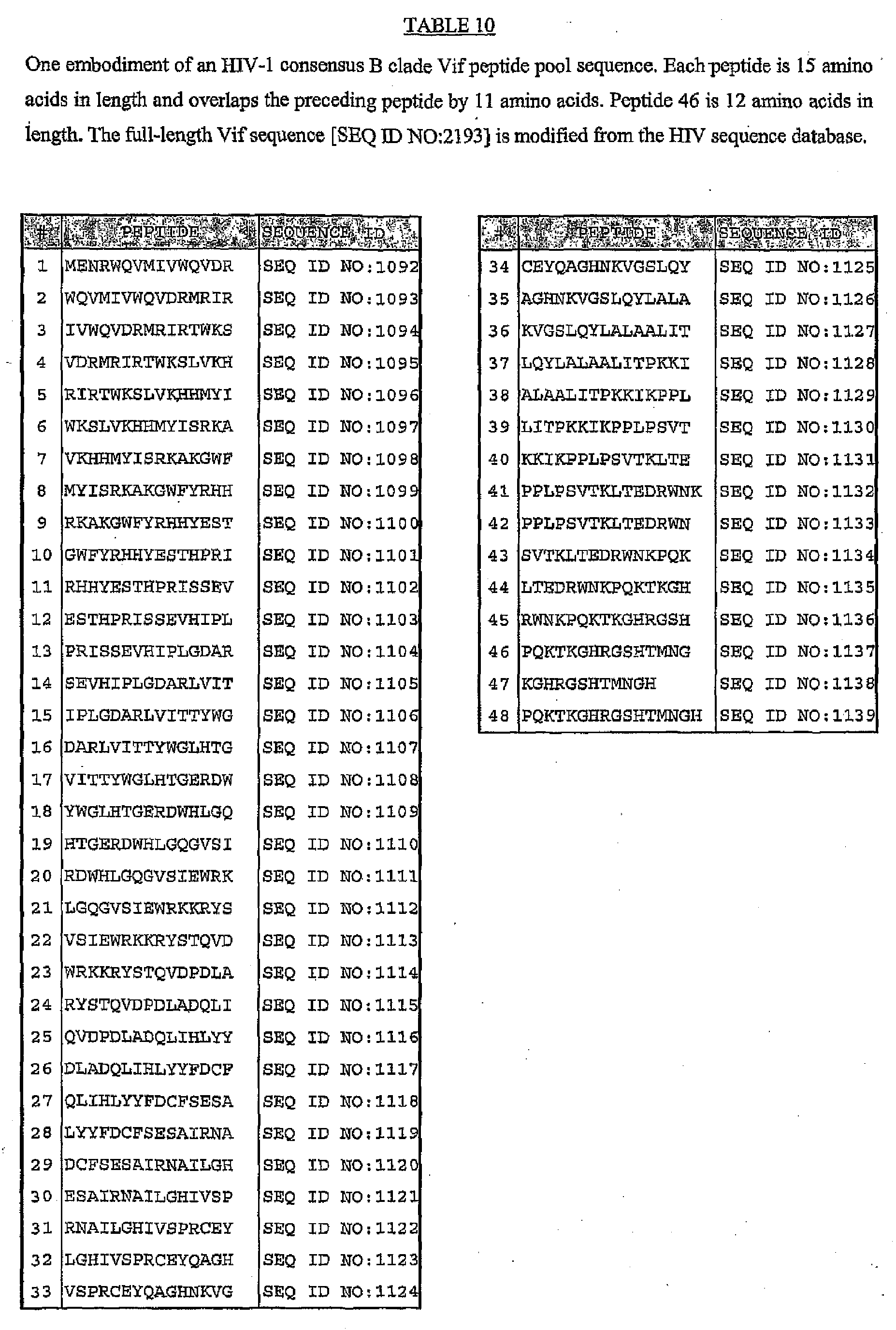

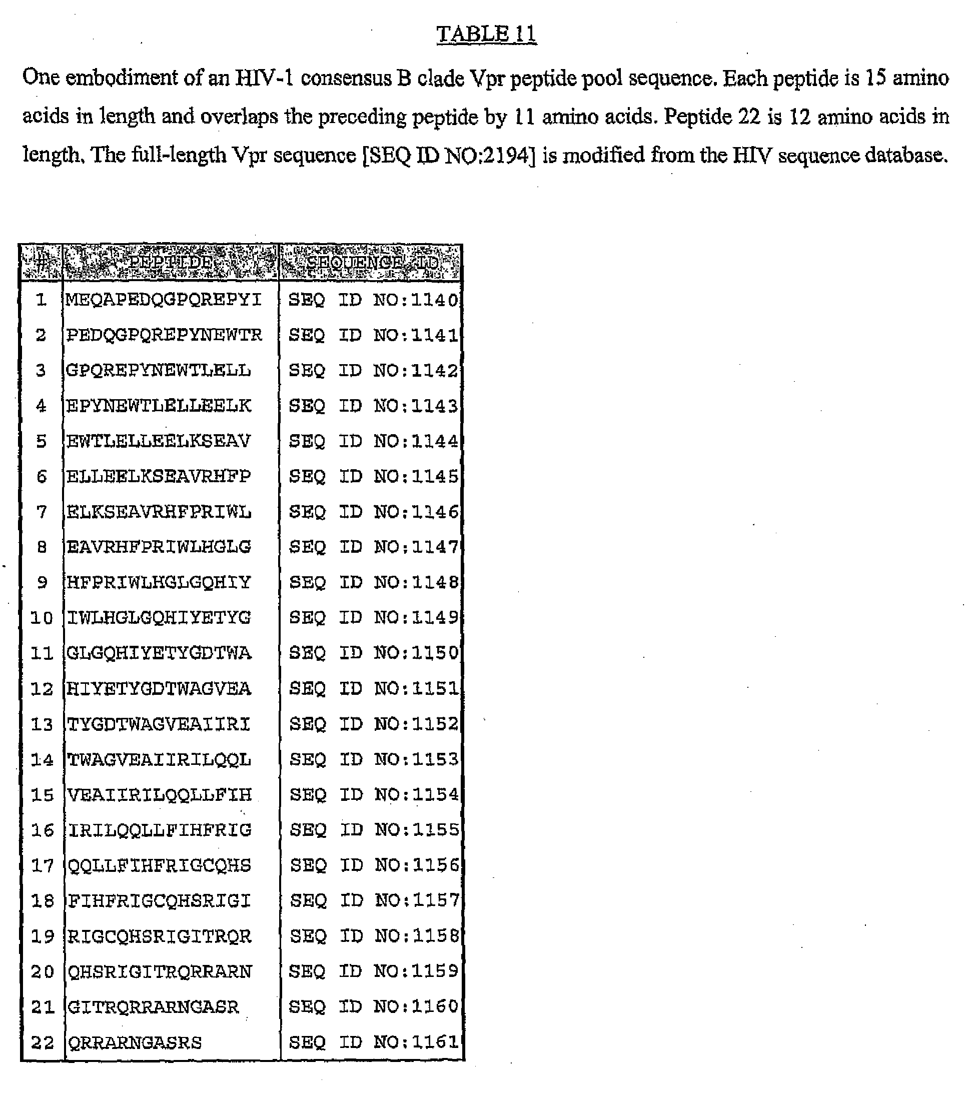

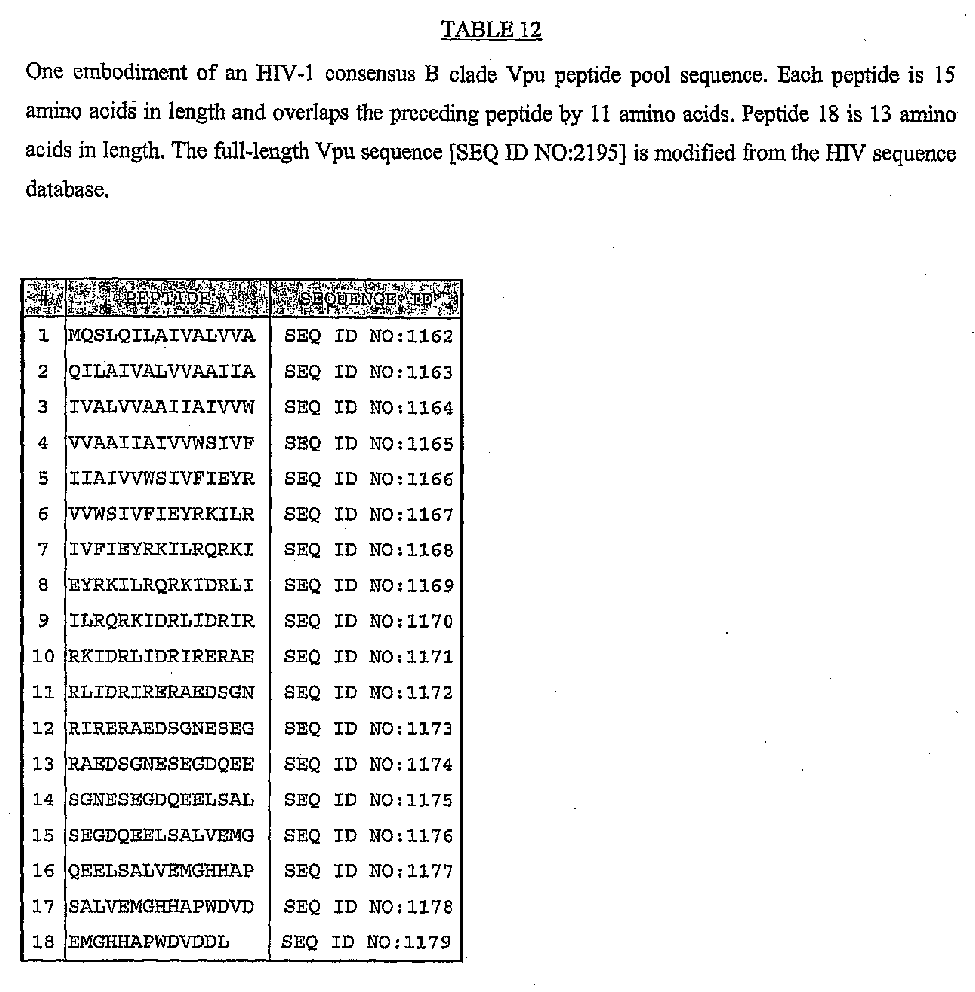

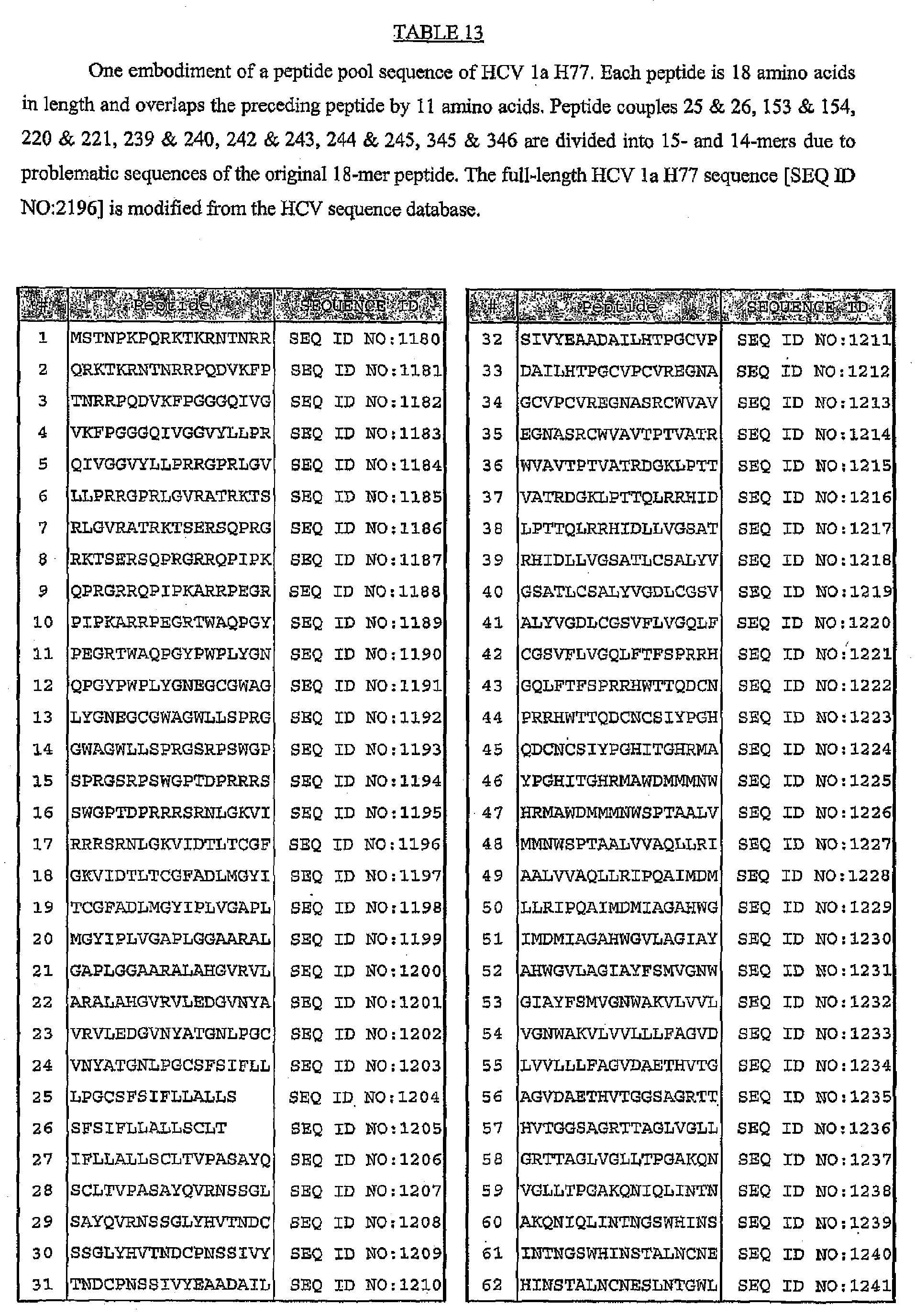

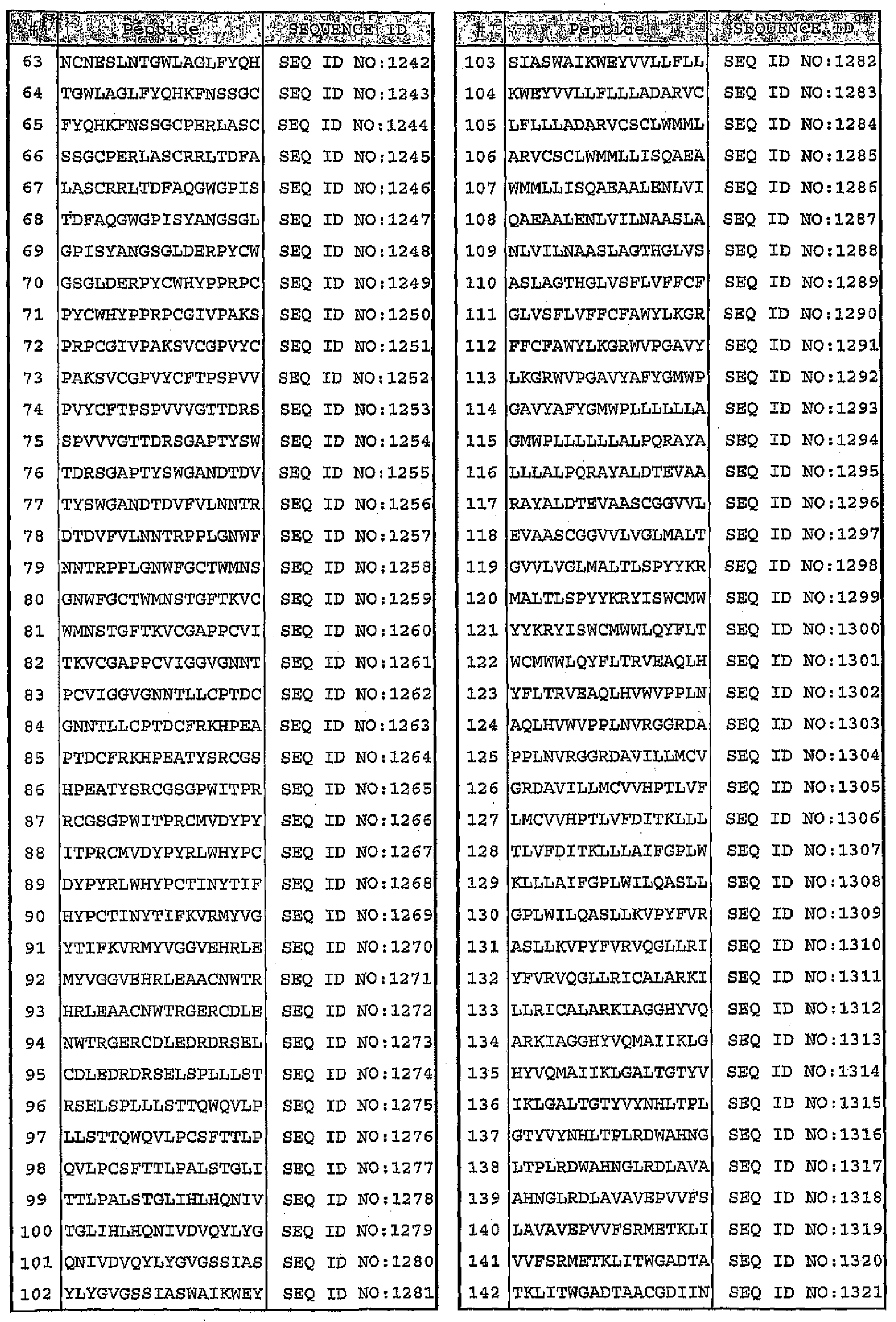

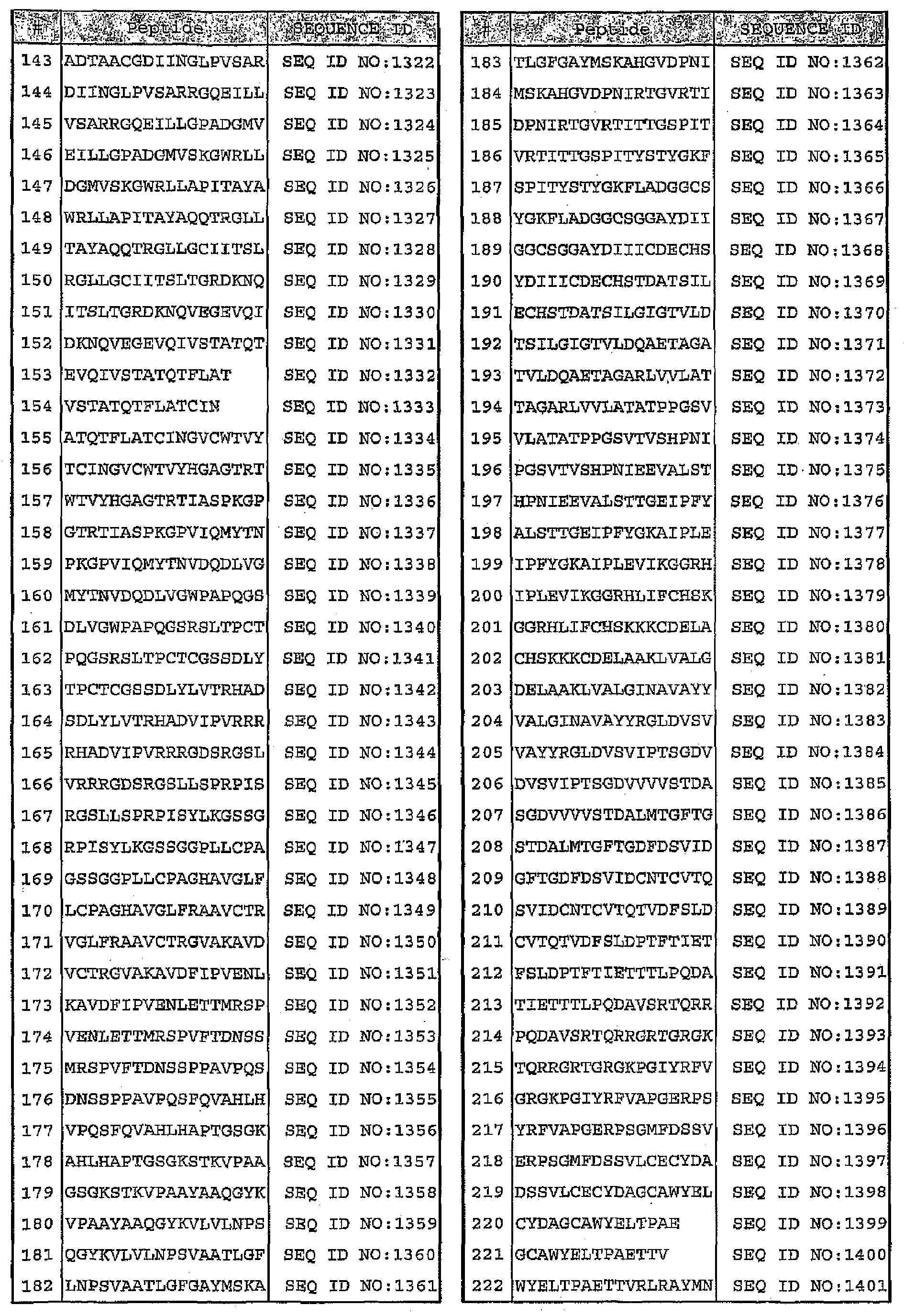

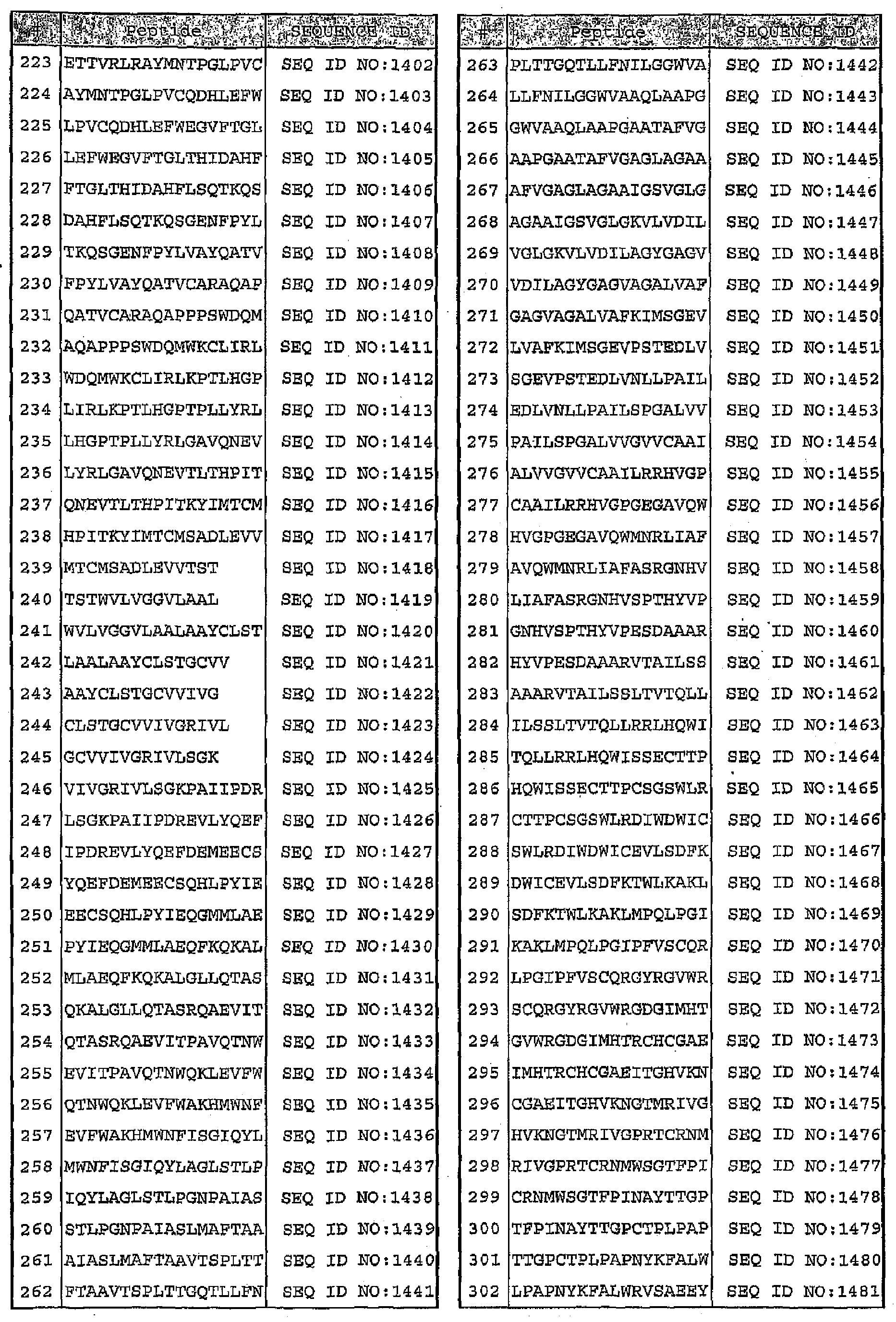

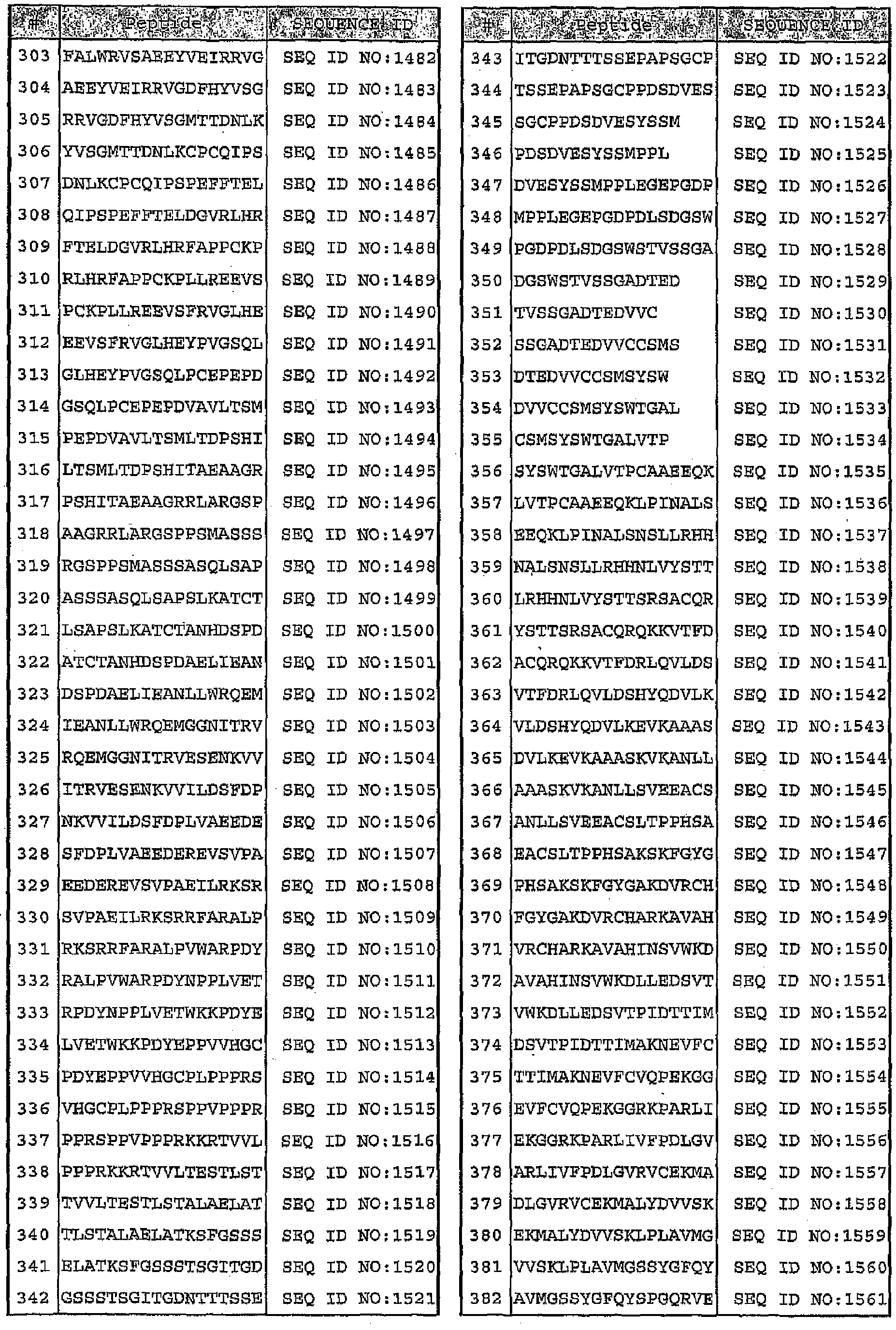

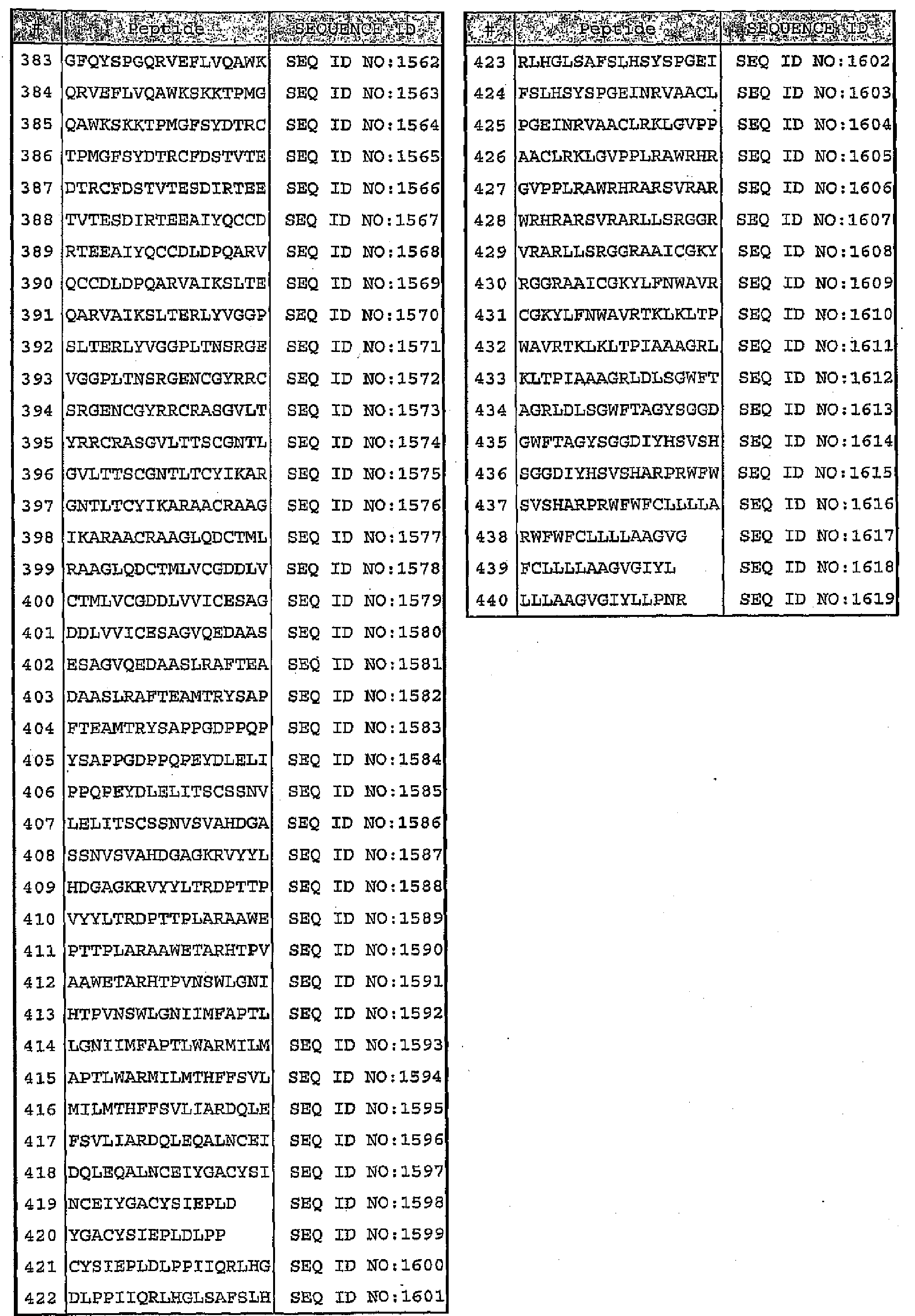

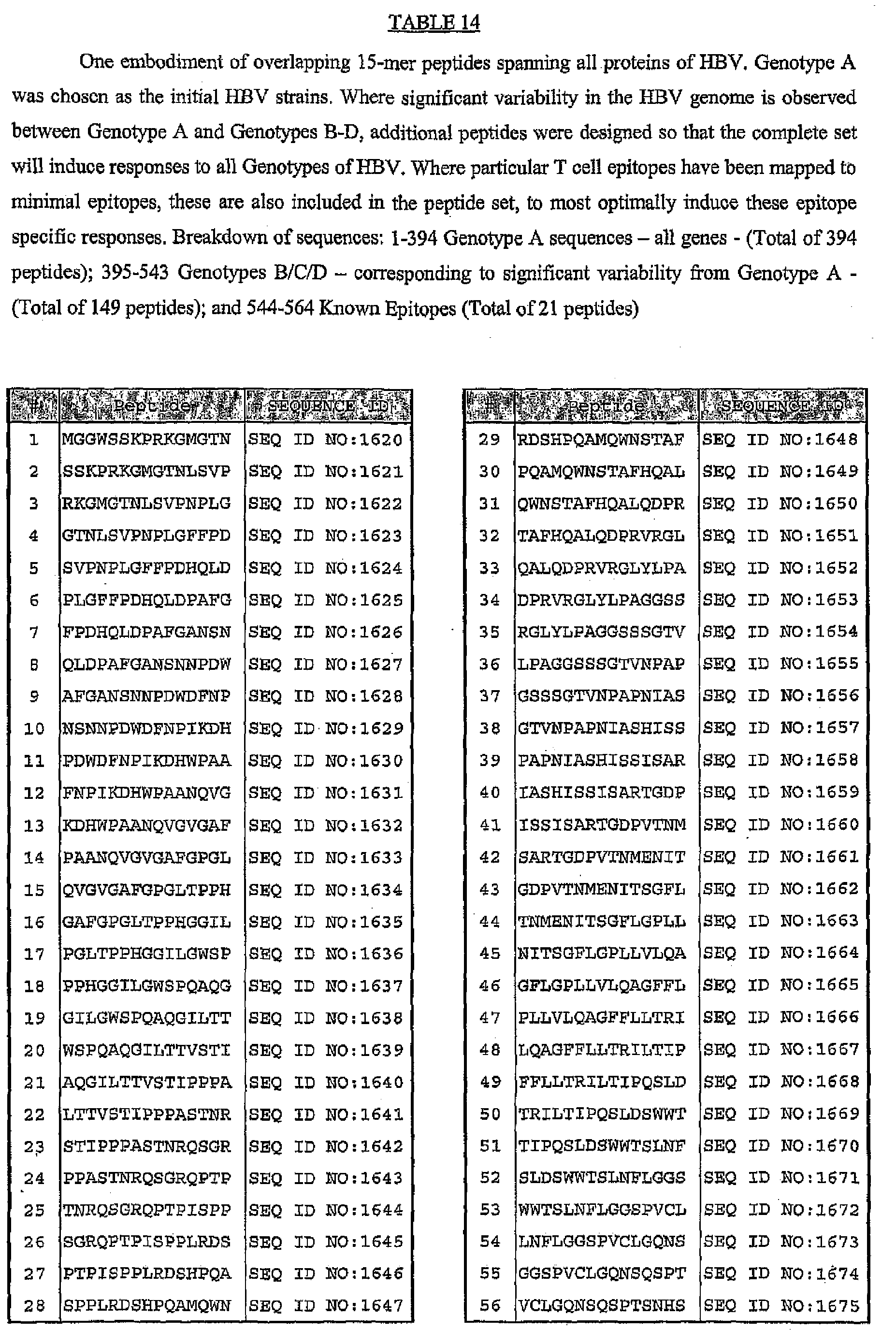

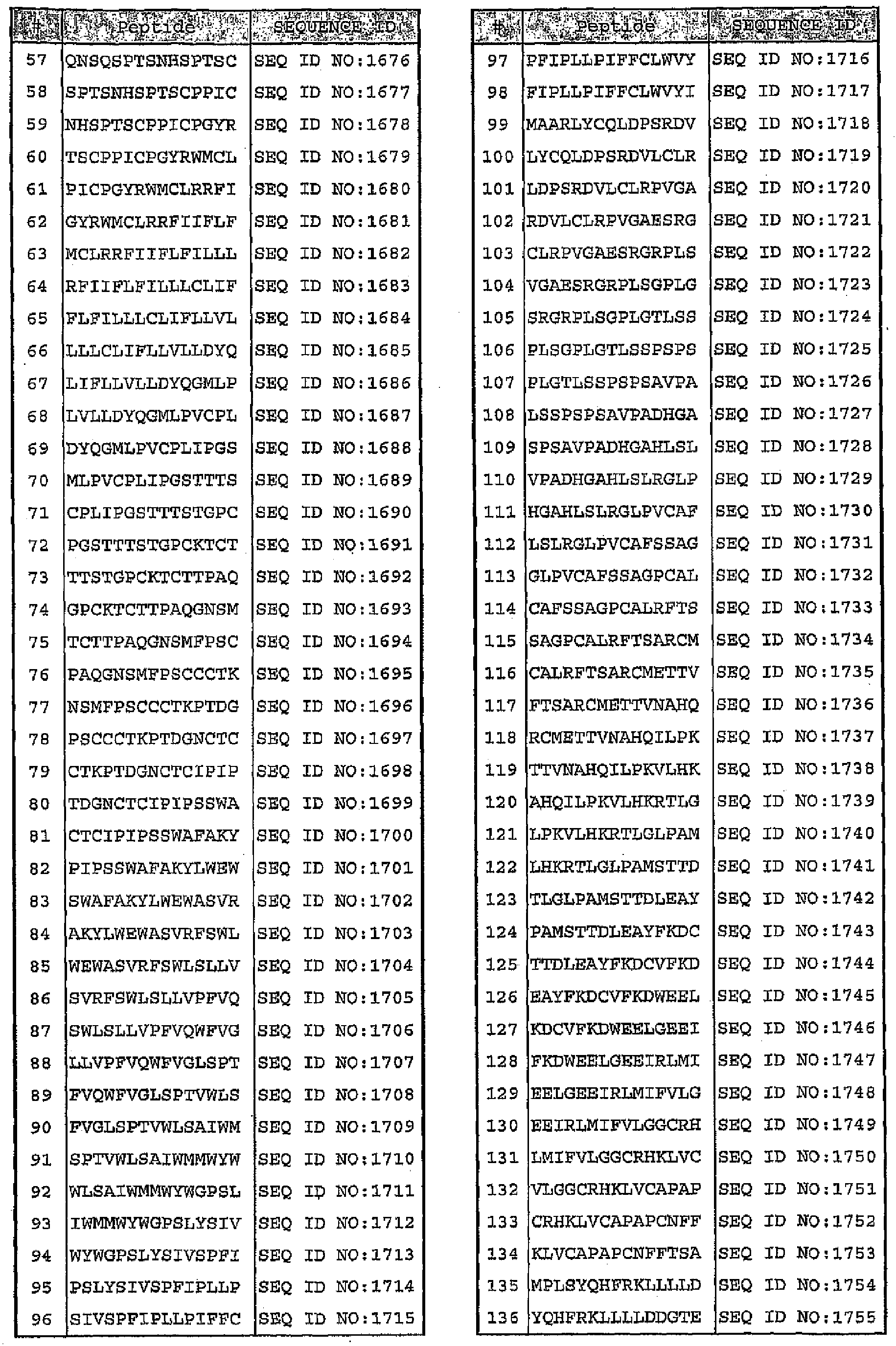

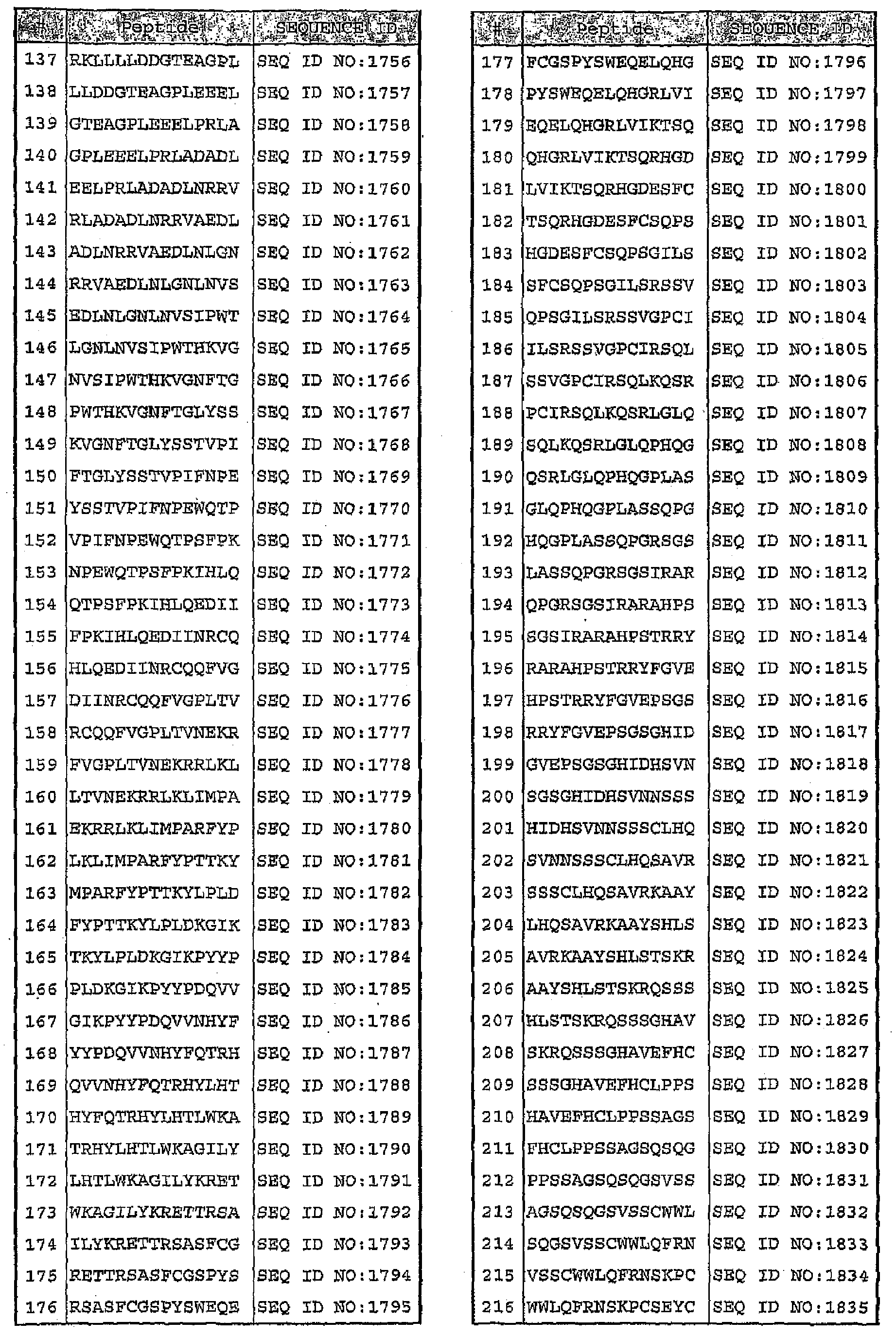

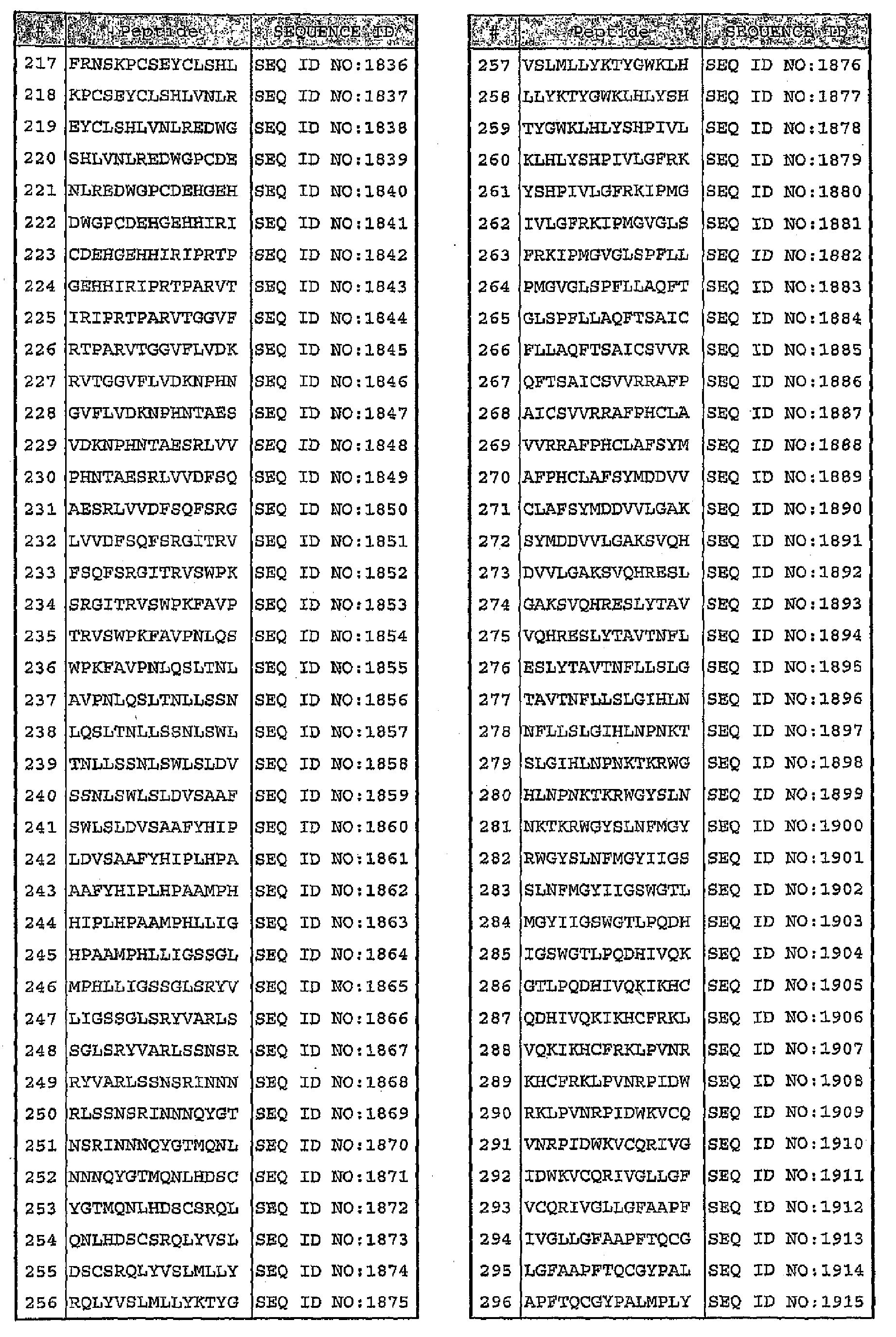

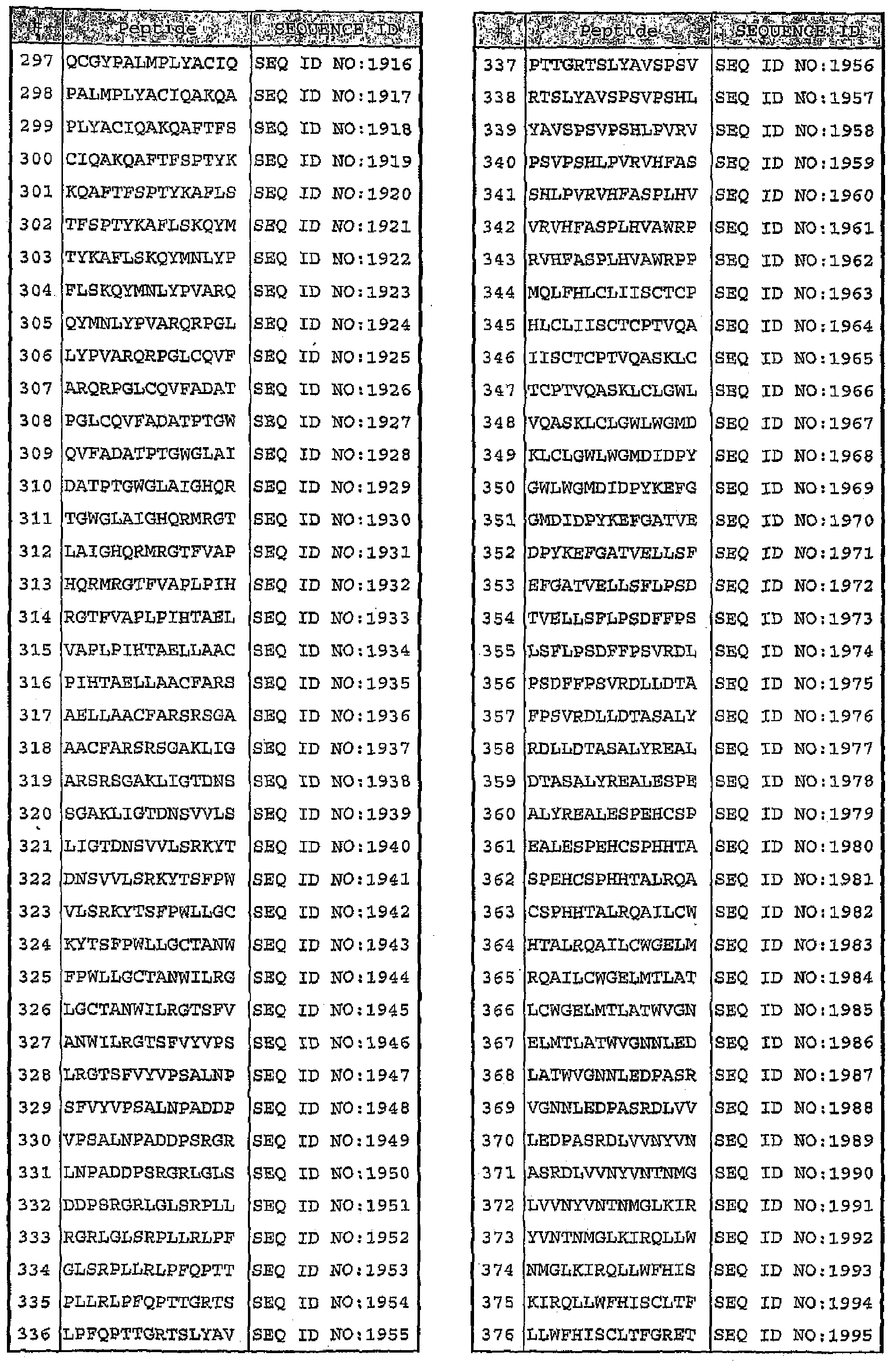

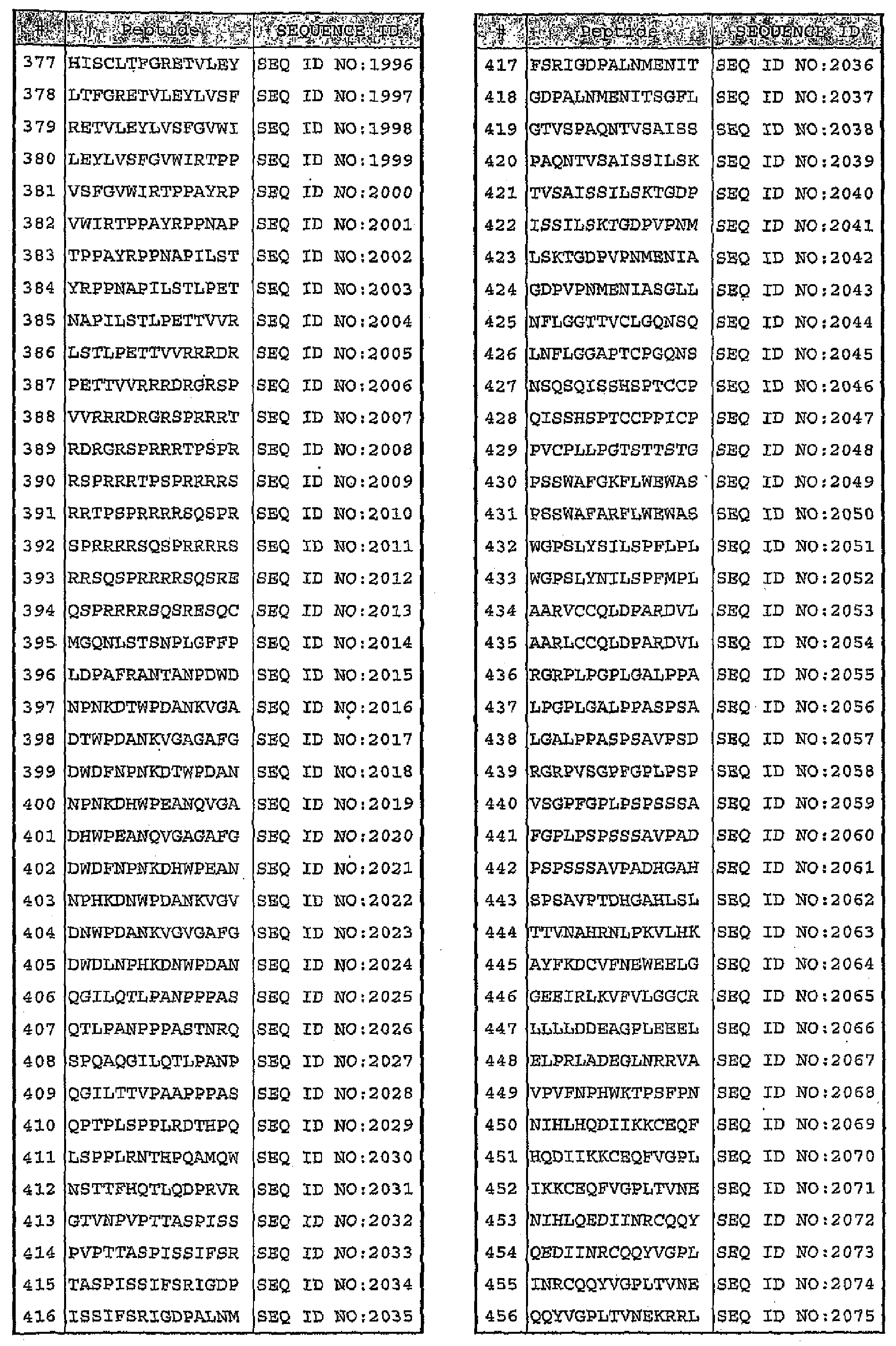

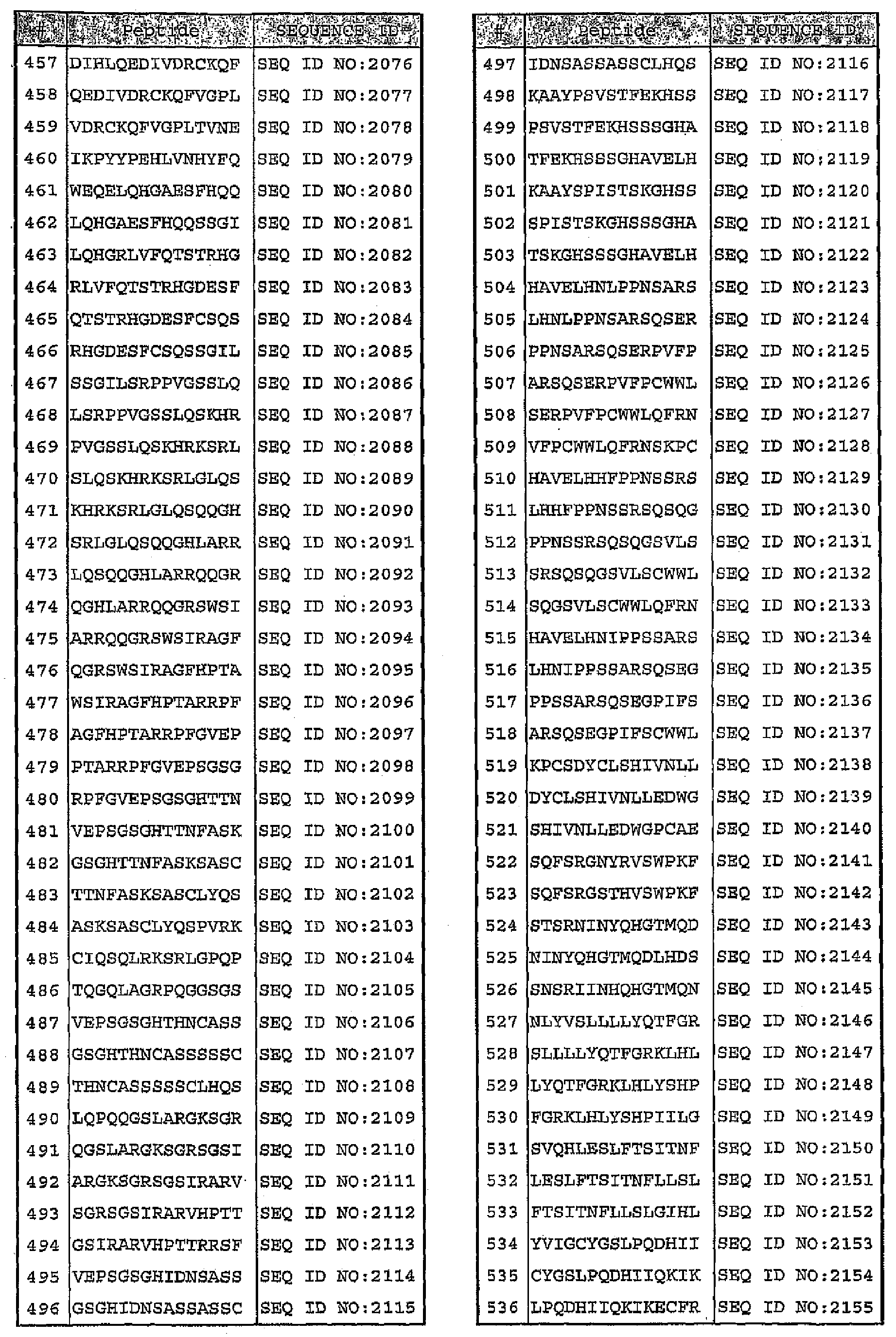

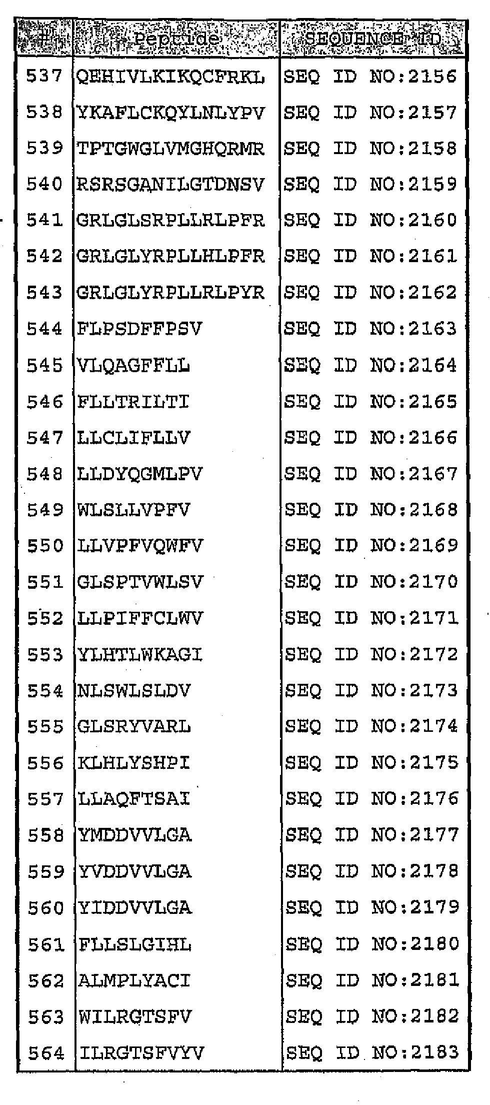

### BIBLIOGRAPHY

1. Piot, P., et al., The global impact of HIV/AIDS. Nature, 2001. 410(6831): p. 968-73.
2. UNAIDS, Global estimates of HIV/AIDS epidemic as of end 2002. 2003, UNAIDS.
3. Borrow, P., et al., Virus-specific CD8+ cytotoxic T-lymphocyte activity associated with control of viremia in primary human immunodeficiency virus type 1 infection, J Virol, 1994. 68(9): p. 6103-10.
4. Koup, R.A., et al., Temporal association of cellular immune responses with the initial control of viremia in primary human immunodeficiency virus type 1 syndrome. J Virol, 1994. 68(7): p. 4650-5,
5. Musey, L., et al., Cytotoxic-T-cell responses, viral load, and disease progression in early human immunodeficiency virus type 1 infection. N Engl J Med, 1997. 337(18): p. 1267-74.
6. Ogg, G.S., et al., Quantitation of HIV-1-specific cytotoxic T lymphocytes and plasma load of viral RNA. Science, 1998. 279(5359): p. 2103-6.
7. Carmichael, A., et al., Quantitative analysis of the human immunodeficiency virus type 1 (HIV- 1)-specific cytotoxic T lymphocyte (CTL) response at different stages of HIV 1 infection differential CTL responses to HIV-1 and Epstein- Barr virus in late disease. J Exp Med, 1993, 177(2): p. 249-56.
8. Rinaldo, C., et al., High levels of anti-human immunodeficiency virus type I (HIV-1) memory cytotoxic T-lymphocyte activity and low viral load are associated with lack of disease in HIV-1-infected long-term nonprogressors, J Virol, 1995. 69(9): p. 5838-42.
9. Hay, C.M., et aL, Lack of viral escape and defective in vivo activation of human immunodeficiency virus type 1-specific cytotoxic T lymphocytes in rapidly progressive infection. J Virol, 1999. 73 (7): p. 5509-19.
10. Johnson, P.R., et al., Inactivated whole SIV vaccine in macaques; evaluation of protective efficacy against challenge with cell-free virus or infected cells AIDS Res Hum Retroviruses, 1992.8(8): p. 1501-5.
11. Cranage, M.P., et al., Studies on the specificity of the vaccine effect elicited by inactivated simian immunodeficiency virus. AIDS Res Hum Retroviruses, 1993.9(1): p, 13-22.
12. Luke, W, et al., Simian immunodeficiency virus (SIV) gp130 oligomers protect rhesus macaques (Macaca mulatta) against the infection with SIVmac32H grown on T-cells or derived ex vivo. Virology, 1996. 216(2): p. 444-50.
13. Mooij, P., et al., A clinically relevant HIV-1 subunit vaccine protects rhesus macaques from in vivo passaged simian-human immunodeficiency virus infection. Aids, 1998,12(5): p. F15-22.
14. VaxGen, I., VaxGen Announces Initial Results of its Phase III AIDS Vaccine Trial. 2003, PR Newswire.
15. Kent, S.J., et al., Vaccination with attenuated simian immunodeficiency virus by DNA inoculation. J Virol, 2001. 75(23): p. 11930-4.
16. Kent, S.J., et al., Enhanced T-cell immunogenicity and protective efficacy of a human immunodeficiency virus type I vaccine regimen consisting of consecutive priming with DNA and boosting with recombinant fowlpox virus. J Virol, 1998.72(12): p. 10180-8.
17. Santra, S., et al., Prior vaccination increases the epitopic breadth of the cytotoxic T-lymphocyte response that evolves in rhesus monkeys following a simian- human immunodeficiency virus infection, J Virol, 2002. 76(12): p. 6376-81.
18. Estcourt, M.J., et al., Prime-boost immunization generates a high frequency, high-avidity CD8(+) cytotoxic T lymphacyte population. Int Immunol, 2002.14(1): p. 31-7.
19. Marzo, A.L., et al., Tumor-specific CD4+ T cells have a major "post-licensing'' role in CTL mediated anti-tumor immunity. J Immunol, 2000.165(11): p. 6047-55.
20. Nelson, D.J., et al., Tumor progression despite efficient tumor antigen cross-presentation and effective "arming" of tumor antigen-specific CTL. J Immunol, 2001. 166(9): p. 5557-66.
21. Ritchie, D.S., et al., Dendritic cell elimination as an assay of cytotoxic T lymphocyte activity in vivo. J Immunol Methods, 2000. 246(1-2): p. 109-17.
22. Carbone, F.R. and M.J. Bevan, Induction of ovalbumin-specific cytotoxic T cells by in vivo peptide immunization. J Exp Med, 1989.169(3): p. 603-12.
23. Carbone, F.R., et al., Induction of cytotoxic T lymphocytes by primary in vitro stimulation with peptides. J Exp Med, 1988. 167(6): p. 1767-79.
24. Kast, W.M., et al., Protection against lethal Sendai virus infection by in vivo priming af vir-us-specific cytotoxic T lymphocytes with a free synthetic peptide. Proc Natl Acad Sci U S A, 1991. 88(6): p. 2283-7.
25. Schulz, M., R.M. Zinkernagel, and H. Hengartner, Peptide-induced antiviral protection by cytotoxic T cells. Proc Natl Acad Sci U S A,1991. 88(3): p. 991-3_{.}
26. Feltkamp, M.C., et al., Vaccination with cytotoxic T lymphocyte epitope-containing peptide protects against a tumor induced by human papillomavirus type 16-transformed cells. Eur J Immunol, 1993. 23 (9): p. 2242-9.
27. Celluzzi, C.M., et al., Peptide-pulsed dendritic cells induce antigen-specific CTL-mediated protective tumor immunity. J Exp Med, 1996. 183(1): p. 283-7.
28. Kearney, E.R., et al., Visualization of peptide-specific T cell immunity and peripheral tolerance induction in vivo. Immunity, 1994. 1(4): p. 327-3 9.
29. Aichele, P., et al., T cell priming versus T cell tolerance induced by synthetic peptides. J Exp Med, 1995, 182(1): p. 261-6.
30. Toes, R.E., et al., Enhanced tumor outgrowth after peptide vaccination. Functional deletion of tumor-specific CTL induced by peptide vaccination can lead to the inability to reject tumors. J Immunol, 1996.156(10): p. 3911-8.
31. Toes, R.E., et al., Peptide vaccination can lead to enhanced tumor growth through specific T-cell tolerance induction. Proc Natl Acad Sci U S A, 1996. 93(15): p. 7855-60.
32. Dale, C.J., et al., Chimeric human papilloma virus-simian/human immunodeficiency virus virus-like-particle vaccines: immunogenicity and protective efficacy in macaques. Virology, 2002. 301(1); p. 176-87.
33. Jin, X., et al., Dramatic rise in plasma viremia after CD8(+) T cell depletion in simian immunodeficiency virus-infected macaques. J Exp Med, 1999.189(6): p. 991-8.
34. Carbone, F.R. and M.J. Bevan, Class I-restricted processing and presentation of exogenous cell- associated antigen in vivo. J Exp Med, 1990. 171(2): p. 377-87.
35. Larsson, M., et al., Efficiency of cross presentation of vaccinia virus-derived antigens by human dendritic cells. Eur J Immunol, 2001. 31(12): p. 3432-42.
36. Chen, Q., et al., A direct comparison of cytolytic T-lymphocyte responses to Melan-A peptides in vitro: differential immunogenicity of Melan-A27-35 and Melan-A26-35. Melanoma Res, 2000. 10(1): p. 16-25.
37. Lu, W., et al., Therapeutic dendritic-cell vaccine for simian AIDS, Nat Med, 2003. 9(1): p. 27-32.
38. Pospisilova, D., et al., Generation of functional dendritic cells for potential use in the treatment of acute lymphoblastic leukernia. Cancer Immunol Immunother, 2002. 51(2): p. 72-8.
39. Melief, C.J., et al., Effective therapeutic anticancer vaccines based on precision guiding of cytolytic T lymphocytes. Immunol Rev, 2002. 188(1): p. 177-82.
40. Mehlhop, E., et al., Enhanced in vitro stimulation of rhesus macaque dendritic cells for activation of SIV-specific T cell responses. J Immunol Methods, 2002. 260(1-2): p. 219-34.
41. Sapp, M., et al., Dendritic cells generated from blood monocytes of HIV-1 patients are not infected and act as competent antigen presenting cells eliciting potent T-cell responses. Immunol Lett, 1999, 66(1-3): p. 121-8.
42. Chougnet, C., et al., Normal immune function of monocyte-derived dendritic cells from HIV-infected individuals: implications for immunotherapy. J Immunol, 1999. 163(3): p. 1666-73.

### Embodiments of the inventions

The following are preferred embodiments of the invention:
1. Use of at least one set of peptides in the preparation of a medicament for modulating an immune response, wherein individual peptides of a respective set comprise different portions of an amino acid sequence corresponding to a single polypeptide of interest and display partial sequence identity or to at least one other peptide of the same set of peptides.
2. A use according to embodiment 1, wherein at least 2 sets of peptides are employed, and wherein peptide sequences in each set are derived from a distinct polypeptide of interest.
3. A use according to embodiment 1, wherein at least 3 sets of peptides are employed, and wherein peptide sequences in each set are derived from a distinct polypeptide of interest.
4. A use according to embodiment 1, wherein the partial sequence identity or similarity is contained at one or both ends of an individual peptide.
5. A use according to embodiment 4, wherein are at least 4 contiguous amino acid residues are contained at one or both of these ends whose sequence is identical or similar to an amino acid sequence contained within at least one other of the peptides.
6. A use according to embodiment 1, wherein the peptide is at least 6 amino acid residues in length.
7. A use according to embodiment 1, wherein the peptide is no more than about 500 amino acid residues in length.
8. A use according to embodiment 1, wherein the length of the peptides is selected to enhance the production of a cytolytic T lymphocyte response.
9. A use according to embodiment 8, wherein the length of the peptides is from about 8 to about 10 amino acid residues.
10. A use according to embodiment 1, wherein the length of the peptides is selected to enhance the production of r a T helper lymphocyte response.
11. A use according to embodiment 10, wherein the length of the peptides is from about 12 to about 20 amino acids residues.
12. A use according to embodiment 1, wherein the peptide sequences are derived from at least about 30% of the sequence corresponding to the polypeptide of interest.
13. A use according to embodiment 1, wherein the peptide sequences are derived from at least about 90% of the sequence corresponding to the polypeptide of interest.
14. A use according to embodiment 1, wherein the polypeptide of interest is an antigen selected from a protein antigen, an antigen expressed by cancer cells, a particulate antigen, an alloantigen, an autoantigen or an allergen, or an immune complex.
15. A use according to embodiment 1, wherein the polypeptide of interest is a disease-or condition-associated polypeptide.
16. A use according to embodiment 15, wherein the disease-or condition-associated polypeptide is a polypeptide produced by a pathogenic organism or a cancer.
17. A use according to embodiment 15, wherein the disease-or condition-associated polypeptide is produced by a of pathogenic organism selected from yeast, viruses, bacteria, helminths, protozoans and mycoplasmas.
18. A use according to embodiment 15, wherein the disease-or condition-associated polypeptide is produced by a cancer selected from melanoma, lung cancer, breast cancer, cervical cancer, prostate cancer, colon cancer, pancreatic cancer, stomach cancer, bladder cancer, kidney cancer, post transplant lymphoproliferative disease (PTLD) and Hodgkin's Lymphoma.
19. An antigen-presenting cell or its precursor which has been contacted with at least one set of peptides for a time and under conditions sufficient for the peptides or processed forms thereof to be presented by the antigen-presenting cell or by its precursor, wherein individual peptides of a respective set comprise different portions of an amino acid sequence corresponding to a single polypeptide of interest and display partial sequence identity or r similarity to at least one other peptide of the same set of peptides.
20. A population of antigen-presenting cells or their precursors which have been contacted with at least one set of peptides for a time and under conditions sufficient for the peptides or processed forms thereof to be presented by the antigen-presenting cells or by their precursors, wherein individual peptides of a respective set comprise different portions of an amino acid sequence corresponding to a single polypeptide of interest and display partial sequence identity or similarity to at least one other peptide of the same set of peptides.
21. A process for producing antigen-presenting cells for modulating an immune response to a polypeptide of interest, the process comprising contacting antigen-presenting cells or their precursors with at least one set of peptides for a time and under conditions sufficient for the peptides or processed form thereof to be presented by the antigen-presenting cells or by their precursors, wherein individual peptides of a respective set comprise different portions of an amino acid sequence corresponding to a single polypeptide of interest and display partial sequence identity or similarity to at least one other peptide of the same set of peptides.
22. A process according to embodiment 21, further comprising culturing for a time and under conditions sufficient to differentiate antigen-presenting cells from the precursors.
23. A process according to embodiment 21, wherein the or each set of peptides is contacted with substantially purified antigen-presenting cells or their precursors.
24. A process according to embodiment 21, wherein the or each set of peptides is contacted with a heterogeneous population of antigen-presenting cells or their precursors.
25. A process according to embodiment 22, wherein the heterogenous pool of cells is selected from blood or peripheral blood mononuclear cells.
26. A process according to embodiment 21, wherein the antigen-presenting cells or their precursors are selected from monocytes, macrophages, cells of myeloid lineage, B cells, dendritic cells or Langerhans cells.
27. A process according to embodiment 21, wherein the or each set of peptides is contacted with an uncultured population of antigen-presenting cells or their precursors.
28. A process according to embodiment 27, wherein the population is homogenous.
29. A process according to embodiment 27, wherein the population is heterogeneous.
30. A process according to embodiment 27, wherein the population is a heterogeneous population selected from whole blood, fresh blood, or fractions thereof selected from peripheral blood mononuclear cells, buffy coat fractions of whole blood, packed red cells, irradiated blood, dendritic cells, monocytes, macrophages, neutrophils, lymphocytes, natural killer cells or natural killer T cells.
31. A method for producing antigen-specific lymphocytes, the method comprising contacting a population of lymphocytes, or their precursors, with the population of antigen-presenting cells or their precursors according to embodiment 19 for a time and under conditions sufficient to produce antigen-specific lymphocytes that modulate an immune response to at least one of the polypeptides of interest.
32. A composition comprising at least one set of peptides and a pharmaceutically acceptable carrier and/or diluent, wherein individual peptides of a respective set comprise different portions of an amino acid sequence corresponding to a single polypeptide of interest and display partial sequence identity or similarity to at least one other peptide of the same set of peptides.
33. A composition according to embodiment 32, further comprising an adjuvant or a compound that stabilises the peptides against degradation by host enzymes.
34. A composition comprising an antigen-presenting cell or its precursor according to embodiment 19, or a population antigen-presenting cells or their precursors according to embodiment 20, and a pharmaceutically acceptable carrier and/or diluent.
35. A composition according to embodiment 34, further comprising an adjuvant.
36. A composition comprising antigen-specific lymphocytes produced according to the of embodiment 31, and a pharmaceutically acceptable carrier and/or diluent.
37. A composition according to embodiment 36, further comprising an adjuvant.
38. A method for modulating an immune response to a polypeptide of interest, comprising administering to a patient in need of such treatment at least one set of peptides for a time and under conditions sufficient to modulate the immune response, wherein individual peptides of a respective set comprise different portions of an amino acid sequence corresponding to a single polypeptide of interest and display partial sequence identity or similarity to at least one other peptide of the same set of peptides.
39. A method for modulating an immune response to a polypeptide of interest, comprising administering to a patient in need of such treatment a population of antigen-presenting cells according to embodiment 20 for a time and under conditions sufficient to modulate the immune response.
40. A method for modulating an immune response to a polypeptide of interest, comprising administering to a patient in need of such treatment antigen-specific lymphocytes produced according to the of embodiment 31 for a time and under conditions sufficient to modulate the immune response.
41. A method for treatment and/or prophylaxis of a disease or condition associated with the presence of a polypeptide of interest, comprising administering to a patient in need of such treatment or prophylaxis an effective amount of at least one set of peptides, wherein individual peptides of a respective set comprise different portions of an amino acid sequence corresponding to a single polypeptide of interest and display partial sequence identity or similarity to at least one other peptide of the same set of peptides.
42. A method for treatment and/or prophylaxis of a disease or condition associated with the presence of a polypeptide of interest, comprising administering to a patient in need of such treatment or prophylaxis an effective amount of a population of antigen-presenting cells according to embodiment 20.
43. A method for treatment and/or prophylaxis of a disease or condition associated with the presence of a polypeptide of interest, comprising administering to a patient in need of such treatment or prophylaxis an effective amount of antigen-specific lymphocytes produced according to the of embodiment 31.
44. A composition of matter for modulating an immune response in a subject to a target antigen, the composition comprising uncultured antigen-presenting cells or their precursors, which have not been subjected to activating conditions, and which have been contacted with an antigen corresponding to the target antigen for a time and under conditions sufficient to express a processed or modified form of the antigen for presentation to the subject's immune system.
45. A composition according to embodiment 44, wherein the uncultured antigen-presenting cells or their precursors are contacted with the antigen from about 1 minute to about 5 days.
46. A composition according to embodiment 44, wherein the uncultured antigen-presenting cells or their precursors are selected from whole blood, fresh blood, or fractions thereof.
47. A composition according to embodiment 46, wherein fractions are selected from peripheral blood mononuclear cells, buffy coat fractions of whole blood, packed red cells, irradiated blood, dendritic cells, monocytes, macrophages, neutrophils, lymphocytes, natural killer cells and natural killer T cells.
48. A composition according to embodiment 44, wherein the antigen corresponding to the target antigen is selected from: nucleic acids; peptides; hormones ; whole protein antigens; cellular material; particulate matter selected from cell debris, apoptotic cells, lipid aggregates, membranous vehicles, microspheres, heat aggregated proteins, virosomes, virus-like particles; and whole organisms selected from bacteria, mycobacteria, viruses, fungi, protozoa or parts thereof.
49. A composition according to embodiment 44, wherein the antigen is selected from a proteinaceous molecule or a nucleic acid molecule.
50. A composition according to embodiment 44, wherein the uncultured cells are contacted with at two or more antigens.
51. A composition according to embodiment 50, wherein the antigens are in a form selected from overlapping peptides, non-overlapping peptides, one or more polynucleotides from which overlapping peptides are expressible or one or more polynucleotides from which non-overlapping peptides are expressible.
52. Use of uncultured antigen-presenting cells or their precursors in the preparation of a medicament for the treatment of a disease or condition in a subject, which disease or condition is associated with the presence or aberrant expression of a target antigen, wherein the antigen-presenting cells or their precursors have not been subjected to activating conditions but have been contacted with an antigen that corresponds to the target antigen for a time and under conditions sufficient to express a processed or modified form of the antigen for presentation to the subject's immune system.

## Claims

1. A composition for use in modulating an immune response in a subject to a target antigen, the composition comprising antigen-presenting cells or their precursors, which have not been subjected to activating conditions and have been incubated or processed under conditions that result in an increase of less than about 50% in cell number as compared to the number of cells at the commencement of the incubation or processing, wherein the antigen presenting cells or their precursors have been removed from an animal, and have been contacted with an antigen corresponding to the target antigen for a time and under conditions sufficient to present a processed or modified form of the antigen for presentation to the subject's immune system, and wherein the antigen-presenting cells or their precursors have been contacted with the antigen for less than about 4 hours.

2. A composition according to claim 1, wherein the antigen-presenting cells or their precursors have been contacted with the antigen for less than 2 hours, preferably for less than 1 hour.

3. A composition according to claim 1, wherein the antigen-presenting cells or their precursors are incubated or processed under conditions that result in an increase of less than about 20% in cell number as compared to the number of cells at the commencement of incubation or processing,
wherein preferably the antigen-presenting cells or their precursors are incubated or processed under conditions that result in an increase of less than about 10% in cell number as compared to the number of cells at the commencement of the incubation or processing,
wherein preferably the antigen-presenting cells or their precursors are incubated or processed under conditions that result in an increase of less than about 5% in cell number as compared to the number of cells at the commencement of the incubation or processing.

4. A composition according to any one of claims 1 to 3, wherein the antigen-presenting cells or their precursors are selected from whole blood, fresh blood, or fractions thereof, wherein preferably fractions are selected from peripheral blood mononuclear cells, buffy coat fractions of whole blood, packed red cells, irradiated blood, dendritic cells, monocytes, macrophages, neutrophils, lymphocytes, natural killer cells and natural killer T cells.

5. A composition according to any one of claims 1 to 4, wherein the antigen corresponding to the target antigen is selected from: nucleic acids; peptides; hormones; whole protein antigens; cellular material; particulate matter selected from cell debris, apoptotic cells,
lipid aggregates, membranous vehicles, microspheres, heat aggregated proteins, virosomes, virus-like particles; and whole organisms selected from bacteria, mycobacteria, viruses, fungi, protozoa or parts thereof,
wherein preferably the antigen is selected from a proteinaceous molecule or a nucleic acid molecule.

6. A composition according to any one of claims 1 to 5, wherein the antigen-presenting cells have been contacted with two or more antigens.

7. A composition according to claim 6, wherein the antigens are in a form selected from overlapping peptides, non-overlapping peptides, one or more polynucleotides from which overlapping peptides are expressible or one or more polynucleotides from which non-overlapping peptides are expressible.

8. A composition according to any one of claims 1 to 6, wherein the antigen-presenting cells have been contacted with at least one set of peptides, wherein individual peptides of a respective set comprise different portions of an amino acid sequence corresponding to a single polypeptide of interest and display partial sequence identity or similarity to at least one other peptide of the same set of peptides,
wherein preferably at least 2 sets of peptides are employed, and wherein peptide sequences in each set are derived from a distinct polypeptide of interest, and/or
wherein the partial sequence identity or similarity is contained at one or both ends of an individual peptide, and/or
wherein the length of the peptides is 8 to 10 amino acid residues for enhancing the production of a cytolytic T lymphocyte response or is 12 to 20 amino acid residues for enhancing the production of a T helper lymphocyte response.

9. A composition according to claim 8, wherein the peptide sequences are derived from at least about 30% of the sequence corresponding to the polypeptide of interest, or
wherein the polypeptide of interest is an antigen selected from a protein antigen, an antigen expressed by cancer cells, a particulate antigen, an alloantigen, an autoantigen or an allergen, or an immune complex, or
wherein the polypeptide of interest is a polypeptide produced by a pathogenic organism or a cancer.

10. A process for producing antigen-presenting cells for modulating an immune response to a target antigen, the process comprising contacting a population of antigen-presenting cells or their precursors, which have been removed from an animal, with an antigen corresponding to the target antigen for a time and under conditions sufficient to present a processed or modified form of antigen, wherein the antigen presenting cells or their precursors are contacted with the antigen for less than 4 hours, and wherein the antigen-presenting cells for modulating said immune response have not been subjected to activating conditions and have been incubated or processed under conditions that result in an increase of less than about 50% in cell number as compared to the number of cells at the commencement of the incubation or processing,
wherein preferably the antigen(s) is as defined in any one of claims 5 to 9

11. A process according to claim 10, wherein the population is a heterogeneous population selected from whole blood, fresh blood, or fractions thereof selected from peripheral blood mononuclear cells, buffy coat fractions of whole blood, packed red cells, irradiated blood, dendritic cells, monocytes, macrophages, neutrophils, lymphocytes, natural killer cells or natural killer T cells.

12. Use of a composition according to any one of claims 1 to 9 in the manufacture of a medicament for treating or preventing a disease or condition selected from a pathogenic infection, a cancer, an autoimmune disease, an allergy or a transplantation disease.

13. Use of antigen-presenting cells or their precursors in the preparation of a medicament for the treatment and/or prophylaxis of a disease or condition in a subject, which disease or condition is associated with the presence of a target antigen wherein the antigen-presenting cells or their precursors have not been subjected to activating conditions and have been incubated or processed under conditions that result in an increase of less than about 50% in cell number as compared to the number of cells at the commencement of the incubation or processing, wherein the antigen presenting cells or their precursors have been removed from an animal and have been contacted with an antigen that corresponds to the target antigen for a time and under conditions sufficient to present a processed or modified form of the antigen to the subject's immune system, wherein the antigen presenting cells or their precursors have been contacted with the antigen for less than about 8 hours, wherein the disease or condition is selected from a pathogenic infection, a cancer, an autoimmune disease, an allergy or a transplantation disease.

14. A composition for use in modulating an immune response in a subject to a target antigen, the composition comprising a heterogeneous population of antigen-presenting cells or their precursors, which have not been subjected to activating conditions, and have been incubated or processed under conditions that result in an increase of less than about 50% in cell number as compared to the number of cells at the commencement of the incubation or processing, wherein the antigen presenting cells or their precursors have been removed from a subject and have been contacted with an antigen corresponding to the target antigen for a time and under conditions sufficient to present a processed or modified form of the antigen for presentation to the subject's immune system, wherein the heterogeneous population is selected from whole blood, fresh blood, packed red cells and irradiated blood.

15. A composition for use in modulating an immune response in a subject to a target antigen, the composition comprising antigen-presenting cells or their precursors, which have not been subjected to activating conditions, and have been incubated or processed under conditions that result in an increase of less than about 50% in cell number as compared to the number of cells at the commencement of the incubation or processing, wherein the antigen presenting cells or their precursors have been removed from an animal and have been contacted with at least one set of peptides wherein individual peptides of a respective set comprise different portions of an amino acid sequence corresponding to a single polypeptide of interest and display partial sequence identity or similarity to at least one other peptide of the same set of peptides.
